# EUROPEAN PATENT SPECIFICATION

(11) **EP 1 163 340 B1**
(45) Date of publication and mention of the grant of the patent: **15.06.2005**
(21) Application number: 00905490.9
(22) Date of filing: 24.02.2000
(51) Int. Cl.: C12N 15/29, C12N 15/82, C12N 15/63, A01H 5/00, A01H 1/00

(54) **COMPOSITIONS AND METHODS FOR THE MODIFICATION OF GENE EXPRESSION**
VERBINDUNGEN UND VERFAHREN ZUR ÄNDERUNG DER GENEXPRESSION
COMPOSITIONS ET METHODES DE MODIFICATION DE L'EXPRESSION GENIQUE

(30) Priority: 25.03.1999 US 276599; 30.07.1999 US 146591 P
(43) Date of publication of application: 19.12.2001
(73) Proprietor: Genesis Research & Development Corporation Limited, Parnell, Auckland (NZ); Rubicon Forests Holdings Limited, Auckland (NZ)
(72) Inventor: PERERA, Ranjan, San Diego, CA 92122 (US); RICE, Stephen J., Papatoetoe, Auckland (NZ); EAGLETON, Clare Katherine, Pakuranga, Auckland (NZ)
(74) Representative: Kremer, Simon Mark
(86) International application number: PCT/NZ2000/000018
(87) International publication number: WO 2000/058474

(56) References cited:
- WO-A-98/13503
- US-A- 5 614 399
- DATABASE EMBL [Online] Acession no. Q38744, 1 November 1996 (1996-11-01) DAVIES K.M. ET AL.: "Nucleotide sequence and characterization of an antirrhinum majus cDNA encoding a polyubiquitin mRNA" XP002193634
- ALLONA ISABEL ET AL: "Analysis of xylem formation in pine by cDNA sequencing" PROCEEDINGS OF THE NATIONAL ACADEMY OF SCIENCES OF USA, vol. 95, no. 16, 4 August 1998 (1998-08-04), pages 9693-9698, XP002186770 ISSN: 0027-8424 -& DATABASE EMBL [Online] Accession no. AA556927, 3 August 1998 (1998-08-03) ALLONA I. ET AL.: "Analysis of xylem formation in pine by cDNA sequencing, 769 Loblolly pine C Pinus taeda cDNA clone 7C5A, mRNA sequence" XP002186771
- DATABASE EMBL [Online] Accession no. AW290494, 17 January 2000 (2000-01-17) SEDEROFF R.: "Molecular Basis of Wood Formation in the Pine Megagenome" XP002193636
- DATABASE GENBANK ACCESSION NO. AF041463, 06 January 1998 SUHANDONO ET AL.
- DATABASE GENBANK ACCESSION NO. U90350, 24 February 1997 WALDEN A.R. ET AL.
- DATABASE GENBANK ACCESSION NO. U73588, 07 October 1996 PERE-GRAU L. ET AL.
- DATABASE GENBANK ACCESSION NO. AF077743, 13 July 1998 REHLI M. ET AL.
- DATABASE GENBANK ACCESSION NO. X74814, 27 August 1993 POEYDOMENGE O. ET AL.
- DATABASE GENBANK ACCESSION NOS. M55147, X51434 LIAUD M. AND CERFF R. & PROC. NATL. ACAD. SCI. USA vol. 87, no. 22, 1990, pages 8918 - 8922
- DATABASE GEN PEPT ACCESSION NO. CAA10056, 12 November 1998 FRUEHLING M.
- DATABASE GEN PEPT ACCESSION NO. CAA63531, 09 November 1995 RUITER R.K.

## Description

### Technical Field of the Invention

This invention relates to the regulation of polynucleotide transcription and/or expression. More specifically, this invention relates to polynucleotide regulatory sequences isolated from plants that are capable of initiating and driving the transcription of polynucleotides, and the use of such regulatory sequences in the modification of transcription of endogenous and/or heterologous polynucleotides and production of polypeptides. Polypeptide sequences are also disclosed.

### Background of the Invention

Gene expression is regulated, in part, by the cellular processes involved in transcription. During transcription, a single-stranded RNA complementary to the DNA sequence to be transcribed is formed by the action of RNA polymerases. Initiation of transcription in eucaryotic cells is regulated by complex interactions between cis-acting DNA motifs, located within the gene to be transcribed, and trans-acting protein factors. Among the cis-acting regulatory regions are sequences of DNA, termed promoters, to which RNA polymerase is first bound, either directly or indirectly. As used herein, the term "promoter" refers to the 5' untranslated region of a gene that is associated with transcription and which generally includes a transcription start site. Other cis-acting DNA motifs, such as enhancers, may be situated further up- and/or down-stream from the initiation site.

Both promoters and enhancers are generally composed of several discrete, often redundant elements, each of which may be recognized by one or more trans-acting regulatory proteins, known as transcription factors. Promoters generally comprise both proximal and more distant elements. For example, the so-called TATA box, which is important for the binding of regulatory proteins, is generally found about 25 basepairs upstream from the initiation site. The so-called CAAT box is generally found about 75 basepairs upstream of the initiation site. Promoters generally contain between about 100 and 1000 nucleotides, although longer promoter sequences are possible.

For the development of transgenic plants, constitutive promoters that drive strong transgene expression are preferred. Currently, the only available constitutive plant promoter that is widely used is derived from Cauliflower Mosaic Virus. Furthermore, there exists a need for plant-derived promoters for use in transgenic food plants due to public conceptions regarding the use of viral promoters. Few gymnosperm promoters have been cloned and those derived from angiosperms have been found to function poorly in gymnosperms. There thus remains a need in the art for polynucleotide promoter regions isolated from plants for use in modulating transcription and expression of polynucleotides in transgenic plants.

### Summary of the Invention

Briefly, isolated polynucleotide regulatory sequences from eucalyptus and pine that are involved in the regulation of gene expression are disclosed, together with methods for the use of such polynucleotide regulatory regions in the modification of expression of endogenous and/or heterologous polynucleotides in transgenic plants. In particular, the present invention provides polynucleotide promoter sequences from 5' untranslated, or non-coding, regions of plant genes that initiate and regulate transcription of polynucleotides placed under their control, together with isolated polynucleotides comprising such promoter sequences.

In a first aspect, the present invention provides isolated polynucleotide sequences comprising a sequence selected from the group consisting of: (a) the sequence recited in SEQ ID NO: 2 or 3, (b) complements of the sequence recited in SEQ ID NO: 2 or 3; (c) reverse complements of the sequence recited in SEQ ID NO: 2 or 3; (d) reverse sequences of the sequence recited in SEQ ID NO: 2 or 3; (e) sequences having at least 75% identity to the sequence recited in SEQ ID NO: 2 or 3 and wherein the polynucleotide is able to regulate transcription of a DNA sequence of interest; and (f) sequences having at least 90% identity to the sequence recited in SEQ ID NO: 2 or 3, wherein the polynucleotide is able to regulate transcription of a DNA sequence of interest, all of which are referred to herein as "polynucleotides of the present invention."

In another aspect, the present invention provides genetic constructs comprising a polynucleotide of the present invention, either alone, or in combination with one or more additional polynucleotides of the present invention, or in combination with one or more known polynucleotides, together with cells and target plants comprising such constructs.

In a related aspect, the present invention provides genetic constructs comprising, in the 5'-3' direction, a polynucleotide promoter sequence of the present invention, a polynucleotide to be transcribed, and a gene termination sequence. The polynucleotide to be transcribed may comprise an open reading frame of a polynucleotide that encodes a polypeptide of interest, or it may be a non-coding, or untranslated, region of a polynucleotide of interest. The open reading frame may be orientated in either a sense or antisense direction. Preferably, the gene termination sequence is functional in a host plant. Most preferably, the gene termination sequence is that of the gene of interest, but others generally used in the art, such as the *Agrobacterium tumefaciens* nopalin synthase terminator may be usefully employed in the present invention. The genetic construct may further include a marker for the identification of transformed cells.

In a further aspect, transgenic plant cells comprising the genetic constructs of the present invention are provided, together with organisms, such as plants, comprising such transgenic cells, and fruits, seeds and other products, derivatives, or progeny of such plants. Propagules of the inventive transgenic plants are included in the present invention. As used herein, the word "propagule" means any part of a plant that may be used in reproduction or propagation, sexual or asexual, including cuttings.

Plant varieties, particularly registrable plant varieties according to Plant Breeders' Rights, may be excluded from the present invention. A plant need not be considered a "plant variety" simply because it contains stably within its genome a transgene, introduced into a cell of the plant or an ancestor thereof.

In yet another aspect, methods for modifying gene expression in a target plant are provided, such methods including stably incorporating into the genome of the plant a genetic construct of the present invention. In a preferred embodiment, the target organism is a woody plant, most preferably selected from the group consisting of eucalyptus and pine species, most preferably from the group consisting of *Eucalyptus grandis* and *Pinus radiata.*

In another aspect, methods for producing a target plant, having modified polypeptide expression are provided, such methods comprising transforming a plant cell with a genetic construct of the present invention to provide a transgenic cell, and cultivating the transgenic cell under conditions conducive to regeneration and mature plant growth.

In other aspects, methods for identifying a gene responsible for a desired function or phenotype are provided, the methods comprising transforming a plant cell with a genetic construct comprising a polynucleotide promoter sequence of the present invention operably linked to a polynucleotide to be tested, cultivating the plant cell under conditions conducive to regeneration and mature plant growth to provide a transgenic plant; and comparing the phenotype of the transgenic plant with the phenotype of non-transformed, or wild-type, plants.

In yet further aspects, the present invention provides isolated polynucleotides comprising the DNA sequence of SEQ ID NO: 21, or a complement, reverse complement or reverse sequences of a sequence recited in SEQ ID NO: 21, together with DNA constructs comprising such polynucleotides and cells transformed with such sequences. As discussed below, removal of the sequence of SEQ ID NO: 21 from a polynucleotide that comprises the sequence of SEQ ID NO: 21 may enhance expression of the polynucleotide. Conversely, the inclusion of the sequence of SEQ ID NO: 21 in a genetic construct comprising a polynucleotide of interest may decrease expression of the polynucleotide.

The above-mentioned and additional features of the present invention and the manner of obtaining them will become apparent, and the invention will be best understood by reference to the following more detailed description.

### Detailed Description of the Invention

The present invention provides isolated polynucleotide regulatory regions that may be employed in the manipulation of plant phenotypes, together with isolated polynucleotides comprising such regulatory regions. More specifically, polynucleotide promoter sequences isolated from pine and eucalyptus are disclosed. As discussed above, promoters are components of the cellular "transcription apparatus" and are involved in the regulation of gene expression. Both tissue- and temporal-specific gene expression patterns have been shown to be initiated and controlled by promoters during the natural development of a plant. The isolated polynucleotide promoter sequences of the present invention may thus be employed in the modification of growth and development of plants, and of cellular responses to external stimuli, such as environmental factors and disease pathogens.

Using the methods and materials of the present invention, the amount of a specific polypeptide of interest may be increased or reduced by incorporating additional copies of genes encoding the polypeptide, operably linked to an inventive promoter sequence, into the genome of a target plant. Similarly, an increase or decrease in the amount of the polypeptide may be obtained by transforming the target plant with antisense copies of such genes.

The polynucleotides of the present invention were isolated from forestry plant sources, namely from *Eucalyptus grandis* and *Pinus radiata,* but they may alternatively be synthesized using conventional synthesis techniques. Specifically, isolated polynucleotides of the present invention include polynucleotides comprising a sequence selected from the group consisting of sequences identified as SEQ ID NO: 2 and 3; complements of the sequences identified as SEQ ID NO: 2 and 3; reverse complements of the sequences identified as SEQ ID NO: 2 and 3, antisense sequences corresponding to any of the above polynucleotides; and variants of any of the above polynucleotides, as that term is described in this specification.

The polynucleotides of the present invention were putatively identified by DNA and polypeptide similarity searches. In the attached Sequence Listing, SEQ ID NOS. 1-14. 20. 22-62. 81-86 and 88-112 are polynucleotide sequences, and SEQ ID NOS. 63-80 and 87 are polypeptide sequences. The polynucleotides of the present invention, have demonstrated similarity to promoters that are known to be involved in regulation of transcription and/or expression in plants. The putative identity of each of the inventive polynucleotides is shown below in Table 1. together with the 5' untranslated region (5' UTR) or putative promoter region (identified by residue number).

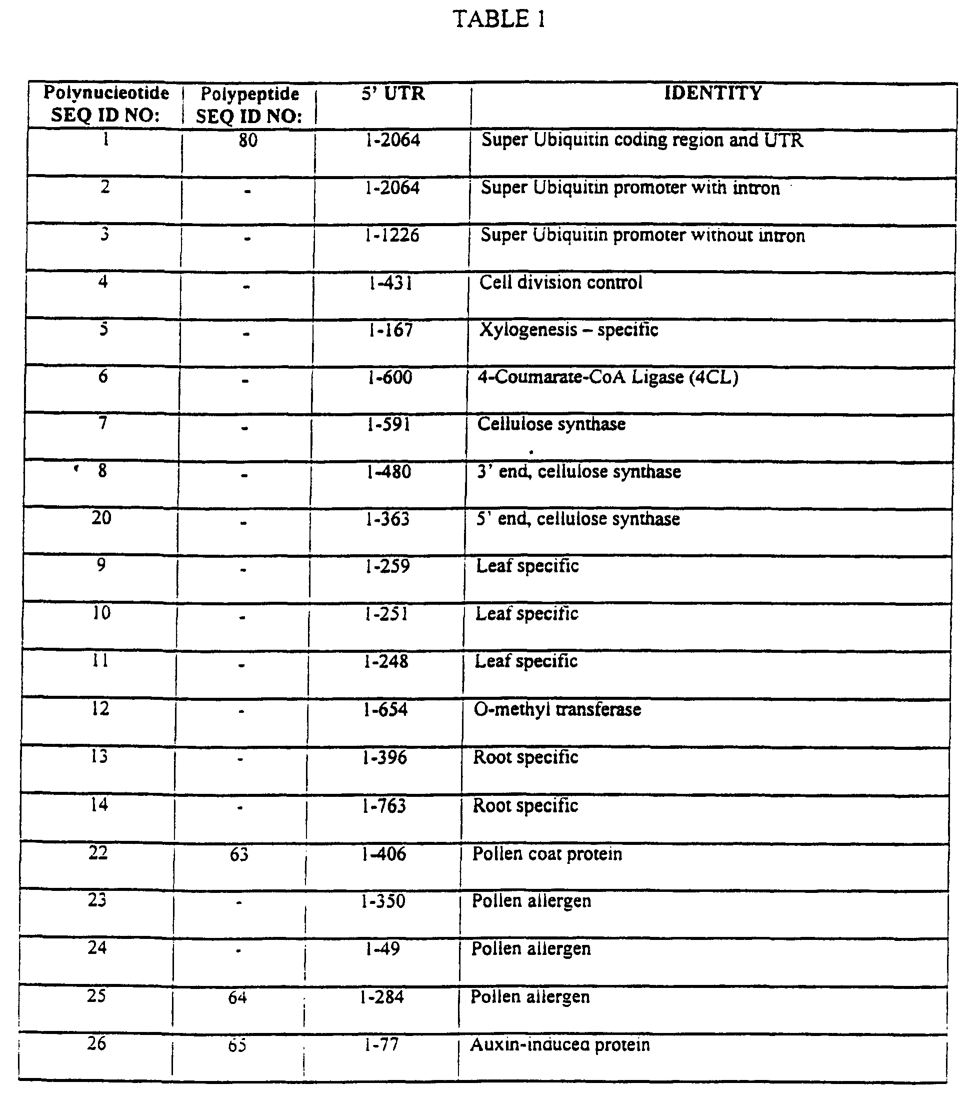

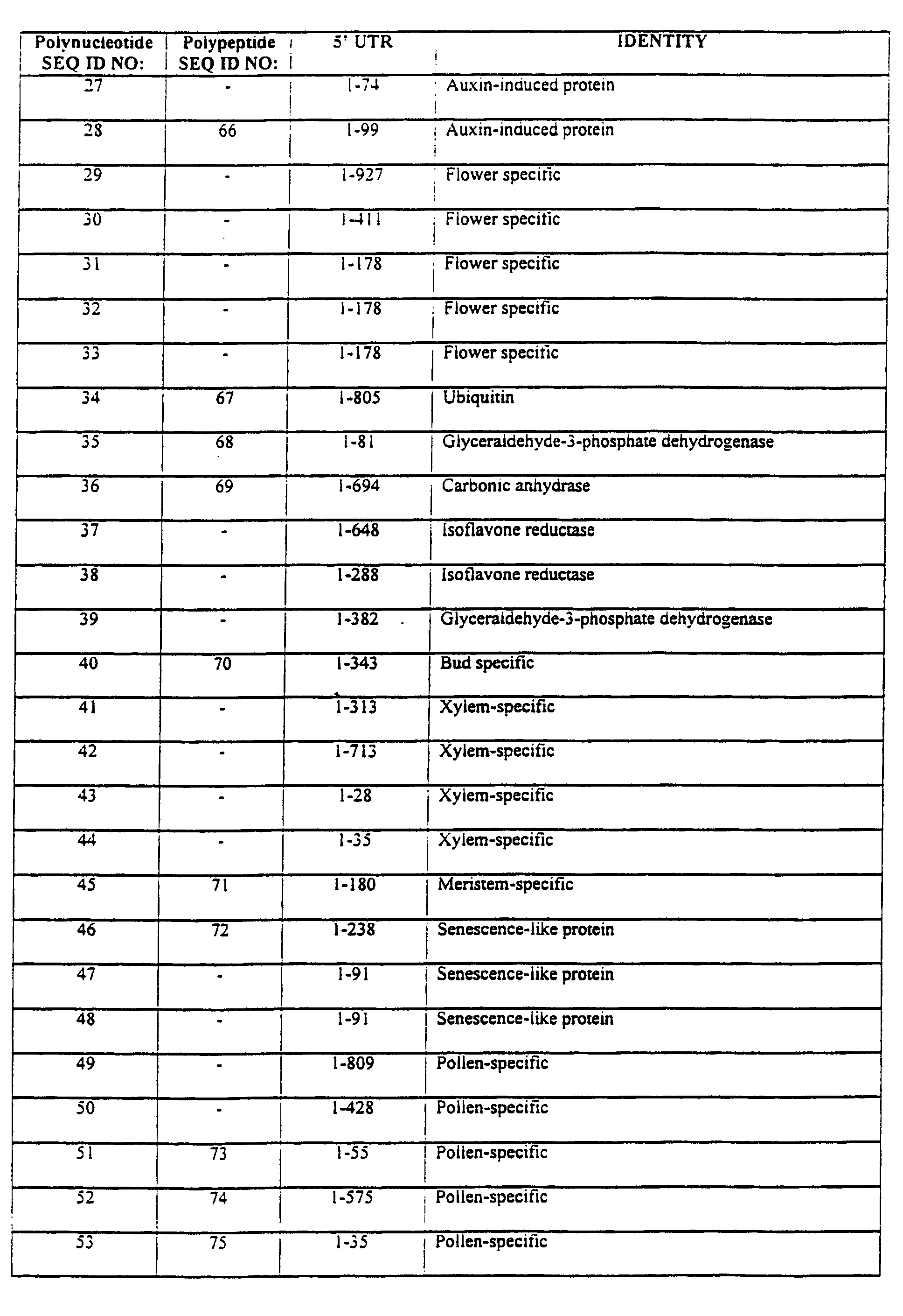

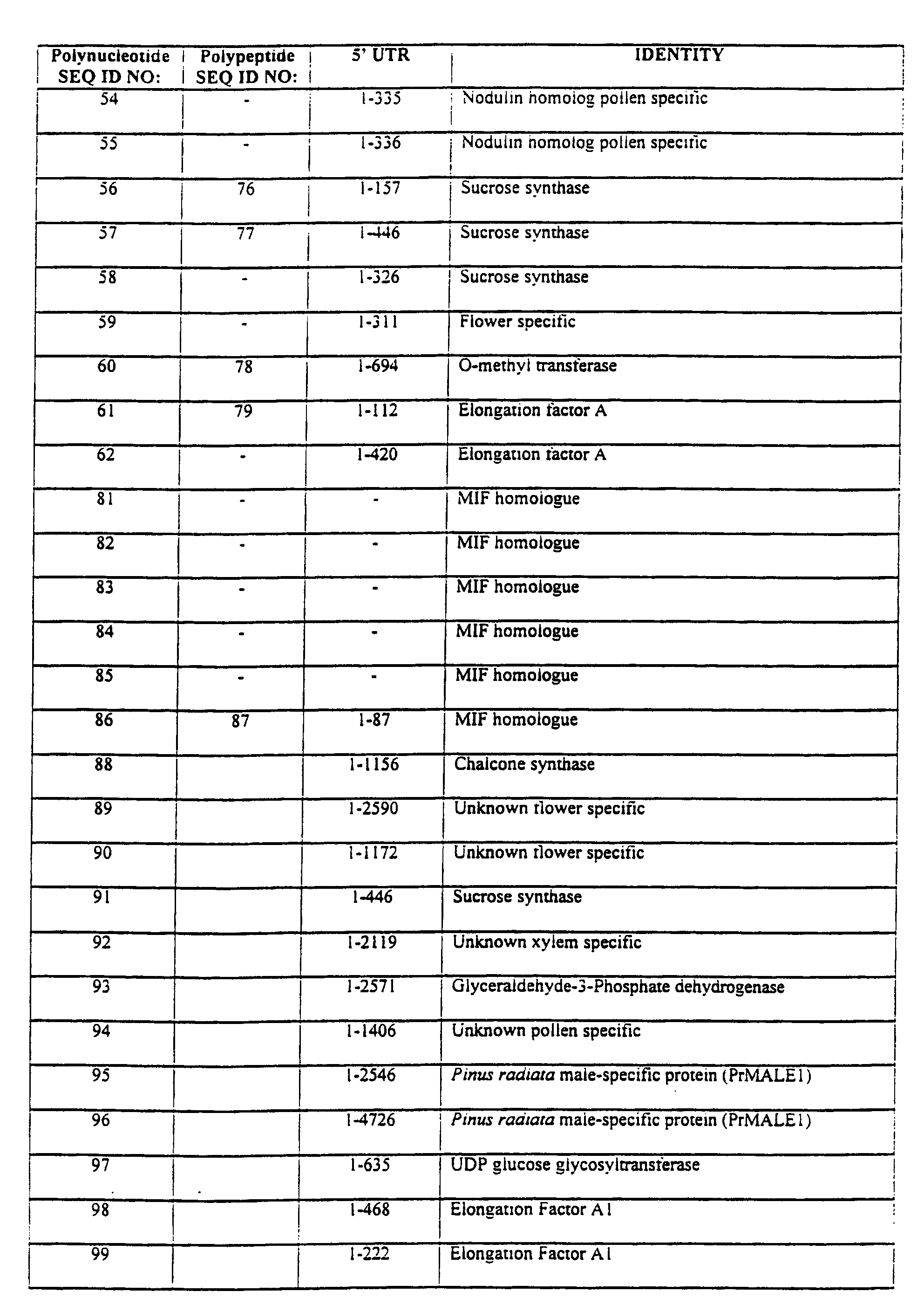

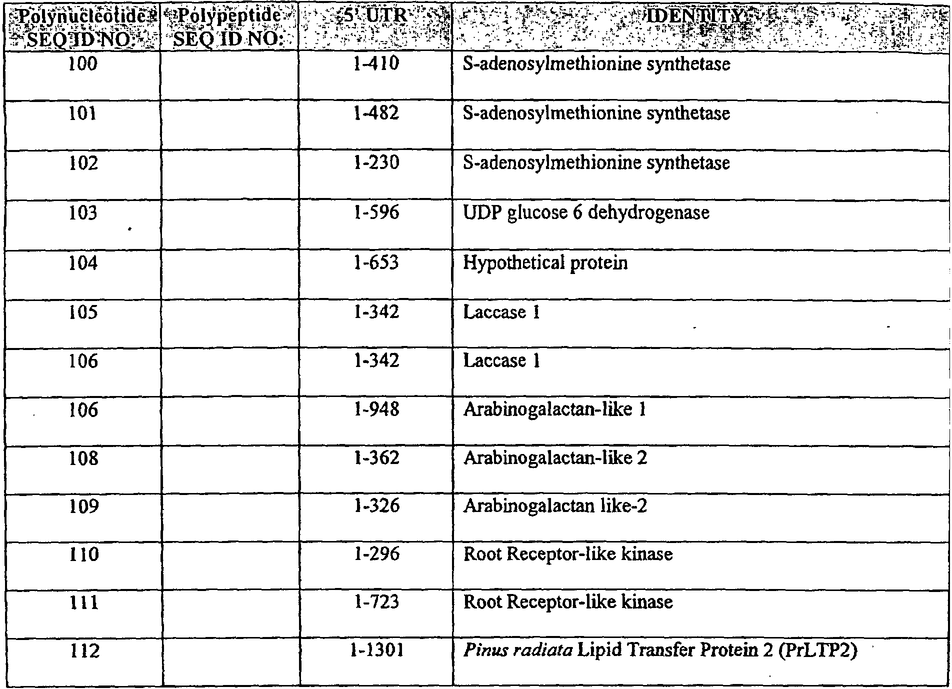

In one embodiment, the present invention relates to polynucleotide sequences isolated from *Pinus radiata*. The full-length sequence of the ubiquitin polynucleotide isolated from *Pinus radiata* is provided in SEQ ID NO: 1, with the sequence of the promoter region including an intron being provided in SEQ ID NO: 2 and the sequence of the promoter region excluding the intron being provided in SEQ ID NO: 3. The sequence of the ubiquitin polynucleotide isolated from *Eucalyptus grandis* is provided in SEQ ID NO: 34.

The term "polynucleotide(s)," as used herein, means a single or double-stranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments. Anti-sense polynucleotides and techniques involving anti-sense polynucleotides are well known in the art and are described, for example, in Robinson-Benion *et al.* "Antisense techniques," *Methods in Enzymol.* 254(23):363-375, 1995; and Kawasaki *et al.,* in *Artific. Organs* 20(8):836-848, 1996.

All of the polynucleotides and polypeptides described herein are isolated and purified, as those terms are commonly used in the art. Preferably, the polypeptides and polynucleotides are at least about 80% pure, more preferably at least about 90% pure, and most preferably at least about 99% pure.

The definition of the terms "complement", "reverse complement" and "reverse sequence", as used herein, is best illustrated by the following example. For the sequence 5' AGGACC 3', the complement, reverse complement and reverse sequence are as follows:

| | |
|---|---|
| Complement | 3' TCCTGG 5' |
| Reverse complement | 3' GGTCCT 5' |
| Reverse sequence | 5' CCAGGA 3' |

Some of the polynucleotides disclosed herein are "partial" sequences, in that they do not represent a full length gene encoding a full length polypeptide. Such partial sequences may be extended by analyzing and sequencing various DNA libraries using primers and/or probes and well known hybridization and/or PCR techniques. Partial sequences may be extended until an open reading frame encoding a polypeptide, a full-length polynucleotide and/or gene capable of expressing a polypeptide, or another useful portion of the genome is identified. Such extended sequences, including full-length polynucleotides and genes, are described as "corresponding to" a sequence identified as one of the sequences of SEQ ID NO: 2 and 3, or a variant thereof, or a portion of one of the sequences of SEQ ID NO: 2 and 3, or a variant thereof, when the extended polynucleotide comprises an identified sequence or its variant, or an identified contiguous portion (*x*-mer) of one of the sequences of SEQ ID NO: 2 and 3, or a variant thereof. Such extended polynucleotides may have a length of from about 50 to about 4,000 nucleic acids or base pairs, and preferably have a length of less than about 4,000 nucleic acids or base pairs, more preferably yet a length of less than about 3,000 nucleic acids or base pairs, more preferably yet a length of less than about 2,000 nucleic acids or base pairs. Under some circumstances, extended polynucleotides of the present invention may have a length of less than about 1,800 nucleic acids or base pairs, preferably less than about 1,600 nucleic acids or base pairs, more preferably less than about 1,400 nucleic acids or base pairs, more preferably yet less than about 1,200 nucleic acids or base pairs, and most preferably less than about 1,000 nucleic acids or base pairs.

Similarly, RNA sequences, reverse sequences, complementary sequences, antisense sequences, and the like, corresponding to the polynucleotides of the present invention, may be routinely ascertained and obtained using the cDNA sequences identified as SEQ ID NO: 2 and 3.

The polynucleotides identified as SEQ ID NO: 2 and 3, may contain open reading frames ("ORFs") or partial open reading frames encoding polypeptides. Additionally, open reading frames encoding polypeptides may be identified in extended or full length sequences corresponding to the sequences set out as SEQ ID NO: 2 and 3. Open reading frames may be identified using techniques that are well known in the art. These techniques include, for example, analysis for the location of known start and stop codons, most likely reading frame identification based on codon frequencies, etc. Suitable tools and software for ORF analysis are available, for example, on the Internet at http://www.ncbi.nlm.nih.gov/gorf/gorf.html. Open reading frames and portions of open reading frames may be identified in the polynucleotides of the present invention. Once a partial open reading frame is identified, the polynucleotide may be extended in the area of the partial open reading frame using techniques that are well known in the art until the polynucleotide for the full open reading frame is identified. Thus, open reading frames encoding polypeptides may be identified using the polynucleotides of the present invention.

Once open reading frames are identified in the polynucleotides of the present invention, the open reading frames may be isolated and/or synthesized. Expressible genetic constructs comprising the open reading frames and suitable promoters, initiators, terminators, etc., which are well known in the art, may then be constructed. Such genetic constructs may be introduced into a host cell to express the polypeptide encoded by the open reading frame. Suitable host cells may include various prokaryotic and eukaryotic cells, including plant cells, mammalian cells, bacterial cells, algae and the like.

Polypeptides encoded by the polynucleotides of the present invention may be expressed and used in various assays to determine their biological activity. Such polypeptides may be used to raise antibodies, to isolate corresponding interacting proteins or other compounds, and to quantitatively determine levels of interacting proteins or other compounds.

The term "polypeptide", as used herein, encompasses amino acid chains of any length including full length proteins, wherein amino acid residues are linked by covalent peptide bonds. Polypeptides of the present invention may be naturally purified products, or may be produced partially or wholly using recombinant techniques. The term "polypeptide encoded by a polynucleotide" as used herein, includes polypeptides encoded by a nucleotide sequence which includes the partial isolated DNA sequences of the present invention.

If desired, polypeptides may be prepared that comprise at least a functional portion of a sequence SEQ ID NO: 80. As used herein, the "functional portion" of a polypeptide is that portion which contains the active site essential for affecting the function of the polypeptide, for example, the portion of the molecule that is capable of binding one or more reactants. The active site may be made up of separate portions present on one or more polypeptide chains and will generally exhibit high binding affinity.

Functional portions of a polypeptide may be identified by first preparing fragments of the polypeptide by either chemical or enzymatic digestion of the polypeptide, or by mutation analysis of the polynucleotide that encodes the polypeptide and subsequent expression of the resulting mutant polypeptides. The polypeptide fragments or mutant polypeptides are then tested to determine which portions retain biological activity, using, for example, the representative assays provided below.

A functional portion comprising an active site may be made up of separate portions present on one or more polypeptide chains and generally exhibits high substrate specificity. The term "polypeptide encoded by a polynucleotide" as used herein, includes polypeptides encoded by a polynucleotide comprising a partial isolated polynucleotide of the present invention.

Portions and other variants of the inventive polypeptides may also be generated by synthetic or recombinant means. Synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. (Merrifield, *J. Am. Chem. Soc.* 85: 2149-2154, 1963). Equipment for automated synthesis of polypeptides is commercially available from suppliers such as Perkin Elmer / Applied Biosystems, Inc. (Foster City, California), and may be operated according to the manufacturer's instructions. Variants of a native polypeptide may be prepared using standard mutagenesis techniques, such as oligonucleotide-directed site-specific mutagensis (Kunkel, *Proc. Natl. Acad. Sci. USA* 82: 488-492, 1985). Sections of DNA sequences may also be removed using standard techniques to permit preparation of truncated polypeptides.

As used herein, the term "variant" comprehends nucleotide or amino acid sequences different from the specifically identified sequences, wherein one or more nucleotides or amino acid residues is deleted, substituted, or added. Variants may be naturally occurring allelic variants, or non-naturally occurring variants. Variant sequences exhibit at least 75%, and most preferably at least 90% identity to a sequence of the present invention wherein the variant sequence is able to regulate transcription of a DNA sequence of interest. The percentage identity is determined by aligning the two sequences to be compared as described below, determining the number of identical residues in the aligned portion, dividing that number by the total number of residues in the inventive (queried) sequence, and multiplying the result by 100.

Polynucleotide and polypeptide sequences may be aligned, and percentage of identical nucleotides in a specified region may be determined against another polynucleotide, using computer algorithms that are publicly available. Two exemplary algorithms for aligning and identifying the similarity of polynucleotide sequences are the BLASTN and FASTA algorithms. Polynucleotides may also be analyzed using the BLASTX algorithm, which compares the six-frame conceptual translation products of a nucleotide query sequence (both strands) against a protein sequence database. The similarity of polypeptide sequences may be examined using the BLASTP algorithm. The BLASTN, BLASTX and BLASTP programs are available on the NCBI anonymous FTP server (ftp://ncbi.nlm.nih.gov) under /blast/executables/. The BLASTN algorithm version 2.0.4 [Feb-24-1998] and version 2.0.6 [Sept-16-1998], set to the default parameters described in the documentation and distributed with the algorithm, are preferred for use in the determination of polynucleotide variants according to the present invention. The BLASTP algorithm, is preferred for use in the determination of polypeptide variants according to the present invention. The use of the BLAST family of algorithms, including BLASTN, BLASTP, and BLASTX, is described at NCBI's website at URL http://www.ncbi.nlm.nih.gov/BLAST/newblast.html and in the publication of Altschul, *et al*., "Gapped BLAST and PSI-BLAST: a new generation of protein database search programs," *Nucleic Acids Res.* 25: 3389-3402, 1997.

The computer algorithm FASTA is available on the Internet at the ftp site ftp://ftp.virginia.edu/pub/fasta/. Version 2.0u4, February 1996, set to the default parameters described in the documentation and distributed with the algorithm, may be used in the determination of variants according to the present invention. The use of the FASTA algorithm is described in Pearson and Lipman, "Improved Tools for Biological Sequence Analysis," *Proc. Natl. Acad. Sci. USA* 85: 2444-2448, 1988; and Pearson, "Rapid and Sensitive Sequence Comparison with FASTP and FASTA," *Methods in Enzymol.* 183: 63-98, 1990.

The following running parameters are preferred for determination of alignments and similarities using BLASTN that contribute to the E values and percentage identity for polynucleotide sequences: Unix running command: blastall -p blastn -d embldb -e 10 -G0 - E0 -r 1. -v 30 -b 30 -i queryseq -o results; the parameters are: -p Program Name [String]; - d Database [String]; -e Expectation value (E) [Real]; -G Cost to open a gap (zero invokes default behavior) [Integer]; -E Cost to extend a gap (zero invokes default behavior) [Integer]; -r Reward for a nucleotide match (BLASTN only) [Integer]; -v Number of one-line descriptions (V) [Integer]; -b Number of alignments to show (B) [Integer]; -i Query File [File In]; and -o BLAST report Output File [File Out] Optional.

The following running parameters are preferred for determination of alignments and similarities using BLASTP that contribute to the E values and percentage identity of polypeptide sequences: blastall -p blastp -d swissprotdb -e 10 -G 0 -E 0 -v 30 -b 30 -i queryseq -o results; the parameters are: -p Program Name [String]; -d Database [String]; -e Expectation value (E) [Real]; -G Cost to open a gap (zero invokes default behavior) [Integer]; -E Cost to extend a gap (zero invokes default behavior) [Integer]; -v Number of one-line descriptions (v) [Integer]; -b Number of alignments to show (b) [Integer]; -I Query File [File In]; -o BLAST report Output File [File Out] Optional.

The "hits" to one or more database sequences by a queried sequence produced by BLASTN, FASTA, BLASTP or a similar algorithm, align and identify similar portions of sequences. The hits are arranged in order of the degree of similarity and the length of sequence overlap. Hits to a database sequence generally represent an overlap over only a fraction of the sequence length of the queried sequence.

The BLASTN, FASTA and BLASTP algorithms also produce "Expect" values for alignments. The Expect value (E) indicates the number of hits one can "expect" to see over a certain number of contiguous sequences by chance when searching a database of a certain size. The Expect value is used as a significance threshold for determining whether the hit to a database, such as the preferred EMBL database, indicates true similarity. For example, an E value of 0.1 assigned to a polynucleotide hit is interpreted as meaning that in a database of the size of the EMBL database, one might expect to see 0.1 matches over the aligned portion of the sequence with a similar score simply by chance. By this criterion, the aligned and matched portions of the polynucleotide sequences then have a probability of 90% of being the same. For sequences having an E value of 0.01 or less over aligned and matched portions, the probability of finding a match by chance in the EMBL database is 1% or less using the BLASTN or FASTA algorithm.

According to one embodiment, "variant" polynucleotides, with reference to each of the polynucleotides of the present invention, preferably comprise sequences having the same number or fewer nucleic or amino acids than each of the polynucleotides or polypeptides of the present invention and producing an E value of 0.01 or less when compared to the polynucleotide or polypeptide of the present invention. That is, a variant polynucleotide is any sequence that has at least a 99% probability of being the same as the polynucleotide or polypeptide of the present invention, measured as having an E value of 0.01 or less using the BLASTN, FASTA, or BLASTP algorithms set at parameters described above. According to a preferred embodiment, a variant polynucleotide is a sequence having the same number or fewer nucleic acids than a polynucleotide of the present invention that has at least a 99% probability of being the same as the polynucleotide of the present invention, measured as having an E value of 0.01 or less using the BLASTN or FASTA algorithms set at parameters described above.

Variant polynucleotides of the present invention may hybridize to the polynucleotide sequences recited in SEQ ID NO: 2 and 3, or complements, reverse sequences, or reverse complements of those sequences under stringent conditions. As used herein, "stringent conditions" refers to prewashing in a solution of 6X SSC, 0.2% SDS; hybridizing at 65°C, 6X SSC, 0.2% SDS overnight; followed by two washes of 30 minutes each in 1X SSC, 0.1% SDS at 65° C and two washes of 30 minutes each in 0.2X SSC, 0.1% SDS at 65°C.

In certain embodiments, variants of the inventive polynucleotides possess biological activities that are the same or similar to those of the inventive polypeptides or polynucleotides. Such variant polynucleotides function as promoter sequences and are thus capable of modifying gene expression in a plant.

The polynucleotides of the present invention may be isolated from various libraries, or may be synthesized using techniques that are well known in the art. The polynucleotides may be synthesized, for example, using automated oligonucleotide synthesizers (*e.g*., Beckman Oligo 1000M DNA Synthesizer) to obtain polynucleotide segments of up to 50 or more nucleic acids. A plurality of such polynucleotide segments may then be ligated using standard DNA manipulation techniques that are well known in the art of molecular biology. One conventional and exemplary polynucleotide synthesis technique involves synthesis of a single stranded polynucleotide segment having, for example, 80 nucleic acids, and hybridizing that segment to a synthesized complementary 85 nucleic acid segment to produce a 5 nucleotide overhang. The next segment may then be synthesized in a similar fashion, with a 5 nucleotide overhang on the opposite strand. The "sticky" ends ensure proper ligation when the two portions are hybridized. In this way, a complete polynucleotide of the present invention may be synthesized entirely *in vitro.*

If desired polynucleotides comprising at least a specified number of contiguous residues (*x*-mers) of any of the polynucleotides identified as SEQ ID NO: 2 and 3, complements, reverse sequences, and reverse complements of such sequences, and their variants may be prepared. Similarly, polypeptides comprising at least a specified number of contiguous residues (*x*-mers) of the polypeptide, identified as SEQ ID NO: 80 may be prepared. As used herein, the term "*x*-mer," with reference to a specific value of "*x*," refers to a sequence comprising at least a specified number ("*x*") of contiguous residues of any of the polynucleotides identified as SEQ ID NO: 2 and 3. The value of *x* is preferably at least 20, more preferably at least 40, more preferably yet at least 60, and most preferably at least 80. Thus, polynucleotides and polypeptides of the present invention comprise a 20-mer, a 40-mer, a 60-mer, an 80-mer, a 100-mer, a 120-mer, a 150-mer, a 180-mer, a 220-mer, a 250-mer, a 300-mer, 400-mer, 500-mer or 600-mer of a polynucleotide identified as SEQ ID NO: 2 and 3, and variants thereof.

As noted above, the inventive polynucleotide promoter sequences may be employed in genetic constructs to drive transcription and/or expression of a polynucleotide of interest. The polynucleotide of interest may be either endogenous or heterologous to an organism, for example a plant, to be transformed. The inventive genetic constructs may thus be employed to modulate levels of transcription and/or expression of a polynucleotide, for example gene, that is present in the wild-type plant, or may be employed to provide transcription and/or expression of a DNA sequence that is not found in the wild-type plant.

In certain embodiments, the polynucleotide of interest comprises an open reading frame that encodes a target polypeptide. The open reading frame is inserted in the genetic construct in either a sense or antisense orientation, such that transformation of a target plant with the genetic construct will lead to a change in the amount of polypeptide compared to the wild-type plant. Transformation with a genetic construct comprising an open reading frame in a sense orientation will generally result in over-expression of the selected polypeptide, while transformation with a genetic construct comprising an open reading frame in an antisense orientation will generally result in reduced expression of the selected polypeptide. A population of plants transformed with a genetic construct comprising an open reading frame in either a sense or antisense orientation may be screened for increased or reduced expression of the polypeptide in question using techniques well known to those of skill in the art, and plants having the desired phenotypes may thus be isolated.

Alternatively, expression of a target polypeptide may be inhibited by inserting a portion of the open reading frame, in either sense or antisense orientation, in the genetic construct. Such portions need not be full-length but preferably comprise at least 25 and more preferably at least 50 residues of the open reading frame. A much longer portion or even the full length DNA corresponding to the complete open reading frame may be employed. The portion of the open reading frame does not need to be precisely the same as the endogenous sequence, provided that there is sufficient sequence similarity to achieve inhibition of the target gene. Thus a sequence derived from one species may be used to inhibit expression of a gene in a different species.

In further embodiments, the inventive DNA constructs comprise a DNA sequence including an untranslated, or non-coding, region of a gene coding for a target polypeptide, or a DNA sequence complementary to such an untranslated region. Examples of untranslated regions which may be usefully employed in such constructs include introns and 5'-untranslated leader sequences. Transformation of a target plant with such a DNA construct may lead to a reduction in the amount of the polypeptide expressed in the plant by the process of cosuppression, in a manner similar to that discussed, for example, by *Napoli et al., Plant Cell* 2:279-290, 1990 and de Carvalho Niebel *et al., Plant Cell* 7:347-358, 1995.

Alternatively, regulation of polypeptide expression can be achieved by inserting appropriate sequences or subsequences (e.g. DNA or RNA) in ribozyme constructs (McIntyre and Manners, *Transgenic Res.* 5(4):257-262, 1996). Ribozymes are synthetic RNA molecules that comprise a hybridizing region complementary to two regions, each of which comprises at least 5 contiguous nucleotides in a mRNA molecule encoded by one of the inventive polynucleotides. Ribozymes possess highly specific endonuclease activity, which autocatalytically cleaves the mRNA.

The polynucleotide of interest is operably linked to a polynucleotide promoter sequence of the present invention such that a host cell is able to transcribe an RNA from the promoter sequence linked to the DNA sequence of interest. The gene promoter sequence is generally positioned at the 5' end of the DNA sequence to be transcribed. Use of a constitutive promoter, such as the *Pinus radiata* ubiquitin polynucleotide promoter sequence of SEQ ID NO: 2 and 3, will affect transcription of the DNA sequence of interest in all parts of the transformed plant. With genetic constructs employing inducible gene promoter sequences, the rate of DNA transcription can be modulated by external stimuli, such as light, heat, anaerobic stress, alteration in nutrient conditions and the like.

The inventive genetic constructs further comprise a gene termination sequence which is located 3' to the polynucleotide of interest. A variety of gene termination sequences which may be usefully employed in the genetic constructs of the present invention are well known in the art. One example of such a gene termination sequence is the 3' end of the *Agrobacterium tumefaciens* nopaline synthase gene. The gene termination sequence may be endogenous to the target plant or may be exogenous, provided the promoter is functional in the target plant. For example, the termination sequence may be from other plant species, plant viruses, bacterial plasmids and the like.

The genetic constructs of the present invention may also contain a selection marker that is effective in cells of the target organism, such as a plant, to allow for the detection of transformed cells containing the inventive construct. Such markers, which are well known in the art, typically confer resistance to one or more toxins. One example of such a marker is the NPTII gene whose expression results in resistance to kanamycin or hygromycin, antibiotics which are usually toxic to plant cells at a moderate concentration (Rogers *et al*., *in* Weissbach A and H, eds. *Methods for Plant Molecular Biology,* Academic Press Inc.: San Diego, CA, 1988). Transformed cells can thus be identified by their ability to grow in media containing the antibiotic in question. Alternatively, the presence of the desired construct in transformed cells can be determined by means of other techniques well known in the art. such as Southern and Western blots.

Techniques for operatively linking the components of the inventive DNA constructs are well known in the art and include the use of synthetic linkers containing one or more restriction endonuclease sites as described, for example, by Sambrook *et al*., (*Molecular cloning: a laboratory manual,* CSHL Press: Cold Spring Harbor, NY, 1989). The genetic construct of the present invention may be linked to a vector having at least one replication system, for example *E. coli,* whereby after each manipulation, the resulting construct can be cloned and sequenced and the correctness of the manipulation determined.

The genetic constructs of the present invention may be used to transform a variety of target plants. Plants which may be transformed using the inventive constructs include both monocotyledonous angiosperms (*e.g.,* grasses, corn, grains, oat, wheat and barley) and dicotyledonous angiosperms (*e.g., Arabidopsis,* tobacco, legumes, alfalfa, oaks, eucalyptus, maple), and Gymnosperms *(e.g.,* Scots pine; *see* Aronen, *Finnish Forest Res. Papers,* Vol. 595, 1996), white spruce (Ellis *et al., Biotechnology* 11:84-89, 1993), and larch (Huang *et al., In Vitro Cell* 27:201-207, 1991). In a preferred embodiment, the inventive genetic constructs are employed to transform woody plants, herein defined as a tree or shrub whose stem lives for a number of years and increases in diameter each year by the addition of woody tissue. Preferably the target plant is selected from the group consisting of eucalyptus and pine species, most preferably from the group consisting of *Eucalyptus grandis* and *Pinus radiata*. Other species which may be usefully transformed with the DNA constructs of the present invention include, but are not limited to: pines such as *Pinus banksiana, Pinus brutia, Pinus caribaea, Pinus clausa, Pinus contorta, Pinus coulteri, Pinus echinata, Pinus eldarica, Pinus ellioti, Pinus jeffreyi, Pinus lambertiana, Pinus monticola, Pinus nigra, Pinus palustrus, Pinus pinaster, Pinus ponderosa, Pinus resinosa, Pinus rigida, Pinus serotina, Pinus strobus, Pinus sylvestris, Pinus taeda, Pinus virginiana;* other gymnosperms, such as *Abies amabilis, Abies balsamea, Abies concolor, Abies grandis, Abies lasiocarpa, Abies magnifica, Abies procera, Chamaecyparis lawsoniona, Chamaecyparis nootkatensis, Chamaecyparis thyoides, Huniperus virginiana, Larix decidua, Larix laricina, Larix leptolepis, Larix occidentalis, Larix siberica, Libocedrus decurrens, Picea abies, Picea engelmanni, Picea glauca, Picea mariana, Picea pungens, Picea rubens, Picea sitchensis, Pseudotsuga menziesii, Sequoia gigantea, Sequoia sempervirens, Taxodium distichum, Tsuga canadensis, Tsuga heterophylla, Tsuga mertensiana, Thuja occidentalis,* *Thuja plicata*; and Eucalypts, such as *Eucalyptus alba, Eucalyptus bancroftii, Eucalyptus botyroides, Eucalyptus bridgesiana. Eucalyptus calophylla, Eucalyptus camaldulensis, Eucalyptus citriodora, Eucalyptus cladocalyx, Eucalyptus coccifera, Eucalyptus curtisii, Eucalyptus dalrympleana, Eucalyptus deglupta, Eucalyptus delagatensis, Eucalyptus diversicolor, Eucalyptus dunnii, Eucalyptus ficifolia, Eucalyptus globulus, Eucalyptus gomphocephala, Eucalyptus gunnii, Eucalyptus henryi, Eucalyptus laevopinea, Eucalyptus macarthurii, Eucalyptus macrorhyncha, Eucalyptus maculata, Eucalyptus marginata, Eucalyptus megacarpa, Eucalyptus melliodora, Eucalyptus nicholii, Eucalyptus nitens, Eucalyptus nova-anglica. Eucalyptus obliqua, Eucalyptus obtusiflora, Eucalyptus oreades, Eucalyptus pauciflora, Eucalyptus polybractea, Eucalyptus regnans, Eucalyptus resinifera, Eucalyptus robusta, Eucalyptus rudis, Eucalyptus saligna, Eucalyptus sideroxylon, Eucalyptus stuartiana, Eucalyptus tereticornis, Eucalyptus torelliana. Eucalyptus urnigera, Eucalyptus urophylla, Eucalyptus viminalis, Eucalyptus viridis, Eucalyptus wandoo* and *Eucalyptus youmanni;* and hybrids of any of these species.

Techniques for stably incorporating genetic constructs into the genome of target plants are well known in the art and include *Agrobacterium tumefaciens* mediated introduction, electroporation, protoplast fusion, injection into reproductive organs, injection into immature embryos, high velocity projectile introduction and the like. The choice of technique will depend upon the target plant to be transformed. For example, dicotyledonous plants and certain monocots and gymnosperms may be transformed by *Agrobacterium* Ti plasmid technology, as described, for example by Bevan, *Nucleic Acids Res.* 12:8711-8721, 1984. Targets for the introduction of the DNA constructs of the present invention include tissues, such as leaf tissue, dissociated cells, protoplasts, seeds, embryos, meristematic regions; cotyledons, hypocotyls, and the like. The preferred method for transforming eucalyptus and pine is a biolistic method using pollen (see, for example, Aronen, *Finnish Forest Res. Papers,* Vol. 595, 53pp, 1996) or easily regenerable embryonic tissues.

Once the cells are transformed, cells having the inventive genetic construct incorporated in their genome may be selected by means of a marker, such as the kanamycin resistance marker discussed above. Transgenic cells may then be cultured in an appropriate medium to regenerate whole plants, using techniques well known in the art. In the case of protoplasts, the cell wall is allowed to reform under appropriate osmotic conditions. In the case of seeds or embryos, an appropriate germination or callus initiation medium is employed. For explants, an appropriate regeneration medium is used. Regeneration of plants is well established for many species. For a review of regeneration of forest trees see Dunstan *et al*., "Somatic embryogenesis in woody plants," *in* Thorpe TA, ed., *In Vitro Embryogenesis of Plants (Current Plant Science and Biotechnology in Agriculture Vol. 20),* Chapter 12, pp. 471-540, 1995. Specific protocols for the regeneration of spruce are discussed by Roberts *et al*., "Somatic embryogenesis of spruce," *in* Redenbaugh K, ed., *Synseed: applications of synthetic seed to crop improvement,* CRC Press: Chapter 23, pp. 427-449, 1993). Transformed plants having the desired phenotype may be selected using techniques well known in the art. The resulting transformed plants may be reproduced sexually or asexually, using methods well known in the art, to give successive generations of transgenic plants.

As discussed above, the production of RNA in target cells can be controlled by choice of the promoter sequence, or by selecting the number of functional copies or the site of integration of the polynucleotides incorporated into the genome of the target host. A target organism may be transformed with more than one genetic construct of the present invention, thereby modulating the activity of more than gene. Similarly, a genetic construct may be assembled containing more than one open reading frame coding for a polypeptide of interest or more than one untranslated region of a gene coding for such a polypeptide.

The isolated polynucleotides of the present invention also have utility in genome mapping, in physical mapping, and in positional cloning of genes. As detailed below, the polynucleotide sequences identified as SEQ ID NO: 2 and 3, and their variants, may be used to design oligonucleotide probes and primers. Oligonucleotide probes designed using the polynucleotides of the present invention may be used to detect the presence and examine the expression patterns of genes in any organism having sufficiently similar DNA and RNA sequences in their cells using techniques that are well known in the art, such as slot blot DNA hybridization techniques. Oligonucleotide primers designed using the polynucleotides of the present invention may be used for PCR amplifications. Oligonucleotide probes and primers designed using the polynucleotides of the present invention may also be used in connection with various microarray technologies, including the microarray technology of Synteni (Palo Alto, California).

As used herein, the term "oligonucleotide" refers to a relatively short segment of a polynucleotide sequence, generally comprising between 6 and 60 nucleotides, and comprehends both probes for use in hybridization assays and primers for use in the amplification of DNA by polymerase chain reaction.

An oligonucleotide probe or primer is described as "corresponding to" a polynucleotide of the present invention, including one of the sequences set out as SEQ ID NO: 2 and 3, or a variant, if the oligonucleotide probe or primer, or its complement, is contained within one of the sequences set out as SEQ ID NO: 2 and 3, or a variant of one of the specified sequences. Such oligonucleotide probes and primers are substantially complementary to a polynucleotide disclosed herein.

Two single stranded sequences are said to be substantially complementary when the nucleotides of one strand, optimally aligned and compared, with the appropriate nucleotide insertions and/or deletions, pair with at least 80%, preferably at least 90% to 95% and more preferably at least 98% to 100% of the nucleotides of the other strand. Alternatively, substantial complementarity exists when a first DNA strand will selectively hybridize to a second DNA strand under stringent hybridization conditions. Stringent hybridization conditions for determining complementarity include salt conditions of less than about 1 M, more usually less than about 500 mM, and preferably less than about 200 mM. Hybridization temperatures can be as low as 5°C, but are generally greater than about 22°C, more preferably greater than about 30°C, and most preferably greater than about 37°C. Longer DNA fragments may require higher hybridization temperatures for specific hybridization. Since the stringency of hybridization may be affected by other factors such as probe composition, presence of organic solvents and extent of base mismatching, the combination of parameters is more important than the absolute measure of any one alone.

Oligonucleotide probes and/or primers may comprise at least about 6 contiguous residues, more preferably at least about 10 contiguous residues, and most preferably at least about 20 contiguous residues complementary to a polynucleotide sequence of the present invention. Such probes and primers may be from about 8 to 100 base pairs in length or, preferably from about 10 to 50 base pairs in length or, more preferably from about 15 to 40 base pairs in length. The probes can be easily selected using procedures well known in the art, taking into account DNA-DNA hybridization stringencies, annealing and melting temperatures, and potential for formation of loops and other factors, which are well known in the art. Tools and software suitable for designing probes, and especially suitable for designing PCR primers, are available on the Internet, for example, at URL http://www.horizonp.ress.com/pcr/. Preferred techniques for designing PCR primers are also disclosed in Dieffenbach, CW and Dyksler, GS. *PCR Primer: a laboratory manual,* CSHL Press: Cold Spring Harbor, NY, 1995.

A plurality of oligonucleotide probes or primers corresponding to a polynucleotide of the present invention may be provided in a kit form. Such kits generally comprise multiple DNA or oligonucleotide probes, each probe being specific for a polynucleotide sequence. Kits of the present invention may comprise one or more probes or primers corresponding to a polynucleotide of the present invention, including a polynucleotide sequence identified in SEQ ID NO: 2 and 3.

In one kit format useful for high-throughput assays, the oligonucleotide probe kits comprise multiple probes in an array format, wherein each probe is immobilized at a predefined, spatially addressable location on the surface of a solid substrate. Array formats which may be usefully employed in the present invention are disclosed, for example, in U.S. Patents No. 5,412,087 and 5,545,451; and PCT Publication No. WO 95/00450.

The polynucleotides of the present invention may also be used to tag or identify a plant or reproductive material therefrom. Such tagging may be accomplished, for example, by stably introducing a non-disruptive non-functional heterologous polynucleotide identifier into an organism, the polynucleotide comprising one of the polynucleotides of the present invention.

The following examples are offered by way of illustration and not by way of limitation.

### EXAMPLE 1

### Isolation and Characterization of a Ubiquitin Gene Promoter from Pinus radiata

*Pinus radiata* cDNA expression libraries were constructed and screened as follows. mRNA was extracted from plant tissue using the protocol of Chang *et al., Plant Molecular Biology Reporter* 11:113-116, 1993 with minor modifications. Specifically, samples were dissolved in CPC-RNAXB (100 mM Tris-Cl, pH 8,0; 25 mM EDTA; 2.0 M NaCl; 2%CTAB; 2% PVP and 0.05% Spermidine*3HCl) and extracted with chloroform:isoamyl alcohol, 24:1. mRNA was precipitated with ethanol and the total RNA preparate was purified using a Poly(A) Quik mRNA Isolation Kit (Stratagene, La Jolla, CA). A cDNA expression library was constructed from the purified mRNA by reverse transcriptase synthesis followed by insertion of the resulting cDNA clones in Lambda ZAP using a ZAP Express cDNA Synthesis Kit (Stratagene), according to the manufacturer's protocol. The resulting cDNAs were packaged using a Gigapack II Packaging Extract (Stratagene) employing 1 µl of sample DNA from the 5 µl ligation mix. Mass excision of the library was done using XL1-Blue MRF' cells and XLOLR cells (Stratagene) with ExAssist helper phage (Stratagene). The excised phagemids were diluted with NZY broth (Gibco BRL, Gaithersburg, MD) and plated out onto LB-kanamycin agar plates containing X-gal and isopropylthio-beta-galactoside (IPTG).

Of the colonies plated and picked for DNA miniprep, 99% contained an insert suitable for sequencing. Positive colonies were cultured in NZY broth with kanamycin and cDNA was purified by means of alkaline lysis and polyethylene glycol (PEG) precipitation. Agarose gel at 1% was used to screen sequencing templates for chromosomal contamination. Dye primer sequences were prepared using a Turbo Catalyst 800 machine (Perkin Elmer/Applied Biosystems Division, Foster City, CA) according to the manufacturer's protocol.

DNA sequence for positive clones was obtained using a Perkin Elmer/Applied Biosystems Division Prism 377 sequencer. cDNA clones were sequenced first from the 5' end and, in some cases, also from the 3' end. For some clones, internal sequence was obtained using subcloned fragments. Subcloning was performed using standard procedures of restriction mapping and subcloning to pBluescript II SK+ vector.

As described below, one of the most abundant sequences identified was a ubiquitin gene, hereinafter referred to as the "Super-Ubiquitin" gene.

### Isolation of cDNA clones containing the ubiquitin gene

Sequences of cDNA clones with homology to the ubiquitin gene were obtained from high-throughput cDNA sequencing as described above. Sequences from several independent clones were assembled in a contig and a consensus sequence was generated from overlapping clones. The determined nucleotide sequence of the isolated Super Ubiquitin clone, comprising the promoter region (including an intron), coding region and 3' untranslated region (UTR) is provided in SEQ ID NO: 1. The 5' UTR is represented by residues 1 to 2064, the intron by residues 1196 to 2033, and the coding region of the gene, which contains three direct repeats, by residues 2065 to 2751. The 3' UTR is 328 residues long (residues 2755 to 3083). The nucleotide sequence of the Super Ubiquitin promoter region only, including the intron, is given in SEQ ID NO: 2. The nucleotide sequence of the Super Ubiquitin promoter region only, excluding the intron, is given in SEQ ID NO: 3. The predicted amino acid sequence for the *Pinus radiata* Super Ubiquitin is provided in SEQ ID NO: 80.

Ubiquitin proteins function as part of a protein degradation pathway, in which they covalently attach to proteins, thereby targeting them for degradation (for a review, see Belknap and Garbarino, *Trends in Plant Sciences* 1:331-335, 1996). The protein is produced from a precursor polypeptide, encoded by a single mRNA. The Super Ubiquitin mRNA contains three copies of the ubiquitin monomer.

### Cloning of the Super Ubiquitin Promoter

Fragments of the Super Ubiquitin promoter were cloned by two different PCR-based approaches.

### Method 1: Long Distance Gene Walking PCR

Using "Long Distance Gene Walking" PCR (Min and Powell, *Biotechniques* 24:398-400, 1998), a 2 kb fragment was obtained that contained the entire coding region of the ubiquitin gene, a 900 bp intron in the 5' UTR and approximately 100 bp of the promoter.

To generate this fragment, 2 nested primers were designed from the 3' UTR of the Super Ubiquitin cDNA sequence isolated from pine. Generally, the 5' UTR is used for primer design to amplify upstream sequence. However, the available 5' UTR of Super Ubiquitin was very short, and two initial primers derived from this region failed to amplify any fragments. Therefore, the primers of SEQ ID NO: 15 and 16 were designed from the 3' UTR.

The method involved an initial, linear PCR step with pine genomic DNA as template using the primer of SEQ ID NO: 15, and subsequent C-tailing of the single stranded DNA product using terminal transferase. The second PCR-step used these fragments as template for amplification with the primer of SEQ ID NO: 16 and primer AP of SEQ ID NO: 17. The AP primer was designed to bind to the polyC tail generated by the terminal transferase. Both primers (SEQ ID NO: 16 and 17) contained a 5'-*Not*I restriction site for the cloning of products into the *Not*I site of a suitable vector. The final PCR product contained fragments of different sizes. These fragments were separated by electrophoresis and the largest were purified from the gel, digested with restriction endonuclease *Not*I and cloned in the *Not*I site of expression vector pBK-CMV (Stratagene, La Jolla, CA). The largest of these clones contained the complete coding region of the gene (no introns were found in the coding sequence) and a 5' UTR which contained a 900 bp intron.

### Method 2: "Genome Walker" kit

The Super Ubiquitin gene promoter was cloned using a "Genome Walker" kit (Clontech, Palo Alto, CA). This is also a PCR-based method, which requires 2 PCR primers to be constructed, one of which must be gene-specific. Although the ubiquitin coding region is highly conserved, the 5' UTR from different ubiquitin genes is not conserved and could therefore be used to design a gene-specific primer. A 2.2 kb fragment was amplified and subcloned in pGEM-T-easy (Promega, Madison, WI). Analysis by PCR and DNA sequencing showed that the clone contained 5' UTR sequence of the Super Ubiquitin gene, including the 900 bp intron and approximately 1 kb of putative promoter region. An intron in the 5' UTR is a common feature of plant polyubiquitin genes and may be involved in determining gene expression levels.

The gene specific primers used for these PCR reactions are provided in SEQ ID NO: 18 and 19.

### Expression of Super Ubiquitin

Using primers derived from the gene-specific 5' and 3' UTR sequences, expression levels of Super Ubiquitin in different plant tissues was examined by means of RT-PCR. Super Ubiquitin was found to be expressed in all plant tissues examined, including branch phloem and xylem, feeder roots, fertilized cones, needles, one year old cones, pollen sacs, pollinated cones, root xylem, shoot buds, structural roots, trunk phloem and trunk. Expression of Super Ubiquitin in plant tissues was also demonstrated in a Northern blot assay using a PCR probe prepared from the 5'UTR.

### Functional analysis of the Super Ubiquitin Promoter

To test the function of the Super Ubiquitin promoter in plants, *Arabidopsis thaliana* were transformed with constructs containing the reporter gene for Green Fluorescent Protein (GFP) operably linked to either the Super Ubiquitin promoter of SEQ ID NO: 2 or SEQ ID NO: 3 (i.e., either with or without the intron). Constructs lacking a promoter were used as a negative control, with a plant T-DNA vector carrying a CaMV 35s promoter cloned in front of GFP being used as a positive control. The constructs were introduced into *Arabidopsis* via *Agrobacterium*-mediated transformation.

All the plant culture media were according to the protocol of Valvekens and Van Montagu, *Proc. Natl. Acad. Sci. USA* 85:5536-5540, 1988 with minor modifications. For root transformation, sterilized seeds were placed in a line on the surface of germination medium, the plates were placed on their sides to facilitate root harvesting, and the seeds were grown for two weeks at 24°C with 16 h photoperiod.

Expression of the constructs was measured by determining expression levels of the reporter gene for Green Fluorescent Protein (GFP). Preliminary GFP expression (transient) was detected in early transgenic roots during T-DNA transfer. Transgenic roots that developed green callus, growing on shoot-inducing medium containing 50 µg/ml Kanamycin and 100 µg/ml Timentin, were further tested for GFP expression. After several weeks of stringent selection on Kanamycin medium, several independent transgenic *Arabidopsis* lines were engineered and tested for GFP expression.

Expression was seen both with the Super Ubiquitin promoter including intron and the Super Ubiquitin promoter without the intron. However, preliminary results indicated that the levels of expression obtained with the Super Ubiquitin intron-less promoter construct were significantly higher than those seen with the promoter including intron, suggesting that the intron may contain a repressor. The sequence of the intron is provided in SEQ ID NO: 21.

### EXAMPLE 2

### Isolation of a CDC Promoter from Pinus radiata

Plant EST sequences homologous to the Cell Division Control (CDC) protein gene were isolated from a *Pinus radiata* cDNA expression library as described in Example 1. Using the "Genome Walker" protocol described above and gene specific primers designed from these plant EST sequences, 5'UTR sequence containing the putative promoter of the *P. radiata* CDC gene was isolated from genomic DNA. The determined nucleotide sequence is given in SEQ ID NO: 4.

### EXAMPLE 3

### Isolation of a Xylogenesis-Specific Promoter from Pinus radiata

Plant EST sequences specific for plant xylogenesis were isolated from *Pinus radiata* cDNA expression libraries prepared from xylem, essentially as described in Example 1. Using the "Genome Walker" protocol described above and gene specific primers designed from these plant EST sequences, sequences containing putative *Pinus radiata* xylogenesis-specific promoters were isolated from genomic DNA. The determined nucleotide sequences are provided in SEQ ID NO: 5 and 41-44. An extended cDNA sequence for the clone of SEQ ID NO: 41-44 is provided in SEQ ID NO: 92.

### EXAMPLE 4

### Isolation of a 4-Coumarate-CoA Ligase Promoter from Pinus radiata

Plant EST sequences homologous to the 4-Coumarate-CoA Ligase (4CL) gene were isolated from a *Pinus radiata* cDNA expression library as described in Example 1. Using the "Genome Walker" protocol described above and gene specific primers designed from these plant EST sequences, sequences containing the putative promoter of the *P. radiata* 4CL gene was isolated from genomic DNA. The determined nucleotide sequence is given in SEQ ID NO: 6.

DNA constructs comprising the reporter gene for Green Fluorescent Protein (GFP) or GUS reporter genes operably linked to the promoter of SEQ ID NO: 6 were prepared and used to transform *Arabidopsis thaliana* plants.

### EXAMPLE 5

### Isolation of a Cellulose Synthase Promoter from Eucalyptus grandis

Plant EST sequences homologous to the cellulose synthase gene were isolated from a *Eucalyptus grandis* cDNA expression library essentially as described in Example 1. Using the "Genome Walker" protocol described above and gene specific primers designed from these plant EST sequences, 5'UTR sequences containing the putative promoter of the *E. grandis* cellulose synthase gene were isolated from genomic DNA. Independent PCR experiments using different DNA bands as templates yielded two sequences which contained a number of base differences. One band was 750 bp in length and the nucleotide sequence of this band is given in SEQ ID NO: 7. The other band was 3 kb in length. The sequence of the 3' end of this band corresponded to the sequence given in SEQ ID NO: 7, with a number of base pair differences. The sequence of this 3' end is given in SEQ ID NO: 8. The sequence of the 5' end of this band is given in SEQ ID NO: 20.

### EXAMPLE 6

### Isolation of a Leaf-Specific Promoter from Eucalyptus grandis

Plant EST sequences specific for leaf were isolated from *Eucalyptus grandis* cDNA expression libraries prepared from leaf tissue, essentially as described in Example 1. Using the "Genome Walker" protocol described above and gene specific primers designed from these plant EST sequences, 5'UTR sequence containing a leaf-specific promoter of a novel *E*. *grandis* gene (of unknown function) was isolated from genomic DNA. Independent PCR experiments using different DNA bands as templates yielded three sequences which contained a number of base differences and deletions. The determined nucleotide sequences of the three PCR fragments are given in SEQ ID NO: 9-11.

### EXAMPLE 7

### Isolation of an O-Methyl Transferase Promoter from Eucalyptus grandis

Plant EST sequences homologous to an O-methyl transferase (OMT) gene were isolated from a *Eucalyptus grandis* cDNA expression library essentially as described in Example 1. Using the "Genome Walker" protocol described above and gene specific primers designed from these plant EST sequences, 5'UTR sequences containing the putative promoter of the *E. grandis* OMT gene was isolated from genomic DNA. The determined nucleotide sequence is given in SEQ ID NO: 12.

DNA constructs comprising the reporter gene for Green Fluorescent Protein (GFP) operably linked to the promoter of SEQ ID NO: 12 were prepared and used to transform *Arabidopsis thaliana*.

### EXAMPLE 8

### Isolation of Root-Specific Promoters from Pinus radiata

Plant EST sequences homologous to the root-specific receptor-like kinase gene were isolated from a *Pinus radiata* cDNA expression library as described in Example 1. Using the "Genome Walker" protocol described above and gene specific primers designed from these plant EST sequences, 5'UTR sequence containing a putative *P. radiata* root-specific promoter was isolated from genomic DNA. Two independent PCR experiments yielded sequences that contained a number of base differences. The determined nucleotide sequences from the two experiments are given in SEQ ID NO: 13, 14, 110 and 111.

### EXAMPLE 9

### Isolation of an EF1-alpha Promoter from Eucalyptus Grandis

Plant EST sequences homologous to the *Eucalyptus* Elongation Factor-alpha (EF1-alpha) gene were isolated from a *Eucalyptus grandis* cDNA expression library and used to screen a *Eucalyptus grandis* genomic DNA library as follows.

The *Eucalyptus grandis* genomic DNA library was constructed using genomic DNA extracted from *Eucalyptus nitens x grandis* plant tissue, according to the protocol of Doyle and Doyle, *Focus* 12:13-15, 1990, with minor modifications. Specifically, plant tissue was ground under liquid nitrogen and dissolved in 2X CTAB extraction buffer (2% CTAB, hexadecyltrimethylammonium bromide; 1.4 M NaCl, 20 mM EDTA pH 8.0, 100 mM Tris.HCl pH 8.0, 1% polyvinylpyrollidone). After extraction with chloroform: isoamylalcohol (24:1), 10% CTAB was added to the aqueous layer and the chloroform:isoamylalcohol extraction repeated. Genomic DNA was precipitated with isopropanol.

The resulting DNA was digested with restriction endonuclease *Sau*3A1 following standard procedures, extracted once with phenol:chloroform:isoamylalcohol (25:24:1) and ethanol precipitated. The digested fragments were separated on a sucrose density gradient using ultracentrifugation. Fractions containing fragments of 9-23 kb were pooled and ethanol precipitated. The resulting fragments were cloned into the lambda DASH II/*Bam*HI vector (Stratagene, La Jolla, CA) following the manufacturer's protocol and packaged using a Gigapack II Packaging Extract (Stratagene). The library was amplified once.

The library was screened with radio-labeled EST fragments isolated from a *Eucalyptus grandis* library (as described in Example 1), that showed homology to the *Eucalyptus* EF1-alpha gene. Phage lysates were prepared from positive plaques and genomic DNA was extracted.

From this genomic DNA, the 5'UTR region containing the putative promoter of the *Eucalyptus* EF1-alpha gene was obtained using the ELONGASE Amplification System (Gibco BRL). A 10 kb fragment was amplified and restriction mapped. The putative promoter region of the *Eucalyptus* elongation factor A (EF1-alpha) gene was identified on a 4kb fragment, which was subcloned into a pUC 19 vector (Gibco BRL) containing an engineered *Not*I-site. The determined genomic DNA sequences of the isolated fragment containing the promoter region are provided in SEQ ID NO: 61 and 62, with the predicted amino acid encoded by SEQ ID NO: 61 being provided in SEQ ID NO: 79.

### EXAMPLE 10

### Isolation of Flower-Specific Promoters from Eucalyptus grandis

Plant EST sequences specific for flower-derived tissue were isolated from *Eucalyptus grandis* cDNA expression libraries prepared from flower tissue, essentially as described in Example 1. Using the "Genome Walker" protocol described above and gene specific primers designed from these plant EST sequences, several sequences, each containing a putative *Eucalyptus grandis* flower-specific promoter, were isolated from genomic DNA. The determined nucleotide sequences are given in SEQ ID NO: 29-33 and 59. An extended cDNA sequence of the clone of SEQ ID NO: 30-33 is provided in SEQ ID NO: 89. An extended cDNA sequence of the clone of SEQ ID NO: 29 is provided in SEQ ID NO: 90.

### EXAMPLE 11

### Isolation of Pollen-Specific Promoters from Eucalyptus grandis and Pinus radiata

Plant EST sequences specific for pollen were isolated from *Eucalyptus grandis* and *Pinus radiata* cDNA expression libraries prepared from pollen, essentially as described in Example 1. Using the "Genome Walker" protocol described above and gene specific primers designed from these plant EST sequences, several sequences, each containing a putative pollen-specific promoter, were isolated from genomic DNA. The determined nucleotide sequences isolated from *Pinus radiata* are given in SEQ ID NO: 49-53, with the predicted amino acid sequences encoded by SEQ ID NO: 51-53 being provided in SEQ ID NO: 73-75, respectively. An extended cDNA sequence for the clone of SEQ ID NO: 49 is provided in SEQ ID NO: 94.

### EXAMPLE 12

### Isolation of Bud-Specific and Meristem-Specific Promoter from Pinus radiata

Plant EST sequences specific for bud and meristem were isolated from *Pinus radiata* cDNA expression libraries prepared from bud and meristem, essentially as described in Example 1. Using the "Genome Walker" protocol described above and gene specific primers designed from these plant EST sequences, two sequences, one containing a putative bud-specific promoter and the other containing a putative meristem-specific promoter, were isolated from genomic DNA. The determined nucleotide sequences for these two promoters are given in SEQ ID NO: 40 and 45, respectively. The predicted amino acid sequences encoded by the DNA sequences of SEQ ID NO: 40 and 45 are provided in SEQ ID NO: 70 and 71, respectively.

### EXAMPLE 13

### Isolation of Promoters from Eucalyptus grandis

Plant EST sequences showing some homology to various known genes were isolated from *Eucalyptus grandis* cDNA expression libraries essentially as described in Example 1. Using the "Genome Walker" protocol described above and gene specific primers designed from these plant EST sequences, sequences containing the putative promoters for the following *E. grandis* genes were isolated from genomic DNA: auxin induced protein (SEQ ID NO: 26-28); carbonic anhydrase (SEQ ID NO: 36); isoflavone reductase (SEQ ID NO: 37 and 38); pollen allergen (SEQ ID NO: 23-25); pollen coat protein (SEQ ID NO: 22), sucrose synthase (SEQ ID NO: 56-58); ubiquitin (SEQ ID NO: 34); glyceraldehyde-3-phosphate dehydrogenase (SEQ ID NO: 35 and 39); O-methyl transferase (OMT; SEQ ID NO: 60); macrophage migration inhibition factor from mammals (MIF; SEQ ID NO: 81-86); UDP glucose 6-dehydrogenase (SEQ ID NO: 103); laccase 1 (SEQ ID NO: 105, 106); arabinogalactan-like 1 (SEQ ID NO: 107); arabinogalactan-like 2 (SEQ ID NO: 108, 109) and a hypothetical protein (SEQ ID NO: 104). The predicted amino acid sequences encoded by the DNA sequences of SEQ ID NO: 22, 25, 26, 28, 34, 35, 36, 56, 57, 60 and 86 are provided in SEQ ID NO: 63, 64, 65, 66, 67, 68, 69, 76, 77, 78 and 87 respectively. Extended cDNA sequences for the clones of SEQ ID NO: 58 and 35 are provided in SEQ ID NO: 91 and 93, respectively.

### EXAMPLE 14

### Isolation of Promoters from Pinus radiata

Plant EST sequences showing some homology to various known genes were isolated from *Pinus radiata* cDNA expression libraries essentially as described in Example 1. Using the "Genome Walker" protocol described above and gene specific primers designed from these plant EST sequences, sequences containing the putative promoters for the following *Pinus radiata* genes were isolated from genomic DNA: senescence-like protein (SEQ ID NO: 46-48); nodulin homolog pollen specific (SEQ ID NO: 54 and 55); chalcone synthase (SEQ ID NO: 88); PrMALE1 (SEQ ID NO: 95, 96); UDP glucose glycosyltransferase (SEQ ID NO: 97); elogation factor 1 alpha (SEQ ID NO: 98, 99); S-adenosylmethionine synthase (SEQ ID NO: 100-102); and *Pinus radiata* lipid transfer protein 2 (PrLTP2; SEQ ID NO: 112). The predicted amino acid sequence encoded by the sequence of SEQ ID NO: 46 is provided in SEQ ID NO: 72.

### EXAMPLE 15

### Polynucleotide and Amino Acid Analysis

The determined cDNA sequences described above were compared to and aligned with known sequences in the EMBL database (as updated to September 1999). Specifically, the polynucleotides identified in SEQ ID NO:22-62 and 88-112 were compared to polynucleotides in the EMBL database using the BLASTN algorithm Version 2.0.6 [Sept-16-1998] set to the following running parameters: Unix running command: blastall -p blastn -d embldb -e 10 -G0 -E0 -r1 -v30 -b30 -i queryseq -o results. Multiple alignments of redundant sequences were used to build up reliable consensus sequences. Based on similarity to known sequences from other plant or non-plant species, the isolated polynucleotides of the present invention identified as SEQ ID NO: 22-62 and 88-112 were putatively identified as having the functions shown in Table 1, above.

The cDNA sequences of SEQ ID NO: 1-22, 23, 25-42, 45-49, 57-59, 62, 88-99 and 101-112 were determined to have less than 40% identity to sequences in the EMBL database using the computer algorithm BLASTN, as described above. The cDNA sequence of SEQ ID NO: 56 was determined to have less than 60% identity to sequences in the EMBL database using BLASTN, as described above. The cDNA sequences of SEQ ID NO: 43, 52, 60 and 61 were determined to have less than 75% identity to sequences in the EMBL database using BLASTN, as described above. The cDNA sequences of SEQ ID NO: 24, 51 and 100 were determined to have less than 90% identity to sequences in the EMBL database using BLASTN, as described above.

## Claims

1. An isolated polynucleotide comprising a sequence selected from the group consisting of:
(a) the sequence recited in SEQ ID NO: 2 or 3,
(b) complements of the sequence recited in SEQ ID NO: 2 or 3;
(c) reverse complements of the sequence recited in SEQ ID NO: 2 or 3;
(d) reverse sequences of the sequence recited in SEQ ID NO: 2 or 3;
(e) sequences having at least 75% identity to the sequence recited in SEQ ID NO: 2 or 3 and
wherein the polynucleotide is able to regulate transcription of a DNA sequence of interest; and
(f) sequences having at least 90% identity to the sequence recited in SEQ ID NO: 2 or 3,
wherein the polynucleotide is able to regulate transcription of a DNA sequence of interest.

2. A DNA construct comprising a polynucleotide according to claim 1.

3. A DNA construct as claimed in claim 2 comprising, in the 5'-3' direction:
(a) a promoter sequence,
(b) a DNA sequence of interest; and
(c) a gene termination sequence,
wherein the promoter sequence comprises an isolated polynucleotide according to claim 1.

4. The DNA construct of claim 3, wherein the DNA sequence of interest comprises an open reading frame encoding a polypeptide of interest.

5. The DNA construct of claim 3, wherein the DNA sequence of interest comprises a non-coding region of a gene encoding a polypeptide of interest.

6. A transgenic cell comprising a DNA construct of any one of claims 2 to 5.

7. A plant comprising a transgenic cell according to claim 6, or a part or propagule or progeny thereof.

8. A method for modifying gene expression in a target plant comprising stably incorporating into the genome of the plant a DNA construct according to any one of claims 2 to 5.

9. A method for producing a plant having modified gene expression comprising:
(a) transforming a plant cell with a DNA construct to provide a transgenic cell, wherein the DNA construct comprises: (i) a promoter sequence comprising a sequence of SEQ ID NO: 2 or 3; (ii) a DNA sequence of interest; and (iii) a gene termination sequence; and
(b) cultivating the transgenic cell under conditions conducive to regeneration and mature plant growth.

10. A method for modifying a phenotype of a target plant, comprising stably incorporating into the genome of the target plant a DNA construct comprising:
(a) a promoter sequence comprising a sequence of SEQ ID NO: 2 or 3;
(b) a DNA sequence of interest; and
(c) a gene termination sequence.

11. A method for identifying a gene responsible for a desired function or phenotype, comprising:
(a) transforming a plant cell with a DNA construct comprising a promoter sequence operably linked to a gene to be tested, the promoter sequence comprising a sequence of SEQ ID NO: 2 or 3;
(b) cultivating the plant cell under conditions conducive to regeneration and mature plant growth to provide a transgenic plant; and
(c) comparing the phenotype of the transgenic plant with the phenotype of non-transformed plants.

12. An isolated polynucleotide comprising a sequence selected from the group consisting of:
(a) the sequence recited in SEQ ID NO: 21;
(b) complements of the sequence recited in SEQ ID NO: 21;
(c) reverse complements of the sequence recited in SEQ ID NO: 21; and
(d) reverse sequences of the sequence recited in SEQ ID NO: 21.

13. A DNA construct comprising a polynucleotide according to claim 12.

14. A transgenic cell comprising a DNA construct according to claim 13.

15. A method for modifying gene expression in a target plant comprising stably incorporating into the genome of the plant a DNA construct according to claim 13.

16. A method for modifying expression of a polynucleotide that comprises the sequence of SEQ ID NO: 21, the method comprising removing the sequence of SEQ ID NO: 21 from the polynucleotide.

## Patentansprüche

1. Isoliertes Polynucleotid, eine aus der aus Folgenden bestehenden Gruppe ausgewählte Sequenz umfassend:
(a) Sequenz der Seq.-ID Nr. 2 oder 3;
(b) Komplemente der Seq.-ID Nr. 2 oder 3;
(c) reverse Komplemente der Seq.-ID Nr. 2 oder 3;
(d) reverse Sequenzen der Seq.-ID Nr. 2 oder 3;
(e) Sequenzen mit zumindest 75 % Identität mit der Seq.-ID Nr. 2 oder 3, worin das Polynucleotid zur Regulierung der Transkription einer DNA-Sequenz von Interesse fähig ist; und
(f) Sequenzen mit zumindest 90 % Identität mit der Seq.-ID Nr. 2 oder 3, worin das Polynucleotid zur Regulierung der Transkription einer DNA-Sequenz von Interesse fähig ist.

2. DNA-Konstrukt, ein Polynucleotid nach Anspruch 1 umfassend.

3. DNA-Konstrukt nach Anspruch 2, in 5'-3'-Richtung Folgendes umfassend:
(a) eine Promotorsequenz;
(b) eine DNA-Sequenz von Interesse; und
(c) eine Genterminationssequenz,
worin die Promotorsequenz ein isoliertes Polynucleotid nach Anspruch 1 umfasst.

4. DNA-Konstrukt nach Anspruch 3, worin die DNA-Sequenz von Interesse ein offenes Leseraster umfasst, das für ein Polypeptid von Interesse kodiert.

5. DNA-Konstrukt nach Anspruch 3, worin die DNA-Sequenz von Interesse eine nichtkodierende Region eines Gens umfasst, das für ein Polypeptid von Interesse kodiert.

6. Transgene Zelle, ein DNA-Konstrukt nach einem der Ansprüche 2 bis 5 umfassend.

7. Pflanze, eine transgene Zelle nach Anspruch 6 oder einen Teil, eine Propagationseinheit oder Nachkommenschaft davon umfassend.

8. Verfahren zur Modifikation der Genexpression in einer Zielpflanze, die stabile Inkorporation eines DNA-Konstrukts nach einem der Ansprüche 2 bis 5 in das Genom der Pflanze umfassend.

9. Verfahren zur Herstellung einer Pflanze mit modifizierter Genexpression, umfassend:
(a) Transformieren einer Pflanzenzelle mit einem DNA-Konstrukt, um eine transgene Zelle bereitzustellen, worin das DNA-Konstrukt Folgendes umfasst: (i) eine Promotorsequenz, die eine Sequenz der Seq.-ID Nr. 2 oder 3 umfasst; (ii) eine DNA-Sequenz von Interesse; und (iii) eine Genterminationssequenz; und
(b) Kultivieren der transgenen Zelle unter Bedingungen, die der Regeneration und dem Wachstum erwachsener Pflanzen förderlich sind.

10. Verfahren zur Modifikation eines Phänotyps einer Zielpflanze, umfassend die stabile Inkorporation eines Folgendes umfassenden DNA-Konstrukts in das Genom der Pflanze, umfassend:
(a) eine Promotorsequenz, die eine Sequenz der Seq.-ID Nr. 2 oder 3 umfasst;
(b) eine DNA-Sequenz von Interesse; und
(c) eine Genterminationssequenz.

11. Verfahren zur Identifikation eines Gens, das für eine gewünschte Funktion oder einen gewünschten Phänotyp verantwortlich ist, umfassend:
(a) Transformieren einer Pflanzenzelle mit einem DNA-Konstrukt, das eine Promotorsequenz umfasst, die operabel an ein zu testendes Gen gebunden ist, wobei die Promotorsequenz eine Sequenz der Seq.-ID Nr. 2 oder 3 umfasst;
(b) Kultivieren der Pflanzenzelle unter Bedingungen, die der Regeneration und dem Wachstum erwachsener Pflanzen förderlich sind, um eine transgene Pflanze bereitzustellen; und
(c) Vergleichen des Phänotyps der transgenen Pflanze mit dem Phänotyp von nicht transformierten Pflanzen.

12. Isoliertes Polynucleotid, eine aus der aus Folgenden bestehenden Gruppe ausgewählte Sequenz umfassend:
(a) Sequenz der Seq.-ID Nr. 21;
(b) Komplemente der Seq.-ID Nr. 21;
(c) reverse Komplemente der Seq.-ID Nr. 21;
(d) reverse Sequenzen der Seq.-ID Nr. 21.

13. DNA-Konstrukt, ein Polynucleotid nach Anspruch 12 umfassend.

14. Transgene Zelle, ein DNA-Konstrukt nach Anspruch 13 umfassend.

15. Verfahren zur Modifikation der Genexpression in einer Zielpflanze, die stabile Inkorporation eines DNA-Konstrukts nach Anspruch 13 in das Genom der Pflanze umfassend.

16. Verfahren zur Modifikation der Expression eines Polynucleotids, das die Sequenz der Seq.-ID Nr. 21 umfasst, wobei das Verfahren das Entfernen der Sequenz der Seq.-ID Nr. 21 aus dem Polynucleotid umfasst.

## Revendications

1. Un polynucléotide isolé comprenant une séquence choisie dans le groupe se composant de :
(a) la séquence énoncée dans ID SEQ NO: 2 ou 3 ;
(b) des compléments de la séquence énoncée dans ID SEQ NO: 2 ou 3 ;
(c) des compléments inverses de la séquence énoncée dans ID SEQ NO: 2 ou 3 ;
(d) des séquences inverses de la séquence énoncée dans ID SEQ NO: 2 ou 3 ;
(e) des séquences présentant au moins 75 % d'identité à la séquence énoncée dans ID SEQ NO: 2 ou 3, et où le polynucléotide est capable de réguler la transcription d'une séquence d'ADN intéressante ; et
(f) des séquences présentant au moins 90 % d'identité à la séquence énoncée dans ID SEQ NO: 2 ou 3, où le polynucléotide est capable de réguler la transcription d'une séquence d'ADN intéressante.

2. Une construction d'ADN comprenant un polynucléotide conforme à la revendication 1.

3. Une construction d'ADN telle que revendiquée à la revendication 2 comprenant, dans la direction 5'-3' :
(a) une séquence de promoteur,
(b) une séquence d'ADN intéressante ; et
(c) une séquence de terminaison génique,
où la séquence de promoteur comprend un polynucléotide isolé conforme à la revendication 1.

4. La construction d'ADN de la revendication 3, où la séquence d'ADN intéressante comprend un cadre de lecture ouvert codant un polypeptide intéressant.

5. La construction d'ADN de la revendication 3, où la séquence d'ADN intéressante comprend une région de non codage d'un gêne codant un polypeptide intéressant.

6. Une cellule transgénique comprenant une construction d'ADN d'une quelconque des revendications 2 à 5.

7. Une plante comprenant une cellule transgénique conforme à la revendication 6, ou une partie ou propagule ou descendante de celle-ci.

8. Une procédé pour modifier l'expression génique dans une plante recherchée comprenant l'étape consistant à incorporer, de manière stable dans le génome de la plante, une construction d'ADN conforme à l'une quelconque des revendications 2 à 5.

9. Un procédé pour produite une plante présentant une expression génique modifiée comprenant les étapes consistant à :
(a) transformer une cellule de plante avec une construction d'ADN pour créer une cellule transgénique, où la construction d'ADN comprend : (i) une séquence de promoteur comprenant une séquence de ID SEQ NO: 2 ou 3 ; (ii) une séquence d'ADN intéressante ; et (iii) une séquence de terminaison génique ; et
(b) cultiver la cellule transgénique sous des conditions conduisant à une régénérescence et une maturation de la croissance de la plante.

10. Un procédé pour modifier un phénotype d'une plante recherchée comprenant l'étape consistant à incorporer, de manière stable dans le génome de la plante recherchée, une construction d'ADN comprenant :
(a) une séquence de promoteur comprenant une séquence de ID SEQ NO: 2 ou 3 ;
(b) une séquence d'ADN intéressante ; et
(c) une séquence de terminaison génique.

11. Un procédé d'identification d'un gêne responsable d'une fonction ou d'un phénotype désiré comprenant les étapes consistant à :
(a) transformer une cellule de plante avec une construction d'ADN comprenant une séquence de promoteur reliée de manière opérable à une gêne devant être testé, la séquence de promoteur comprenant une séquence de ID SEQ NO: 2 ou 3 ;
(b) cultiver la cellule de plante sous des conditions conduisant à une régénérescence et une maturation de la croissance de la plante pour créer une plante transgénique ; et
(c) comparer le phénotype de la plante transgénique avec le phénotype de plantes non transformées.

12. Un polynucléotide isolé comprenant une séquence choisie dans le groupe se composant de :
(a) la séquence énoncée dans ID SEQ NO: 21 ;
(b) des compléments de la séquence énoncée dans ID SEQ NO: 21 ;
(c) des compléments inverses de la séquence énoncée dans ID SEQ NO: 21 ; et
(d) des séquences inverses de la séquence énoncée dans ID SEQ NO: 21.

13. Une construction d'ADN comprenant un polynucléotide conforme à la revendication 12.

14. Une cellule transgénique comprenant une construction d'ADN conforme à la revendication 13.

15. Un procédé pour modifier une expression génique dans une plante recherchée comprenant l'étape consistant à incorporer, de manière stable dans le génome de la plante, une construction d'ADN conforme à la revendication 13.

16. Un procédé pour modifier l'expression d'un polynucléotide qui comprend la séquence de ID SEQ NO: 21, le procédé comprenant le fait de retirer la séquence de ID SEQ NO: 21 du polynucléotide.


### SEQUENCE LISTING

<110> Genesis Research & Development Corporation Limited
<110> Fletcher Challenge Forests Limited
<120> Compositions and Methods for the Modification of Gene Expression
<130> SMK/FP5955075
<140> EP 00905490.9
   <141> 2000-02-24
<150> PCT/NZ00/00018
   <151> 2000-02-24
<150> US 09/276,599
   <151> 1999-03-25
<150> US 60/146,591
   <151> 1999-07-30
<160> 112
<170> FastSEQ for Windows Version 3.0
<210> 1
   <211> 3083
   <212> DNA
   <213> Pinus radiata
<220>
   <221> 5'UTR
   <222> (1)...(2064)
<221> intron
   <222> (1196)...(2033)
<221> CDS
   <222> (2065)...(2751)
<221> 3'UTR
   <222> (2755)...(3083)
<400> 1
   aaaacccctc acaaatacat aaaaaaaatt ctttatttaa ttatcaaact ctccactacc     60
   tttcccacca accgttacaa tcctgaatgt tggaaaaaac taactacatt gatataaaaa    120
   aactacatta cttcctaaat catatcaaaa ttgtataaat atatccactc aaaggagtct    180
   agaagatcca cttggacaaa ttgcccatag ttggaaagat gttcaccaag tcaacaagat    240
   ttatcaatgg aaaaatccat ctaccaaact tactttcaag aaaatccaag gattatagag    300
   taaaaaatct atgtattatt aagtcaaaaa gaaaaccaaa gtgaacaaat attgatgtac    360
   aagtttgaga ggataagaca ttggaatcgt ctaaccagga ggcggaggaa ttccctagac    420
   agttaaaagt ggccggaatc ccggtaaaaa agattaaaat ttttttgtag agggagtgct    480
   tgaatcatgt tttttatgat ggaaatagat tcagcaccat caaaaacatt caggacacct    540
   aaaattttga agtttaacaa aaataacttg gatctacaaa aatccgtatc ggattttctc    600
   taaatataac tagaattttc ataactttca aagcaactcc tcccctaacc gtaaaacttt    660
   tcctacttca ccgttaatta cattccttaa gagtagataa agaaataaag taaataaaag    720
   tattcacaaa ccaacaattt atttctttta tttacttaaa aaaacaaaaa gtttatttat    780
   tttacttaaa tggcataatg acatatcgga gatccctcga acgagaatct tttatctccc    840
   tggttttgta ttaaaaagta atttattgtg gggtccacgc ggagttggaa tcctacagac    900
   gcgctttaca tacgtctcga gaagcgtgac ggatgtgcga ccggatgacc ctgtataacc    960
   caccgacaca gccagcgcac agtatacacg tgtcatttct ctattggaaa atgtcgttgt   1020
   tatccccgct ggtacgcaac caccgatggt gacaggtcgt ctgttgtcgt gtcgcgtagc   1080
   gggagaaggg tctcatccaa cgctattaaa tactcgcctt caccgcgtta cttctcatct   1140
   tttctcttgc gttgtataat cagtgcgata ttctcagaga gcttttcatt caaaggtatg   1200
   gagttttgaa gggctttact cttaacattt gtttttcttt gtaaattgtt aatggtggtt   1260
   tctgtggggg aagaatcttt tgccaggtcc ttttgggttt cgcatgttta tttgggttat   1320
   ttttctcgac tatggctgac attactaggg ctttcgtgct ttcatctgtg ttttcttccc   1380
   ttaataggtc tgtctctctg gaatatttaa ttttcgtatg taagttatga gtagtcgctg   1440
   tttgtaatag gctcttgtct gtaaaggttt cagcaggtgt ttgcgtttta ttgcgtcatg   1500
   tgtttcagaa ggcctttgca gattattgcg ttgtacttta atattttgtc tccaaccttg   1560
   ttatagtttc cctcctttga tctcacagga accctttctt ctttgagcat tttcttgtgg   1620
   cgttctgtag taatatttta attttgggcc cgggttctga gggtaggtga ttattccagt   1680
   gatgtgcttt ccctataagg tcctctatgt gtaagctgtt agggtttgtg cgttactatt   1740
   gacatgtcac atgtcacata ttttcttcct cttatccttc gaactgatgg ttctttttct   1800
   aattcgtgga ttgctggtgc catattttat ttctattgca actgtatttt agggtgtctc   1860
   tttctttttg atttcttgtt aatatttgtg ttcaggttgt aactatgggt tgctagggtg    1920
   tctgccctct tcttttgtgc ttctttcgca gaatctgtcc gttggtctgt atttgggtga   1980
   tgaattattt attccttgaa gtatctgtct aattagcttg tgatgatgtg caggtatatt   2040
   cgttagtcat atttcaattt caag atg cag atc ttt gtc aag act ctc acc      2091
                              Met Gln Ile Phe Val Lys Thr Leu Thr
                               1               5
   ggt aag acc atc act ctc gag gtc gag agc tct gac acc att gac aat     2139
   Gly Lys Thr Ile Thr Leu Glu Val Glu Ser Ser Asp Thr Ile Asp Asn
    10                  15                  20                  25
   gtt aaa gct aag atc cag gac aag gaa ggg att ccc ccc gac cag cag     2187
   Val Lys Ala Lys Ile Gln Asp Lys Glu Gly Ile Pro Pro Asp Gln Gln
                    30                  35                  40
   cgt ctg atc ttc gca gga aag cag ctt gag gac ggc cga acc ctt gcc     2235
   Arg Leu Ile Phe Ala Gly Lys Gln Leu Glu Asp Gly Arg Thr Leu Ala
                45                  50                  55
   gat tac aac atc cag aaa gaa tct acc ctc cac ctt gtt ctc cgt ttg     2283
   Asp Tyr Asn Ile Gln Lys Glu Ser Thr Leu His Leu Val Leu Arg Leu
            60                  65                  70
   agg ggt ggc atg caa atc ttt gta aaa aca cta act gga aag aca att     2331
   Arg Gly Gly Met Gln Ile Phe Val Lys Thr Leu Thr Gly Lys Thr Ile
        75                  80                  85
   aca ttg gaa gtt gag agc tcg gac acc att gac aac gtc aag gcc aag     2379
   Thr Leu Glu Val Glu Ser Ser Asp Thr Ile Asp Asn Val Lys Ala Lys
    90                  95                 100                 105
   atc cag gac aag gaa gga att ccc cct gac cag cag agg ctt atc ttc     2427
   Ile Gln Asp Lys Glu Gly Ile Pro Pro Asp Gln Gln Arg Leu Ile Phe
                   110                 115                 120
   gct ggt aag cag ctg gag gat ggc agg acc ttg gct gat tac aat att     2475
   Ala Gly Lys Gln Leu Glu Asp Gly Arg Thr Leu Ala Asp Tyr Asn Ile
               125                 130                 135
   caa aag gaa tcg acc ctg cat ttg gtg ctt cgt cta aga gga ggc atg     2523
   Gln Lys Glu Ser Thr Leu His Leu Val Leu Arg Leu Arg Gly Gly Met
           140                 145                 150
   caa atc ttt gtg aaa acc ctt aca ggt aaa acc att act ctg gaa gtg     2571
   Gln Ile Phe Val Lys Thr Leu Thr Gly Lys Thr Ile Thr Leu Glu Val
       155                 160                 165
   gaa agc tcg gac acc att gac aat gtg aag gct aag atc cag gac aag      2619
   Glu Ser Ser Asp Thr Ile Asp Asn Val Lys Ala Lys Ile Gln Asp Lys
   170                 175                 180                 185
   gag gga att cca cct gac cag cag agg ttg atc ttt gcc ggt aag cag      2667
   Glu Gly Ile Pro Pro Asp Gln Gln Arg Leu Ile Phe Ala Gly Lys Gln
                   190                 195                 200
   ctg gaa gat ggt cgt act ctc gcc gat tac aat att cag aag gaa tcg      2715
   Leu Glu Asp Gly Arg Thr Leu Ala Asp Tyr Asn Ile Gln Lys Glu Ser
               205                 210                 215
   acc ctt cac ctg gtg ctc cgt ctc cgc ggt ggc ttt taggtttggg           2761
   Thr Leu His Leu Val Leu Arg Leu Arg Gly Gly Phe
           220                 225
   tgttatttgt ggataataaa ttcgggtgat gttcagtgtt tgtcgtattt ctcacgaata   2821
   aattgtgttt atgtatgtgt tagtgttgtt tgtctgtttc agaccctctt atgttatatt   2881
   tttcttttcg tcggtcagtt gaagccaata ctggtgtcct ggccggcact gcaataccat   2941
   ttcgtttaat ataaagactc tgttatccgt tatgtaattc catgttatgt ggtgaaatgt   3001
   ggatgaaatt cttagaaatt attattgtaa tttgaaactt ccttcgtcaa taatctgcac   3061
   aacacattta ccaaaaaaaa aa                                             3083
<210> 2
   <211> 2064
   <212> DNA
   <213> Pinus radiata
<220>
   <221> 5'UTR
   <222> (1) ... (2064)
<221> intron
   <222> (1196) ... (2033)
<400> 2
   aaaacccctc acaaatacat aaaaaaaatt ctttatttaa ttatcaaact ctccactacc      60
   tttcccacca accgttacaa tcctgaatgt tggaaaaaac taactacatt gatataaaaa     120
   aactacatta cttcctaaat catatcaaaa ttgtataaat atatccactc aaaggagtct     180
   agaagatcca cttggacaaa ttgcccatag ttggaaagat gttcaccaag tcaacaagat     240
   ttatcaatgg aaaaatccat ctaccaaact tactttcaag aaaatccaag gattatagag     300
   taaaaaatct atgtattatt aagtcaaaaa gaaaaccaaa gtgaacaaat attgatgtac     360
   aagtttgaga ggataagaca ttggaatcgt ctaaccagga ggcggaggaa ttccctagac     420
   agttaaaagt ggccggaatc ccggtaaaaa agattaaaat ttttttgtag agggagtgct     480
   tgaatcatgt tttttatgat ggaaatagat tcagcaccat caaaaacatt caggacacct     540
   aaaattttga agtttaacaa aaataacttg gatctacaaa aatccgtatc ggattttctc      600
   taaatataac tagaattttc ataactttca aagcaactcc tcccctaacc gtaaaacttt     660
   tcctacttca ccgttaatta cattccttaa gagtagataa agaaataaag taaataaaag     720
   tattcacaaa ccaacaattt atttctttta tttacttaaa aaaacaaaaa gtttatttat     780
   tttacttaaa tggcataatg acatatcgga gatccctcga acgagaatct tttatctccc     840
   tggttttgta ttaaaaagta atttattgtg gggtccacgc ggagttggaa tcctacagac     900
   gcgctttaca tacgtctcga gaagcgtgac ggatgtgcga ccggatgacc ctgtataacc     960
   caccgacaca gccagcgcac agtatacacg tgtcatttct ctattggaaa atgtcgttgt    1020
   tatccccgct ggtacgcaac caccgatggt gacaggtcgt ctgttgtcgt gtcgcgtagc    1080
   gggagaaggg tctcatccaa cgctattaaa tactcgcctt caccgcgtta cttctcatct    1140
   tttctcttgc gttgtataat cagtgcgata ttctcagaga gcttttcatt caaaggtatg    1200
   gagttttgaa gggctttact cttaacattt gtttttcttt gtaaattgtt aatggtggtt    1260
   tctgtggggg aagaatcttt tgccaggtcc ttttgggttt cgcatgttta tttgggttat    1320
   ttttctcgac tatggctgac attactaggg ctttcgtgct ttcatctgtg ttttcttccc    1380
   ttaataggtc tgtctctctg gaatatttaa ttttcgtatg taagttatga gtagtcgctg    1440
   tttgtaatag gctcttgtct gtaaaggttt cagcaggtgt ttgcgtttta ttgcgtcatg    1500
   tgtttcagaa ggcctttgca gattattgcg ttgtacttta atattttgtc tccaaccttg    1560
   ttatagtttc cctcctttga tctcacagga accctttctt ctttgagcat tttcttgtgg    1620
   cgttctgtag taatatttta attttgggcc cgggttctga gggtaggtga ttattccagt    1680
   gatgtgcttt ccctataagg tcctctatgt gtaagctgtt agggtttgtg cgttactatt    1740
   gacatgtcac atgtcacata ttttcttcct cttatccttc gaactgatgg ttctttttct    1800
   aattcgtgga ttgctggtgc catattttat ttctattgca actgtatttt agggtgtctc    1860
   tttctttttg atttcttgtt aatatttgtg ttcaggttgt aactatgggt tgctagggtg    1920
   tctgccctct tcttttgtgc ttctttcgca gaatctgtcc gttggtctgt atttgggtga    1980
   tgaattattt attccttgaa gtatctgtct aattagcttg tgatgatgtg caggtatatt    2040
   cgttagtcat atttcaattt caag                                            2064
<210> 3
   <211> 1226
   <212> DNA
   <213> Pinus radiata
<220>
   <221> 5'UTR
   <222> (1) ... (1266)
<400> 3
   aaaacccctc acaaatacat aaaaaaaatt ctttatttaa ttatcaaact ctccactacc       60
   tttcccacca accgttacaa tcctgaatgt tggaaaaaac taactacatt gatataaaaa      120
   aactacatta cttcctaaat catatcaaaa ttgtataaat atatccactc aaaggagtct      180
   agaagatcca cttggacaaa ttgcccatag ttggaaagat gttcaccaag tcaacaagat      240
   ttatcaatgg aaaaatccat ctaccaaact tactttcaag aaaatccaag gattatagag      300
   taaaaaatct atgtattatt aagtcaaaaa gaaaaccaaa gtgaacaaat attgatgtac      360
   aagtttgaga ggataagaca ttggaatcgt ctaaccagga ggcggaggaa ttccctagac      420
   agttaaaagt ggccggaatc ccggtaaaaa agattaaaat ttttttgtag agggagtgct      480
   tgaatcatgt tttttatgat ggaaatagat tcagcaccat caaaaacatt caggacacct      540
   aaaattttga agtttaacaa aaataacttg gatctacaaa aatccgtatc ggattttctc      600
   taaatataac tagaattttc ataactttca aagcaactcc tcccctaacc gtaaaacttt      660
   tcctacttca ccgttaatta cattccttaa gagtagataa agaaataaag taaataaaag      720
   tattcacaaa ccaacaattt atttctttta tttacttaaa aaaacaaaaa gtttatttat      780
   tttacttaaa tggcataatg acatatcgga gatccctcga acgagaatct tttatctccc      840
   tggttttgta ttaaaaagta atttattgtg gggtccacgc ggagttggaa tcctacagac      900
   gcgctttaca tacgtctcga gaagcgtgac ggatgtgcga ccggatgacc ctgtataacc      960
   caccgacaca gccagcgcac agtatacacg tgtcatttct ctattggaaa atgtcgttgt     1020
   tatccccgct ggtacgcaac caccgatggt gacaggtcgt ctgttgtcgt gtcgcgtagc     1080
   gggagaaggg tctcatccaa cgctattaaa tactcgcctt caccgcgtta cttctcatct     1140
   tttctcttgc gttgtataat cagtgcgata ttctcagaga gcttttcatt caaaggtata     1200
   ttcgttagtc atatttcaat ttcaag                                          1226
<210> 4
   <211> 485
   <212> DNA
   <213> Pinus radiata
<220>
   <221> 5'UTR
   <222> (1)...(431)
<221> TATA_signal
   <222> (350)...(356)
<221> CAAT_signal
   <222> (326)...(333)
<400> 4
   agtaaaattg gcccatgtag gactaagtca aaatcaaaat tccatctcta aaagcggaac       60
   tttgtcccct gaaaattttg actaatttcc aaccaaaaaa aagtggggga aaatataaaa      120
   ctctaactaa taaaacaata atcaccaaaa atctatcacc aaaaatgaaa aaagattttg      180
   aatactaggc catatgagct acacaaattt caaaagtatc ttacacttat tacgcacccg      240
   gatgtcccca ctttcgaaaa acccgtttca agcctttcac gaaagtccaa cggtcagaaa      300
   attcaaaatg actgtttgag gcagagccaa tctaggacca cgctccattt atatatggcc      360
   tctgcttctc tcgaccctta gagtcctctg ctctgcgaat cttgttgtta gttactgtgt      420
   acgctgtaac aatggatgcc tatgagaagt tggagaaggt gggagaagga acctatggga      480
   aggtg                                                                  485
<210> 5
   <211> 246
   <212> DNA
   <213> Pinus radiata
<220>
   <221> 5'UTR
   <222> (1)...(167)
<221> TATA_signal
   <222> (185)...(191)
<400> 5
   tgagaacatg ataagctgtg taaattcatg ctagtcacca taacttttct cattgctttt      60
   catccacact gttgattcat tcattatata agatcagatt cgtatgatat acaggcaacc     120
   atagaaacaa ccagcaaagt tactagcagg aaatccaact aggtatcatg aagactacca     180
   acgcaggctc gataatgttg gtgctcatta tttttgggtg ctgtttcatt ggggtcatag     240
   ctacat                                                                246
<210> 6
   <211> 600
   <212> DNA
   <213> Pinus radiata
<220>
   <221> 5'UTR
   <222> (1) ... (167)
<221> TATA_signal
   <222> (471) ... (477)
<221> CAAT_signal
   <222> (444) ... (451)
<400> 6
   caccaattta atgggatttc agatttgtat cccatgctat tggctaagcc atttttctta      60
   ttgtaatcta accaattcca atttccaccc tggtgtgaac tgactgacaa atgcggcccg     120
   aaaacagcga atgaaatgtc tgggtgatcg gtcaaacaag cggtgggcga gagaacgcgg     180
   gtgttggcct agccgggatg ggggtaggta gacggcgtat taccggcgag ttgtccgaat      240
   ggagttttcg gggtaggtag taacgtagac gtcaatggaa aaagtcataa tctccgtcaa      300
   aaatccaacc gctccttcac accgcagagt tggtggccac gggaccctcc acccactcac     360
   tcaatcgatc gcctgccgtg gttgcccatt attcaaccat acgccacttg actcttcacc     420
   aacaattcca ggccggcttt cgagacaatg tactgcacag gaaaatccaa tataaaaggc     480
   cggcctccgc ttccttctca gtagccccca gctcattcaa ttcttcccac tgcaggctac    540
   atttgtcaga cacgttttcc gccatttttc gcctgtttct gcggagaatt tgatcaggtt     600
<210> 7
   <211> 591
   <212> DNA
   <213> Eucalyptus grandis
<220>
   <221> 5'UTR
   <222> (1)...(591)
<221> TATA_signal
   <222> (432) ... (437)
<400> 7
   agtttggaat gtgttgtgtg tgatgtgatg gagagtatca gcattccaaa catgacatgg 60
   ttttaactta tctgcaatgg tttctttttt attcagcgaa ctcgatggct gatgctgaga 120
   gaaatgaatt gggaagtcga tcgacaatgg cagctcaact caatgatcct caggtataag 180
   catttttttg gcagctctgg tcattgtgtc ttcaactttt agatgagagc aaatcaaatt 240
   gactctaata ccggttatgt gatgagtgaa tcatttgctt ttagtagctt taatttatgc 300
   ccccatctta gttgggtata aaggttcaga gtgcgaagat tacatctatt ttggttcttg 360
   caggacacag ggattcatgc tagacacatc agcagtgttt ctacgttgga tagtggtatg 420
   tacttagcta ctataaagga aattttgata gatatgtttg atatggtgct tgtacagatc 480
   tatttaatgt caatgtattt gaaactatct tgtctcataa ctttcttgaa gaatacaatg 540
   atgagactgg gaaccctatc tggaagaata gagtggagag ctggaaggac a 591
<210> 8
   <211> 480
   <212> DNA
   <213> Eucalyptus grandis
<220>
   <221> 5'UTR
   <222> (1) ... (480)
<400> 8
   atgctgagag aaatgaattg ggaagtcgat cgacaatggc agctcaactc aatgatcctc 60
   aggtataagc atttttttgg cagctctggt cattgtgtct tcaactttta gatgagagca 120
   aatcaaattg actctaatac cggttatgtg atgagtgaat catttgcttt tagtagcttt 180
   aatttatgcc cccatcttag ttgggtataa aggttcagag tgcgaagatt acatctattt 240
   tggttcttgc aggacacagg gattcatgct agacacatca gcagtgtttc tacgttggat 300
   agtggtatgt acttagctac tataaaggaa attttgatag atatgtttga tatggtgctt 360
   gtacagatct atttaatgtc aatgtatttg aaactatctt gtctcataac tttcttgaag 420
   aatacaatga tgagactggg aaccctatct ggaagaatag agtggagagc tggaaggaca 480
<210> 9
   <211> 308
   <212> DNA
   <213> Eucalyptus grandis
<220>
   <221> 5'UTR
   <222> (1)...(259)
<400> 9
   gcccatctca ggtgcaacgg tttaactgat gtttactaca cgcaaggggg aggtatccgg 60
   aaagcttgca aatcgggtaa aaacgaaaat gggcgacgtg gactcagcct gcccatgttt 120
   tcggtctctc tcctggactt ccatgcccga taagggccgc caactctctc tctctctctc 180
   tttttctctc acatctctct gcctgttcat gtcgcctgca agtgaagatt cgtcggagca 240
   agaaggacga accgggcaca tggcggggtc ggcggtcgcg acggttctaa aggtctcttt 300
   cctggtgt 308
<210> 10
   <211> 300
   <212> DNA
   <213> Eucalyptus grandis
<220>
   <221> 5'UTR
   <222> (1) ... (251)
<400> 10
   gcccatctca ggtgcaacgg tttaactgat gtttactaca cgcaaggggg aggtatccgg      60
   aaagcttgca aatcgggtaa aaacgaaaat gggcgacgtg gactcagcct gcccatgttt     120
   tcggtccctc tcctggactt ccatgcccga taaaggccgc caactctctc tctttttctc     180
   tcacatctct ctgcctgttc atgtcgcctg caagtgaaga ttcgtcggag caagaaggac     240
   gaactgggca tatggcgggg tcggcggtcg cgacggttct aaagggtctc ttcctggtgt     300
<210> 11
   <211> 297
   <212> DNA
   <213> Eucalyptus grandis
<220>
   <221> 5'UTR
   <222> (0)...(0)
<400> 11
   gtgcaacggt ttaactgatg tttactacac gcaaggggga ggtatccgga aagcttgcaa      60
   atcgggtaaa aacgaaaatg ggcgacgtgg actcagcctg cccatgtttt cggtctctct     120
   cctggacttc catgcccgat aagggccgcc aactctctct ctctctctct ttttctctca     180
   catctctctg cctgttcatg tcgcctgcaa gtgaagattc gtcggagcaa gaaggacgaa     240
   ctgggcatat ggcggggtcg gcggtcgcga cggttctaaa gggtctcttc ctggtgt        297
<210> 12
   <211> 661
   <212> DNA
   <213> Eucalyptus grandis
<220>
   <221> 5'UTR
   <222> (1)...(654)
<221> TATA_signal
   <222> (537) ... (543)
<221> CAAT_signal
   <222> (499) ... (502)
<400> 12
   ctgagccatt taattcgaga gcacatcgcc caaaattatt cttcttgctg ccataactgt      60
   cgaattttct cttttaggta agtaaccaat gatgcatcat gttgacaaaa aggctgatta     120
   gtatgatctt ggagttgttg gtgcaaattt gcaagctgac gatggcccct cagggaaatt     180
   aaggcgccaa cccagattgc aaagagcaca aagagcacga tccaaccttt ccttaacaag     240
   atcatcacca gatcggccag taagggtaat attaatttaa caaatagctc ttgtaccggg     300
   aactccgtat ttctctcact tccataaacc cctgattaat ttggtgggaa agcgacagcc     360
   aacccacaaa aggtcagatg tcatcccacg agagagagag agagagagag agagagagag     420
   agagttttct ctctatattc tggttcaccg gttggagtca atggcatgcg tgacgaatgt     480
   acatattggt gtagggtcca atattttgcg ggagggttgg tgaaccgcaa agttcctata     540
   tatcgaacct ccaccaccat acctcacttc aatccccacc atttatccgt tttatttcct     600
   ctgctttcct ttgctcgagt ctcgcggaag agagagaaga gaggagagga gagaatgggt     660
   t                                                                     661
<210> 13
   <211> 336
   <212> DNA
   <213> Pinus radiata
<400> 13
   actagtgatt tgttgagaat gagtaggcat tgctacaccc atcatcacaa gcatcatcat      60
   gaggagaaga agatccattt ctcactctat tactcgaact tccttcagat taggctgtgt     120
   atttctcact ctaccactcc aacttccttc aaatgctgtg agtttttgtt gtaattgccc     180
   cgtctattta taatcgcagc agcactcgtc atataaagac ccgtgtgtgt gaacaacaac     240
   caagtgattt gaattggaaa tgaagagcga gaatggcggt gtcatgaccg ggagcaacca     300
   gcccgggccg tcgaccacgc gtgccctata gtaatc                              336
<210> 14
   <211> 763
   <212> DNA
   <213> Pinus radiata
<400> 14
   actagtgatt tgttgagaat gagtaggcat tgctacaccc atcatcacaa gcatcaacat      60
   gaagagaaga agacgatcca tttctcactc tatcactcca acttccttca gattaggctg     120
   tgtatttctc actctaccac tccaactacc actccaactt attgccgcaa aagagagagg     180
   ttcccaaact ctgtcggaat tctcccactc aaagcattaa aggaaagatc taattgctgc     240
   aaaaaagaga gattcccaat atatttctca actcccttca aatgatttct cactctacca     300
   ctccaactcc cttcaaatga tttctcactc taccactcca acttccttca aatgctgtga     360
   gtttttgttg taattgcccc gtctatttat aatcgcagca gcactcgtca tataaagacc     420
   cgtgcgtgtg aacaacaatg gcggtgtctt gactgggagc aaccgcataa agaaagtggg     480
   cttcatacat taaaaaaatc tgtaaatttt acggatttgg aaaaaggaag agcaggaggg     540
   acctcccgac ttgacccgag aatggcggtg tcttgaccgc gtaaagaaag tggtcttctg     600
   tacccgactt gacccgaaaa aagaggaaac gttgaacgag acaatctctg ggaacttcat     660
   cgaaatgaac ctcacgactt gactctttcg attgtactgt tttcattgtt cccgcgtaaa     720
   acgaccagcc cgggccgtcg accacgcgtg ccctatagta atc                       763
<210> 15
   <211> 40
   <212> DNA
   <213> Artificial sequence
<220>
   <223> Made in a lab
<400> 15
   acggataaca gagtctttat attaaacgaa atggtattgc 40
<210> 16
   <211> 51
   <212> DNA
   <213> Artificial sequence
<220>
   <223> Made in a lab
<400> 16
   tgacgcggcc gcgaccgacg aaaagaaaaa tataacataa gagagtctga a 51
<210> 17
   <211> 27
   <212> DNA
   <213> Artificial Sequence
<220>
   <223> Made in a lab
<400> 17
   tatagcggcc gcgggggggg ggggggg 27
<210> 18
   <211> 30
   <212> DNA
   <213> Artificial Sequence
<220>
   <223> Made in a lab
<400> 18
   cggagaacaa ggtggagggt agattctttc 30
<210> 19
   <211> 31
   <212> DNA
   <213> Artificial Sequence
<220>
   <223> Made in a lab
<400> 19
   tctgcatctt gaaattgaaa tatgactaac g 31
<210> 20
   <211> 363
   <212> DNA
   <213> Eucalyptus grandis
<220>
   <221> 5'UTR
   <222> (1)...(363)
<400> 20
   aatcgggtga aaatagggcc gccctaaatt agaattgaca acatttcttg ggcaaagtta      60
   atgtaagtta catgaaaaaa aaaaaaaagg atagtttgtt ggaagtaatg gagcatttgt     120
   attgtgaaat tcacgataga gctaacaaaa ataaaggtag ttggtgggtt aacccagtta     180
   aaaaagaaca ataatttgaa gagaggagag agagagagag gagggggaga gcatttcgat     240
   aaattcacta gaaaaaatgc gtgttttagt ataaatgaga gtggaaatag ggccatctag     300
   ggaacgatcg atcgcccctg cacccggcca tctggagagt ctgtttatac ttctctccgg     360
   ctt                                                                   363
<210> 21
   <211> 839
   <212> DNA
   <213> Pinus radiata
<400> 21
<210> 22
   <211> 881
   <212> DNA
   <213> Eucalyptus grandis
<400> 22
   acgtgacgat gctcgagtct cgcgttctcc tctctcttgt tctgcaaaac agaaaagaga 60
   gaatggaggt tggcctctct caattacgtg gacgccaatg agataactca ggtgggcgac 120
   aaaacaaacg cctcttgatt tcctcaaacc ccaaaccgaa tccctcgtca aggggcaagg 180
   cttttggtcc cgcggcccca cggatcgctc gttcccgtct cgccacgtcg cgtcgcagcg 240
   tgtcgagcaa acagaggggt ccgagcgact ataaaatccc gacgccatcg acaccacagt 300
   ccatcgaaaa ccttgttcaa ttcccaagtg aaagtgagta actgtgaacg aagagttgaa 360
   ctttgcatct cggcgtgtgg attcaagagg aagcagcaaa gtggaaatgg acaactccaa 420
   gatgggcttc aatgcagggc aggccaaggg ccagactcag gagaagagca accagatgat 480
   ggataaggca tccaacactg ctcaatctgc aagggattcc atgcaagaga ctggtcagca 540
   gatgaaggcc aaagcccagg gtgctgctga tgcagtgaag aatgccaccg ggatgaacaa 600
   atgaagagct caagacatga atgaataaat aattaagctc tggttatcat ttgcttttcc 660
   ggtcgtttgt tgtcctgttt ttccttgtca agagcttatt atgagggtcc ttttgctctt 720
   tccttagttc tttttgtttc ttggttgttc catgaagaga gcaactctct gtgtttgaga 780
   gtactcatct cgcttcataa ggtctcagta tgtagttgcc tttcgagaat gttatgttct 840
   ctctcataat gctattctga ttttataaaa aaaaaaaaaa a 881
<210> 23
   <211> 350
   <212> DNA
   <213> Eucalyptus grandis
<400> 23
   ctatagggca cgcgtggtcg acggcccggg ctggtccttt cttacaaaaa gcaaaattct 60
   tataattttt tttgatataa taaaaatgat ccataaactt ttgcttaatg tgcaacgtaa 120
   accataatat attcaacgtg atgcttaaac tttaatcgag tatgcaatgt agtccataat 180
   atattcaata tgatccttca atccaattga agtgtgcaat gtggtcgcta gattttttta 240
   tgtattcaac ttagtcttta agctaccaac cttccaataa tttatgtttt agaaataata 300
   tcgaacatct tttatattat tcaaggaata aaacgaacat gcatcaaaag 350
<210> 24
   <211> 49
   <212> DNA
   <213> Eucalyptus grandis
<400> 24
   actatagggc acgcgtggtc gacggcccgg gctggtactt tttttttct 49
<210> 25
   <211> 909
   <212> DNA
   <213> Eucalyptus grandis
<400> 25
   cagggtaaag aaaatggaat atttgcttgg ccccccagct ttgaaagttg ctgtaagaac 60
   acactcacct tgcatttata cgatggttgt gagcagtgca ggctggtggt gctgcaaatt 120
   tatgatgctg atgtgatagg cagatgaatg gcagttgagc taagttaaag ccctcataca 180
   tagatcagag caggaggagt agtatatata ggcatcttgg caagtcccta aaagagcggc 240
   ttcgtgtatt cccacatatt cctctctcgt tagaacgttc agaaatgggt ggccctttga 300
   ctcttgatgc agaggttgag gttaagtctc ctgcagacaa gttctgggtg agcgtgagag 360
   actccaccaa actgttccca aagatcttcc cggaccagta caagaatatt gaagtccttg 420
   agggagatgg gaaggctcct ggctcagttc gcctcttcac gtatggtgaa ggttctccac      480
   ttgttaaagt atcaaaggag aagattgatg gtgtggacga agcagacaag gtcgtgacct      540
   acagcgttat agacggtgat ctcctgaagt actacaagaa tttcaatggc agcatcaagg      600
   taattcctaa aggagacgga agcttggtga aatggtcgtg tgggtttgag aaggcaagcg      660
   atgaaattcc tgatccccac gtaatcaagg acttcgcaat ccagaatttc aaagagcttg      720
   atgagttcat cctcaaggca tagatgccgc caatcgtcta tccggatttg cactaaatat      780
   caataaaata atgcggagct ggactccgca cttctatatg catctagtat gagagtcccc      840
   tgctgtctct gtttgtattc acttgaaggg ttttctatta agctctcttt actgcctccg      900
   aaaaaaaaa                                                              909
<210> 26
   <211> 430
   <212> DNA
   <213> Eucalyptus grandis
<400> 26
   tggagcttga gatagatcga ccgagagatc ccagcggaaa tagaagattt cctgatacca       60
   tcgatccttc ttctccaatg gctgcgaatt tcgtcattcc gaccaaaatg aaggcttggg      120
   tgtaccgtga gcacggaaac gtcgccgacg tattgggatt ggacccggaa ctcaaggtcc      180
   ctgaattgca agaaggccaa gtgctggtta aagttcttgc cgcagcgctc aatccagtcg      240
   acgccgcgag aatgaagggg gttatcaagc tcccgggctt ttctctaccg gccgtgccag      300
   gttacgatct cgccggcgtt gtggtaaagg tgggccgcga agtgaaggag ctcaagatcg      360
   gggacgaggt atatggattt atgtttcacg ccaagaaaga cgggacgctg gctgagtacg       420
   cagccgtgga                                                             430
<210> 27
   <211> 1253
   <212> DNA
   <213> Eucalyptus grandis
<400> 27
   gcttgagata gatcgactga gagatcctag tggaaataga agatttcctg ataccatcga       60
   tccattcttc tccaatggct gcgaatttcg tcattccaac caaaatgaag gcttgggtgt      120
   accgtgagca cggagacgtc gccaacgtat tgggattgga cccggaactc aaggtccctg      180
   aattgcaaga aggccaagtg ctggttaaag ttcttgccgc ggcgctcaat ccaatcgaca      240
   ccgcgagagt gaagggggtt atcaagctcc cgggcttttc tctaccggcc gtgccaggtt     300
   acgatctcgc cggcgttgtg gtgaaggtgg gccgcgaagt gaaggagctc aaggtcgggg      360
   acgaggtata tggatttatg tttcacgcca agaaagacgg gacgctggct gagtacgcag      420
   ccgtggaaga gtcgttcttg gctttgaagc ccaagaagct gcgtttcggg gaggctgctt      480
   ctctgccggt ggtcattcag accgcctatg gaggccttga aagagctggc ctctctcatg      540
   gcaagtccct cctcgtctta ggtggtgctg gtggcgtcgg cacactcata atacagctag      600
   ctaaggaagt ttttggtgca tcaagagtag cagctacatc cagcactggg aagctagagt      660
   tgttgaagag cttgggtgct gatctggcca ttgactacac caaagtcaac tttgaagacc      720
   tcccagaaaa gtttgatgtt gtctacgata cagttgggga aattgagcgg gcagcgaagg      780
   ctgtgaagcc aggagggagc atcgtgacga tcgtaaaaca aaacaagaca ttacccccgc      840
   ctgctttctt ttttgcagta acttcgaacc gttcgacctt ggagaagttg aagcccttct      900
   tggagagcgg gaaggtgaag ccggtgatcg accccaagag cccgttccca ttttcgcaag      960
   ccattgaggc cttctcgtat cttcaaaccc gccgggcaac tggaaaactc gtgattcacc     1020
   ccgtcccatg atacacaaac gagaaagaaa taaagcgtcc acatggatct gccttaatca     1080
   cgagtcctta attagtagtc gatggtgctt gctgtttgtc tccgtacatt cagcttctct     1140
   ttgcatagta gtttctacat agtgcgtgta gagaagcaag tggatgtaca agtaaaataa     1200
   ttactttttc tataaacaat attacaaact caaaaaaaaa aaaaaaaaaa aaa            1253
<210> 28
   <211> 99
   <212> DNA
   <213> Eucalyptus grandis
<400> 28
   gatagatcga ccgagagatc ccagcggaaa tagaagattt cctgatacca tcgatccatt      60
   cttctccaat ggctgcgaat ttcgtcattc cgaccaaaa                              99
<210> 29
   <211> 927
   <212> DNA
   <213> Eucalyptus grandis
<400> 29
   cgacgtcgca tgctcccggc cgccatgcgg ccgcgggaat tcgattacta tagggcacgc      60
   gtggtcgacg gcccgggctg gtactctcac taattcttta gttttccaat ttagcccctt     120
   ctgtaattgc tcatcttctt taccaaattc tctaatttgg ccggcgaagg gctgacaagg     180
   gattggtcat gtcaccctca ccaaaggttg ccgaaggtcc ggtgacctca gctgacggcc     240
   acctacacca aatctagctc actagcagcc taagcccttc atcaactcta gtgaaaggtt     300
   ttgagtattt tttaataaaa aatatttaaa aaatatatag cgagagctca ttacaaaaaa     360
   attttaaaaa aaaatctaaa cattacttga actcaaagtg actttataaa gagtttttac     420
   caaaggatct tggtttcatc atttgcacta cacccaaaac ccaatttcta agttaaatca     480
   aacccactgt ctaatagaga taaggtaaat gttataaacc aaattccaaa attccgaagc     540
   actaaatata tttgctgatc ttataatcgc caattgagag ggtctcattc tccaagggat     600
   tgtgacatat tagtaattga tagggtctca tccgtaggac tccgactcag ccgcgccacg     660
   tgactggatc gctgaacggc gcggaaccag aggagcgtga ttacctaata ttttctccta     720
   ccttggcctt gagattgaat ttcagaaaaa gaaaaagaaa aaggaacaac ttcgccgact     780
   gttctataaa atgcatgcgc caccccgacc cccacccacg catcacatcc atccagcctc     840
   cacgacagac gcataaacac aacacacgtc ggttagagag agagagagag agagagagag     900
   agagagagag atgcttggac agttgtc                                         927
<210> 30
   <211> 411
   <212> DNA
   <213> Eucalyptus grandis
<400> 30
   actatagggc acgcgtggtc gacggcccgg gctggtctga aactgtcgct cggcgatgca      60
   taccaaaggc tgaaggtatc agaatctaat gcagcttatg taaaagcgcg atcaatttat     120
   tgaccccgac gaccttgact ccatacttca cgcctcagct ttgtgttgga tggtcttgac     180
   ctctctcacc ctaaaaggta gctcaaaaga atgagacttt ccgtcatact tataaaccga     240
   ccaccagcct ctttcacaac cgacatggga caacctcaaa tagaattttt aacaacaccc     300
   ttgcacgctc tttctatcca ctttattatg ccatcacatg agcgttttcc acgcgtaaat     360
   cggctaccac ccactttcac acggcggcga aacgagaaaa aggtcctacc t              411
<210> 31
   <211> 178
   <212> DNA
   <213> Eucalyptus grandis
<400> 31
   cgagtcagca gaaacccagt tacactccgc ccaaacggaa gctaaacctg atgggccata      60
   cgatttcttt cactgagcct cttgcttttc ctccggaatc tcacggcacc ggaatgccgg     120
   aggaacttgg gaagaaccaa tgatgcctgg tcactgagtg atcgatgaat gcaatagt       178
<210> 32
   <211> 178
   <212> DNA
   <213> Eucalyptus grandis
<400> 32
   gtccaatgtc ctgtcaaagg aggaaagatg actatggccc cggcgccggc ggggactgca      60
   tgggatttag tatgttgatt gagtacccgt cgccaccacc ttcaagtaaa tcaggagtca     120
   gcagaaaccc agtacactcg ccaaacggag ctaaacctga tggccatacg atttcttt       178
<210> 33
   <211> 178
   <212> DNA
   <213> Eucalyptus grandis
<400> 33
   gcatgggatt tagtatgttg attgagtacc cgtcgccacc accttcaagt aaatcaggag      60
   tcagcagaaa cccagtacac tcgccaaacg gagctaaacc tgatggccat acgatttctt     120
   tcactgagcc tcttgctttt cctccggaat ctcacggcac cggaatgccg gaggcaac       178
<210> 34
   <211> 1274
   <212> DNA
   <213> Eucalyptus grandis
<400> 34
   ctatagggca cgcgtggtcg acggcccggg ctggtccttt cttacaaaaa gcaaaattct      60
   tataattttt tttgatataa taaaaatgat ccataaactt ttgcttaatg tgcaacgtaa     120
   accataatat attcaacgtg atgcttaaac tttaatcgag tatgcaatgt agtccataat     180
   atattcaata tgatccttca attttaattg aatgtgcaat gtggtcgcta gattttttta     240
   tgtattcaac ttagtcttta agctaccaac cttccaataa tttatgttta gaaataatat     300
   cgaacatctt ttatattatt caaggaataa aacgaacatg catcaaaagt ttaaatatat     360
   caaataaaat aaaattttaa gaattatatt acatattaaa attaaagttc atgattaaat      420
   tgaaataaaa taaaaattta aaaatcacgt tgtatgttgt gccgaaacaa aattcagtga     480
   cttgtggtgt caattttctt aggtggagct ccacaagcat tgagatggag tgttccttcc     540
   gccgaggttt tcattgcgtg gctcaaaacg gtggcgcgtt ttgcacgaca cgagatgcct     600
   cgattgccgc atcgtgtagg cgacgcaacg gaaaaacgcg ttgccgtggc gtctatccgg     660
   ggtttcgtct ccgatgcggc acgtagccta taaatgcgca cgatctcccg gtctgccaat     720
   tcgctatcga ttgcagaaga aaactcaaac cctaggcgct ctctctccgt tcgacctctc     780
   gaagttctcc tctcttcgcg tcaagatgca aatctttgtg aaaaccctta ctggcaagac     840
   aatcaccctc gaggtggaaa gctcggacac agtcgataat gtgaaagcaa aaatccagga     900
   caaggaaggg atccctccgg accagcagag gcttatcttt gctggcaagc agctggaaga     960
   tggccgaacc ttggccgatt ataacattca gaaggagtcc accctccact tggtgctccg    1020
   tctcagggga ggcatgcaaa tttttgtgaa gactcttact ggcaagacaa tcaccctcga    1080
   ggtggaaagc tccgacacag ttgataatgt gaaagcaaaa atccaggaca aggaagggat    1140
   ccctccggac cagcagaggc ttatctttgc tggcaagcag ctggaagatg gccgaacctt    1200
   ggccgattat aacattcaga aggagtccac cctccacttg gtgctccgtc tcaagggagg    1260
   catgcaaatc tttg                                                      1274
<210> 35
   <211> 795
   <212> DNA
   <213> Eucalyptus grandis
<400> 35
   aaaaatacag gctttcgaaa gctagtgcgg tataaataac ctgggaaaag caagccgctt     60
   gagctttagt ttcagtcagc catggccact cacgcagctc ttgctccctc aaccctcccc    120
   gccaatgcca agttctctag caagagctcc tctcactcct tccccactca atgcttctct    180
   aagaggctcg aggtggcgga attctcaggc cttcgtgctg gatcgtgtgt gacttatgcg    240
   aagaatgccg gggagggatc cttcttcgat gctgtggctg ctcagctcac tcccaagact    300
   tcagcaccag ctccagctaa gggagagact gtcgctaaac tgaaggtggc aatcaatggt    360
   ttcggtcgca ttggtcggaa cttccttaga tgctggcacg ggagaaagaa ctcgcccctt    420
   gatgtcattg ttgtcaatga cagcggtggt gtcaaaaatg cttcacattt gctgaagtat    480
   gattccatgc tggggacttt caaagctgat gtgaaaattg tggacaatga gaccatcagc    540
   gtcgatggga agcccgttaa ggtcgtctct aaccgggacc ctctcaagct cccctgggct    600
   gagctcggca tcgacattgt cattgaggga actggagtct tcgtggatgg ccctggtgct    660
   ggaaaacata ttcaagctgg tgccaagaaa gttatcatca ctgcaccagc aaaaggcgct    720
   gatataccca cctacgtcta tggtgtgaat gagacagatt attcgcatga agttgctaac    780
   ataatcagca atgct                                                      795
<210> 36
   <211> 1200
   <212> DNA
   <213> Eucalyptus grandis
<400> 36
   aaaatatcca tcgacagcat caccccgctt agagaacggt gtctcggctt ctcacaatgt     60
   ctatagccga atgtacaaaa tcggcataat gttctataat atagcggact ttacagatga    120
   gcattcaaat acgtacgccg tactcgattc ccattcgatt gttcattcat ccgcatgcaa    180
   atttcataga gataatatct gtgcacgtcc ttagattaag aacaaccaaa gagtatctgg    240
   tggaagtttg aagcatgacc accgaagtca gatggaacaa acaaggtggg tggtggggat    300
   atagtggaca aaggaacgag aggtgaatag gaaaaggaga aggcaagatg cgggagatag    360
   gatttacgtg gcgagcggcg attgcacgca tggtccaccc caccctcaac ctcaaacttt    420
   cgaaaatgca acgggcatca gggtggcgat gaaggagacg atggagatat tgttgctttc    480
   tccccccaaa aaacatcatc caatccatcc ccattcctca tcttcaccac aaggagtctg    540
   aagctctcct tcaccggtcc gtcgctttct ctcttatctt cttcttctcc ctcctcttct    600
   cgttcttcct tcgaccgttc tctcggtatc gtgaatttat tgcggggtgg ttcgcatgct    660
   ataaattcca cagcaacgag ggccccttgc cacaatgtcg acgtctccgg ttagcagctg    720
   gtgcgccacc tccttctccc ctgcccattc ctcgctcaag agagccgccg gcctacggcc    780
   ctctctctcc gcccgcctcg gcccttcctc ctcctcctcc tccgtctctc ctccgaccct    840
   catccgtaac gagcccgttt tcgccgcccc cgcccctgtc atcaacccca cttggacaga    900
   agagatgggc aaggactatg acgaggccat tgaggctctc aagaaactcc tcagtgagaa    960
   gggggacctg aaagccacag cagccgcaaa agtggagcaa ataactgcgg agttgcaaac   1020
   tgcttcccca gacatcaagc catccagctc cgttgacaga atcaaaactg gcttcacctt   1080
   cttcaagaag gagaaatacg acaagaaccc tgctttatat ggtgaactgg caaagcagag   1140
   tccaaagttc atggtgtttg cttgctcgga ctcgagagtg tgcccatctc atgtgctgga   1200
<210> 37
   <211> 648
   <212> DNA
   <213> Eucalyptus grandis
<400> 37
   cgacggactc ctttcacgat atcgaaacga ggaaacggag gagaagcaga agaaagaaga      60
   tgaagaaagg cagatggttg gtgatggatg aaactgtcgg gaagctggga gcttcaggga     120
   gttctattta tggggcgaaa caggggaggg gaaaccgaat ttaccaagat gcccttcttg     180
   gtgggattgg acatggagct gcacgaccgt cgtcccatca cgaagagtct tgctcttcgg     240
   tacacatgca atcgtcggcg aaccgacctt atccgaccgg ttccaagctt gtcctggtaa     300
   aaggtttcga accttggaaa aggcttaaga gatgtatcgg tgccttaacc attattccat     360
   gttcacataa tatttggccc ggttttcagg tcaattttgg agtagcccgg ttcggttcta     420
   gtcccgctcc cgattcaaaa attcattggg aacaaatttt gacactgtct ggtatttttg     480
   gtctaagacc ctacccaatt ttagaactgt acacccttgc tttatcccaa aataaaattg     540
   tcaattagtc aacttttcac acttgatgat cgattaagta gatggatgac atggtctttt     600
   accagcccgg gccgtcgacc acgcgtgccc tatagtgagt cgtattac                  648
<210> 38
   <211> 288
   <212> DNA
   <213> Eucalyptus grandis
<400> 38
   gattgtaata cgactcacta tagggcacgc gtggtcgacg gcccgggctg gtatcgtgaa      60
   agaagtccgt cgacgacaat ggccgagaag agcaaggtcc tgatcatcgg agagaagagc     120
   aaggtcctga tcatcggaga gaagagcaag gtcctgatca tcggagagaa gagcagggtc     180
   cttatcatcg gagaatcgaa ttcccgcggc cgccatggcg gccgggagca tgcgacgtcg     240
   ggcccaattc gccctatagt gagtcgtatt acaattcact ggccgtcg                  288
<210> 39
   <211> 382
   <212> DNA
   <213> Eucalyptus grandis
<400> 39
   acagcaatct catctgatga ttcttcagtt cggagctcag aggatacatc atctatagct      60
   gaattgagct gtgcaatctt ctcggcaagc accttcctcg ttttctgaaa atcatcagat     120
   tttaaggtga atccatattt cgcagatggc catgttactg ctacactctc ttcacagcat     180
   acatgaagga ggtcacatag caagcataca taggacctca tatacaaata tgacagcaga     240
   ccagcccggg ccgtcgacca cgcgtgccct atagtagtag tggggaagga gtgagaggag     300
   ctcttgatga ggaatgtcgg cttttcttcc atcagttgat gttccgggtt cctagtcatt     360
   atgccgatgg tggccactcc ag                                              382
<210> 40
   <211> 986
   <212> DNA
   <213> Eucalyptus grandis
<400> 40
   aaatacaaac tggtttaata ttcaactcag ataattacat gacaccacct aaataatgga 60
   aagtcaagca aatagacata ttatccccac acataatcaa ctatattcat gactggagag 120
   gtgctagatg gtatagagtc cctagttatt atttattttt ttgggcccga gaagatcctg 180
   atggatctat gctgtttgat actttcagat ttgttttgtc tacagctcaa ataaattagt 240
   gcttgggttt tgatatatta tctaatctga tacaagtctt tgtcctggcc aatttttgca 300
   gagtttcctg caaaacagtg cactaaagct tccagaggac ctcatgccat gcccaagggc 360
   accacctatg atggaacgga gaatcaaacc acagactgaa caggcgttga aatgccccag 420
   atgtgattct acaaacacaa aattctgtta ctataacaac tacaatcttt cacaacctcg 480
   ccatttctgc aagacctgca ggcgatactg gaccaaagga ggtgccttac gtaacgttcc 540
   tgttggtggg ggttgcagaa agaataaacg agccaagcga gcagtagacc atcctgtctc 600
   tgctcagaat gaagcatcca cctctgcagc cccaggcaac gaagtacctg accggtctcc 660
   ctttgagcca ccatcttcaa aatccattta ctatggggga gaaaacatga acttaaccgg 720
   tctccccttt agcagaattc agcaggaccg agctgcattg gcccactgca actcttcttc 780
   ctttctagga atgtcatgtg gcacccaatc ggcctctctg gaaccacatc tttcggcttt 840
   aaatacattt aattcattca agtctaacaa tcctggtctg gattttccta gcttaagcac 900
   agaccagaat tcactgtttg agaccagcca gccacaactg tcaagagcaa tggcatctgc 960
   ccttttttct atgccaatgg ctcctg 986
<210> 41
   <211> 313
   <212> DNA
   <213> Pinus radiata
<400> 41
   aaaggaaaat tcaaagatct ttagccaatt tttgttgttg tgaccttgaa tttctaaaaa 60
   atttaatgga ttcgttttct aaattcctga ttcgtcaaag gctgaagggc acgatagtaa 120
   tagaaaatgg acggcagttt atcctttcat ggctggacac acagaatttg tggagggact 180
   ctccattctg gtttatccgc cgttagttct ctctgtactc cacccttagt tctctttgta 240
   ctcgagacct ttaatgatta gccctgctta tgctgtcatt actgaactca cttccagagc 300
   cccaaaaatc tct 313
<210> 42
   <211> 713
   <212> DNA
   <213> Pinus radiata
<400> 42
   taattcacaa gtagaaaatg agatttttgc aattttgtaa ctaacatttc ccggtctcct 60
   ctgtatgttt tcacccctta atgtaattga aatttgcacc cgggttagat tcaaagcgga 120
   gaataacatc ggggccttgt tctagacaga gatttttcac aaataacagg ttcgaaggta 180
   tgtgtagaca tctgggtagt tgtagaataa agacggagcc cattaggtga tccaatcgaa 240
   gagctcagat gggaaaacag ataaaaatta tcgggtggac cttccttcac atgttaatta 300
   tatatcaagt gtcgccaatc cttatgtgaa acatttagta aagcttcgcc agagcacttc 360
   ttataggcat tctgtgggct ctgttgttgt ggttggaagt actcctttaa gggaggtatc 420
   tgaatatttg caacagaagt cagttaaaca agtggttgac tgtctgtttg tacaagatgt 480
   tactggcata cctgtgggct tgatagagac ttccaggcgc attgtgcatg taaatcattt 540
   ggtgatgcag aagctagccg gagtagagtc tatagagccc actgaagcaa ttggtgtaat 600
   caagcttcct agcagcttct acaacttgga atctcttgaa attcactcta gttcccagat 660
   atggtgctcg tcgccacatc gtctgcttgt acttgatggc attcaggatc ctg 713
<210> 43
   <211> 28
   <212> DNA
   <213> Pinus radiata
<400> 43
   ccacctcaca tcaataaatt ttatacga 28
<210> 44
   <211> 35
   <212> DNA
   <213> Pinus radiata
<400> 44
   gctgtttcat tggggtcata gctacgtggt gctga 35
<210> 45
   <211> 1729
   <212> DNA
   <213> Pinus radiata
<400> 45
   cttattgaca tataaaagca aagttggatc catctgttat tttgggtccc ctccagaagc 60
   cttactaaat gcggacaaaa aatccacgta aagaacttct gaatttaccg tcatctgggc 120
   tctgtaatta cgaatttagg gtttcctctg tcaatatctg gtagtgacaa acaaggttta 180
   atggcagcct tagcaacaac tgaagtttgt gatacatatc cacgccttgt ggagaatggt 240
   gagcttcgtg tcttgcaacc aattttccag atatatggtc gacgtcgagc tttctctgga 300
   cctatagtta cactgaaggt ctttgaggac aatgtccttt tgcgggaatt ccttgaggag 360
   agaggtaatg gaagagtttt ggtagttgat ggaggaggaa gccttagatg tgccatactg 420
   gggggcaatg tagttgtatc tgcccaaaac aatggttggt ctggaataat tgtcactggc 480
   tgcataaggg acgttgatga aataaacaga tgtgacattg gtataagagc actgacatct 540
   aacccactga aggccaacaa gaagggtgtg ggtgaaaaac atgcgcctat ttacattgct 600
   ggtacccgca ttcttccggg ggaatggtgt tatgctgaca gtgatggtat tcttgtttca 660
   cagcaagagt tatcactgtg agataataaa attcataagt ttcagattgt gactttcatg 720
   tcctgtggaa catatatttg actcgagtta gattctaata ggattaattg atagattctg 780
   aaaattgagg aatatctctg gtcatgaaaa tcttcttctc atgtgatctt ttatgctcag 840
   ctttgagtac aggatgataa gaagtttgtg catgtttgtc taaaggttta gcaagtatta 900
   tcggaccatc ataagagata gattatggaa ctcagggact tgctattttt aatccaaaat 960
   aacatttatt ctttgtgttt ttgccaaatt aacttttatt tcccttggca ccactagtga 1020
   tttgcaatat ccagttgctg agaacataga agtgggcaac ggtgagagtt gcaacagtat 1080
   ctagcataga tttaacaagt attgttggat cattataaga aaataaacta cagaaccaag 1140
   ggaatctagt tgacaacata gttaaagtag gcatggtgct actgtatcga tacatcttca 1200
   taaacagaaa aatatgaaca agctctaatg atgggagaaa ctccagcttg gtgttttgat 1260
   taagcatcca tattcacacc taaaaggtta caagttccaa aataaaaatt ccaatgaatt 1320
   tagccaatct aatcagacct tataagaaat acactaggca tctggggatc aaaatccagt 1380
   agtttagaaa gtagttgtaa ataacccaga gacaaaaatc tcaatgatag cttgcttggg 1440
   tcataggttt gataataatt gaaaacatag ttgaaaggag aatcctagca atggctagct 1500
   tgaataatag atgtacagca aaattacagt agttgagaac aaagatggaa ggataatccc 1560
   aacgatagct agcttggaca gtaggatgat tacatcaaaa tcatagcagt tgagaacata 1620
   gttggaagga gaatccttat gatggctacg ttggataata ggcgtgatta tcgtaggtag 1680
   attagagcac aagatcaaac taatagctgg cgcagctatc gactatttt 1729
<210> 46
   <211> 1038
   <212> DNA
   <213> Pinus radiata
<400> 46
   tgattactat agggcacgcg tggtcgacgg cccgggctgg taaatgagaa catgataagc      60
   tgtgtaaatt catgctagtc accataactt ttctcattgc ttttcatcca cactgttgat     120
   tcattcatta tataagatca gattcgtatg atatacaggc aaccatagaa acaaccagca     180
   aagttactag caggaaatcc aactaggtat catgaagact accaacgcag gctcgataat     240
   gttggtgctc attatttttg ggtgctgttt cattggggtc atagctacat cttttgattt     300
   ctattacttc gttcaacagt ggcctggttc atactgcgat actcgtagag gatgctgtta     360
   ccctcgcacg ggaaggcctg cttccgaatt ttccattcat ggcctctggc ccaactacaa     420
   gaccggtaaa tggccacagt tctgtggttc ctccgaagaa ttcgactact caaagatctc     480
   agatctggag gaggagctga acaggtattg gggttcgtta agctgtccaa gcagcgatgg     540
   acaggaattt tggggacacg agtgggagaa acatggcact tgctctctca atcttgatga     600
   gcattcatac tttgagaagg ctctctcctt gagacaaaat atagacattc ttggggctct     660
   taaaactgca ggtattaaac ccgatggaag ccaatacagt ttgagcgata tcaaggaagc     720
   cattaaacaa aacactgggc agctcccagg aatcgattgc aacacgagcg cagagggaga     780
   gcatcaacta tatcaggtgt atgtgtgtgt tgataaatcc gatgcttcca ctgttattga     840
   atgccccatt tatccacaca gcaattgccc atccatggtt gtgtttcctc cttttgggga     900
   ggatcaggag gaccgagatg gttacacaga aggaatgtac gagctgtaga tctggacaaa     960
   cagcatttct tctctccgca tttgattttt atcaatgaaa tttccgattc caacattttg    1020
   taaaaaaaaa aaaaaaaa                                                  1038
<210> 47
   <211> 91
   <212> DNA
   <213> Pinus radiata
<400> 47
   aattttccat tcatgcctct gcccaactac aagaccggta aatggccaca gttctgtggt      60
   tcctccgaag aattcgatat caagcttatc g                                     91
<210> 48
   <211> 91
   <212> DNA
   <213> Pinus radiata
<400> 48
   gcttttcatc cacactggtg cctcattcat tatataagat cagattcgtg tgatatacag      60
   gcaaccatag aaacaaccgg caaagttact a                                     91
<210> 49
   <211> 809
   <212> DNA
   <213> Pinus radiata
<400> 49
   tgatatatat aacttctagc agaatgacac gcgacttgta tatcttttca ttttttaacc      60
   catgaaaacc gattagggta ttgcaaatta gggcattgcc attcaaataa ttctcagatg     120
   aaagattctc tctaacaatt acaaatgatt atttttttcc atgagtgttg catgttcgaa     180
   cggtctgccc agtctgtgag agagcataga gaaccctccc tgcccaattt gttagagcat     240
   agagaaccct actgcatgag tagtaagaaa aatattcggt ctcaattcgg caaagaccac     300
   ctcgaatgga tgacttcaac gacaatctca tgatagtgtt ctgatcagca ccagttcacc     360
   tatatatttt atctagggtt tagtttgcat gtatcaatcc tctggtgcac taggtaattc     420
   tttcccagta tcatatatcc ttaatactgt tttgtctttt aatccatggc taccatcaga     480
   acaagctcaa agcagaataa gggagcatca gccatcctct tgcttatcgc gattgcaggg     540
   ttagtaaatg cgtgcaacgc tgtgggtatt gagccaatgt gcgacactgt ggtgtcgagt     600
   cttctgaggc ttctgccatg caggacggct gttgatccct caattgccgc cattccactt     660
   ccaagctgct gcaacgcggt tgagtcagct gggcttcaat gcctctgtct cgtcgttaac     720
   ggccctcctt ttccaggggt cgaccgcggc ctcgcaatgc agctgcctgc caaatgccat     780
   ctcacccttc ctccctgtaa cagttagtt                                       809
<210> 50
   <211> 428
   <212> DNA
   <213> Eucalyptus grandis
<400> 50
   tttcttgtga ctattcattt tcctcctgat tatccattca agcccccgaa ggttgcattt      60
   aggactaaag ttttccaccc aaatataaat aacaatggaa gtatctgcct tgacatcttg     120
   aaggaacagt ggagtcctgc tttgacaatc tccaaggttt tgctctcaat ttgctctttg     180
   ttgacggatc caaacccaga tgatcctctt gtaccagaga ttgctcatat gtacaagact     240
   gataggggca aatatgagtc cactgcacgg agttggactc agaaatatgc aatgggttaa     300
   ctttaaaaac tatatatcag tgatggaact ttatccctaa gttggaatct cttcgaatca     360
   atgacttgtt tgcttgtaag aaatgtttcc ttaagataag tggctttcct caaaacttga     420
   ttgaagtg                                                              428
<210> 51
   <211> 525
   <212> DNA
   <213> Pinus radiata
<400> 51
   cccttctttg ccttcaacta atcctgctca tcctctcctg cccccattcc caaagatggc      60
   tgcacccaga tcatccgcta aattgggtgc acttttggca atactgctca tagttgcggc     120
   agcgcaggct caagattgct caaatgccat ggacaaattg gctccatgca cttcagcagt     180
   gggactgtct agcaatggag tgaagccctc atctgagtgc tgtgatgccc tcaaaggaac     240
   cagtactggc tgcgtctgca agtctgtgag agcagtgata tcacttcctg ctaagtgcaa     300
   tctcccagcc ataacctgct ctggatctcg ctgaaggctc tctgttatgg cgattctcag     360
   atcgtggatc tctttaagat tttcagcaag caagtgatag aataaattct cagattttga     420
   gatatctata tagcgatttt cagtatcaga ttgtctatag tactcatata tttaagtgat     480
   tgaatagcat tctccgattc cgagttggaa acacagacac aatga                     525
<210> 52
   <211> 1126
   <212> DNA
   <213> Pinus radiata
<400> 52
   actagtgatt actatagggc acgcgtggtc gacggcccgg gctggtaaat acccaactta      60
   atttaattgt tattgagcca gagagatgcg tagtcgctca tgtcacttgt gtttaccaaa     120
   aagacataca taaacacctg cacctaaaag ttataatgat aacatgcata caaccctaca     180
   acgtacgtag tcacatgcgg ctagaactta aacccctacc acaaacatag ccacctgcac     240
   ccagaagtta taataataac atacatagaa cccttacaat aaaaaaagtt atctccaatg     300
   attattaatc tactgcaggc cagccatact cagcttgaac gtgaaaattc gcattgtaag     360
   catggcgcca cattaaaata acctcggcaa tattttcatg tccaagtggc cggccagcca     420
   cgctcctcgc actctgagaa tactctattc atccacttgt ctctgccccg caactcatat     480
   aaatgtggcc aacccaagca ccatatccat gttcattaat cccctctttg ccttcaacta     540
   atcctgctca tcccctcttg ccccaattcc caaagatggc tgcacccaga tcatccgcta     600
   aatcggctgc acttttcgca atactgctca tagttgcggc agtacaggct gaagattgct     660
   caaatgccat ggacaaattg gctccatgca cttcagcagt gggactgtct agcaatggag     720
   tgaagccctc atctgagtgc tgtgatgccc tcaaaggaac cagtactggc tgcgtctgca     780
   aatctgtgag agcagtgata tcacttcctg ctaagtgcaa tctcccagcc ttaacctgct     840
   ctggatctcg ctgaaggctc tctgttatgg cgattctcag atcgtggatc tctttaagat     900
   tttcaggaag caagtgatag aataaattct cagatgttga gatatctata tagcgatttt     960
   cagtatcaga ttgtctacag taccaatata tttaagtgat tgaatggaat tctcggattc    1020
   tgagatagaa atataggcac agaatgtggc cggaggaatg ttcgaattcg agaatgataa    1080
   taaataataa atgattgatt tctctctgca aaaaaaaaaa aaaaaa                  1126
<210> 53
   <211> 454
   <212> DNA
   <213> Pinus radiata
<400> 53
   atcctgctca tcctctcctg cccccattcc caaagatggc tgcacccaga tcatccgcta      60
   aattgggtgc acttttggca atactgctca tagttgcggc agcgcaggct caagattgct     120
   caaatgccat ggacaaattg gctccatgca cttcagcagt gggactgtct agcaatggag     180
   tgaagccctc atctgagtgc tgtgatgccc tcaaaggaac cagtactggc tgcgtctgca     240
   agtctgtgag agcagtgata tcacttcctg ctaagtgcaa tctcccagcc ataacctgct     300
   ctggatctcg ctgaaggctc tctgttatgg cgattctcag atcgtggata tctttaagat     360
   tttcagcaag tgatagaata aattctcaga ttttgagata tctatatagc gattttcagt     420
   atcagattgt ctatagtact catatattta agtg                                 454
<210> 54
   <211> 335
   <212> DNA
   <213> Pinus radiata
<400> 54
   agaagcacct gttaaaaagg aggcctgctc tttgttcatg agcttataga taagccctag      60
   tctgcaagga ttattgccct gtagttattt ggaagtagat cattttcaca ggcccagatg     120
   cattatattc taatgcagtt gtttgttaat tgaagtgcaa atagttccaa aatgtttaca     180
   tgaatcaata gtgaacaaat ccctctgttt tatatcatat tgatggatta ttcgattttt     240
   tggtgacgtg gcgcgaaact gcttttcgaa ctcatggaaa tagtaattgt tataatccat     300
   aggcatgaga ttcttgttaa tcgtgcacaa ggttt                                335
<210> 55
   <211> 336
   <212> DNA
   <213> Pinus radiata
<400> 55
   aaaccttgtg cacgattaac aagaatctca tgcctatgga ttataacaat tactatttcc     60
   atgagttcga aaagcagttt cgcgccacgt caccaaaaaa tcgaataatc catcaatatg    120
   atataaaaca gagggatttg ttcactattg attcatgtaa acattttgga actatttgca    180
   cttcaattaa caaacaactg cattagaata taatgcatct ggtgcctgtg aaaatgatct    240
   acttccaaat aactacaggg caataatcct tgcagactag ggcttatcta taagctcatg    300
   aacaaagagc aggcctcctt tttaacaggt gcttct                              336
<210> 56
   <211> 532
   <212> DNA
   <213> Pinus radiata
<400> 56
   cgttcgttcc cttccctttc cattgttgcg tttaagccct ccaattttct tttggcgtcc     60
   cgtttttggg gctcccttga agatctcctc ttcatttcgg gatttcctgc cttcgccgcg    120
   ccatttgaag ttctttttct gagagaagaa tttagacatg gctgatcgca tgttgactcg    180
   aagccacagc cttcgcgagc gtttggacga gaccctctct gctcaccgca acgatattgt    240
   ggccttcctt tcaagggttg aagccaaggg caaaggcatc ttgcagcgcc accagatttt    300
   tgctgagttt gaggccatct ctgaggagag cagagcaaag cttcttgatg gggcctttgg     360
   tgaagtcctc aaatccactc aggaagcgat tgtgtcgcct ccatgggttg ctcttgctgt    420
   tcgtccaagg ccgggcgtgt gggagcacat ccgtgtgaac gtccatgcgc ttgttcttga    480
   gcaattggag gttgctgagt atctgcactt caaagaagag cttgctgatg ga            532
<210> 57
   <211> 3103
   <212> DNA
   <213> Eucalyptus grandis
<400> 57
   gggtgaaaac aattaatgag atcatttgaa ttaaggaaag tggaaaggcg gttttctgat     60
   tggtacactg aaacaacagg aaggtggtgg aggccgcaat gatggaattt atccacttta    120
   atcattttat gaaatcgata cactaacctt tgtttctcct aaacccaaag gcattaatcc    180
   ctgtcctcct cactcgatct cgaaggccag aagggggagg ccgagcctct tgcttttttt    240
   cgtgtataaa agggcctccc ccattcctca tttttcacca tcctccgttc gttcgttccc    300
   ttccctttcc attgttgcgt ttaagccctc caattttctt ttggcgtccc gtttttgggg    360
   ctcccttgaa gatctcctct tcatttcggg atttcctgcc ttcgccgcgc catttgaagt    420
   tctttttctg agagaagaat ttagacatgg ctgatcgcat gttgactcga agccacagcc     480
   ttcgcgagcg tttggacgag accctctctg ctcaccgcaa cgatattgtg gccttccttt     540
   caagggttga agccaagggc aaaggcatct tgcagcgcca ccagattttt gctgagtttg     600
   aggccatctc tgaggagagc agagcaaagc ttcttgatgg ggcctttggt gaagtcctca     660
   aatccactca ggaagcgatt gtgtcgcctc catgggttgc tcttgctgtt cgtccaaggc     720
   cgggcgtgtg ggagcacatc cgtgtgaacg tccatgcgct tgttcttgag caattggagg     780
   ttgctgagta tctgcacttc aaagaagagc ttgctgatgg aagcttgaat ggtaactttg     840
   tgcttgagct tgactttgag ccattcactg cctcttttcc gcgcccgact ctttccaagt     900
   ctattggcaa tggcgtcgag tttctcaatc gccatctctc cgctaagctc ttccatgaca     960
   aggaaagctt gcaccctctg cttgaattcc tccaagtcca ctgctacaag gggaagaaca     1020
   tgatggtgaa tgccagaatc cagaatgtgt tctccctcca acatgtcctg aggaaggcgg    1080
   aggagtatct gacctcgctc aaacccgaga ccccgtactc ccagttcgag cacaagttcc    1140
   aggagatcgg gctcgagcgg gggtggggtg acacggctga gcgcgtcctc gagatgatcc    1200
   agctcctgtt ggatctcctt gaggctcccg acccgtgcac tctcgagaag ttcttggata    1260
   gggttcccat ggtcttcaac gtcgtgatca tgtctcccca cggatacttt gctcaggacg    1320
   acgtccttgg ttatccggat accggtggcc aggttgttta catcctggat caagttcgtg    1380
   ccctagagga agaaatgctt caccgcatta agcaacaagg actggatatt actcctcgga    1440
   ttctcattat cactcggctt cttccagacg cggttggaac cacctgtggc cagcgccttg    1500
   agaaagtttt tgggaccgag tactcccaca ttcttcgcgt ccccttcaga aatgagaagg    1560
   gagtcgtccg caagtggatt tcccggttcg aggtgtggcc ctatttggaa agatacactg    1620
   aggatgtcgc gagcgaactt gctggagagt tgcagggcaa gcctgatctg atcatcggaa    1680
   actacagtga tggaaacatt gttgcttcct tgttagcaca taaattaggt gttacacagt    1740
   gtacaatagc ccatgccctc gagaagacga agtacccaga gtcagacata tactggaaga    1800
   aatttgagga aaagtaccac ttctcttgcc agttcactgc tgatctcatc gccatgaacc    1860
   acaccgactt cattatcacc agcaccttcc aagaaattgc tggaagcaag gatacagtgg    1920
   ggcagtatga gagtcacatg aacttcactc ttcctggact ctaccgagtt gtccacggga    1980
   tcgacgtctt cgacccgaag ttcaacattg tttcaccagg tgctgacatg agcatctact    2040
   ttgcttacac cgaacaggag cggcggttga aatccttcca ccctgagatc gaggaactcc    2100
   tcttcagcga tgttgagaac aaggaacact tgtgtgtgtt gaaagataag aagaagccta    2160
   ttattttcac catggcaagg ctggaccgtg tcaagaactt gacagggctt gttgagtggt    2220
   atggcaagaa ctccaagttg agggaactcg ccaacttggt cgtggttgga ggtgacagga    2280
   ggaaggattc gaaggacttg gaagagcagt ctgagatgaa gaaaatgtac gacctcatcg    2340
   aaaagtacaa gctgaatggc cagttcaggt ggatttcctc ccagatgaac cgggtgagga    2400
   atggagagct ctaccgctac atctgtgaca cgaagggagt cttcgttcaa ccggctatct    2460
   atgaagcttt cgggttgacc gtggttgagg ccatgacttg tggattgcca acctttgcca    2520
   cttgcaatgg tggaccagct gagatcattg tgcatggcaa atcgggctac cacattgatc    2580
   cttaccatgg tgaccaggcg gccgagcttc ttgtagactt cttcaacaag tgcaagattg    2640
   accagtccca ctgggacgag atctcaaagg gtgccatgca gagaattgaa gagaagtata    2700
   catggaaaat atattctgag aggctgttga acctgactgc cgtgtatggc ttctggaagc    2760
   atgtgactaa ccttgatcgg cgcgagagtc gccggtacct tgaaatgttc tatgccctca    2820
   agtatcgccc actggcacag tctgttcctc cggctgtcga gtaaacaaag agacagattg    2880
   ttaccagaag acggaagcat tggacttttg aagttttcaa ggaataaaca ttggaaattg    2940
   tttgaatttg ggattgccaa gagcgatctt tttcgtttcc tttttttggt cctttttctc     3000
   ttctttgttt ccattccgcg aatgtttgca ttttggggtt tgtacccatc aattcagtaa    3060
   atggttcatt ttcttttcaa aaaaaaaaaa aaaaaaaaaa aaa                        3103
<210> 58
   <211> 326
   <212> DNA
   <213> Eucalyptus grandis
<400> 58
   ctcgaaaccg agacgctgac tgtgggttga gctctaacca atgggagtga tgtctctctt      60
   acgtgcctgc cgtgggcccc agtgacgggc cccaaaagtg taaacgaagg aagctcccgg     120
   ggatctgatt ggccgcgacg tccgcctctg acgtggcacc accgacgatt tttttttaat    180
   atcttggtca agtcctaatt taactatggg gtccagatta gaagcttatc cactatggat     240
   taaattaaat caaatgggaa ttaaattaaa ttaaaatcat cgtgcggagg tgcacgagat     300
   gcacgagatc cgacggcgca gagcag                                          326
<210> 59
   <211> 311
   <212> DNA
   <213> Eucalyptus grandis
<400> 59
   attactatag ggcacgcgtg gtcgacggcc cgggctggta ctctcactaa ttctttagtt      60
   ttccaattta gccccttctg taattgctca tcttctttac caaattctct aatttggccg     120
   gcgaagggct gacaagggat tggtcatgtc accctcacca aaggttgccg aaggtccggt     180
   gacctcagct gacggccacc tacaccaaat ctagctcact agcagcctaa gcccttcatc     240
   aactctagtg aaaggttttg agtatttttt aataaaaaat atttaaaaaa tatatagcga     300
   gagctcatta c                                                          311
<210> 60
   <211> 2096
   <212> DNA
   <213> Eucalyptus grandis
<400> 60
   gattactata gggcacgcgt ggtcgacggc ccgggctggt ctgagccatt taattcgaga 60
   gcacatcgcc caaaattatt cttcttgctg ccataactgt cgaattttct cttttaggta 120
   agtaaccaat gatgcatcat gttgacaaaa aggctgatta gtatgatctt ggagttgttg 180
   gtgcaaattt gcaagctgac gatggcccct cagggaaatt aaggcgccaa cccagattgc 240
   aaagagcaca aagagcacga tccaaccttt ccttaacaag atcatcacca gatcggccag 300
   taagggtaat attaatttaa caaatagctc ttgtaccggg aactccgtat ttctctcact 360
   tccataaacc cctgattaat ttggtgggaa agcgacagcc aacccacaaa aggtcagatg 420
   tcatcccacg agagagagag agagagagag agagagagag agagtttctt ctctatattc 480
   tggttcaccg gttggagtca atggcatgcg tgacgaatgt acatattggt gtagggtcca 540
   atattttgcg ggagggttgg tgaaccgcaa agttcctata tatcgaacct ccaccaccat 600
   acctcacttc aatccccacc atttatccgt tttatttcct ctgctttcct ttgctcgagt 660
   ctcgcggaag agagagaaga gaggagagga gagaatgggt tcgaccggat ccgagaccca 720
   gatgaccccg acccaagtct cggacgagga ggcgaacctc ttcgccatgc agctggcgag 780
   cgcctccgtg ctccccatgg tcctcaaggc cgccatcgag ctcgacctcc tcgagatcat 840
   ggccaaggcc gggccgggcg cgttcctctc cccgggggaa gtcgcggccc agctcccgac 900
   ccagaacccc gaggcacccg tcatgctcga ccggatcttc cggctgctgg ccagctactc 960
   cgtgctcacg tgcaccctcc gcgacctccc cgatggcaag gtcgagcggc tctacggctt 1020
   agcgccggtg tgcaagttct tggtcaagaa cgaggacggg gtctccatcg ccgcactcaa 1080
   cttgatgaac caggacaaaa tcctcatgga aagctggtat tacctgaaag atgcggtcct 1140
   tgaaggcgga atcccattca acaaggcgta cgggatgacc gcgttcgagt atcatggcac 1200
   cgacccgcga ttcaacaaga tctttaaccg gggaatgtct gatcactcca ccattactat 1260
   gaagaagata ctggaaacat acaagggctt cgagggcctc gagaccgtgg tcgatgtcgg 1320
   aggcggcact ggggccgtgc tcagcatgat cgttgccaaa tacccatcaa tgaaagggat 1380
   caacttcgac cgccccaacg gattgaagac gccccacccc ttcctggtgt caagcacgtc 1440
   ggaggcgaca tgttcgtcag cgttccaaag ggagatgcca ttttcatgaa gtggatatgc 1500
   catgactgga gtgacgacca ttgcgcgaag ttcctcaaga actgctacga tgcgcttccc 1560
   aacaatggaa aggtgatcgt tgcagagtgc gtactccctg tgtacccaga cacgagccta 1620
   gcgaccaaga atgtgatcca catcgactgc atcatgttgg cccacaaccc aggcgggaaa 1680
   gagaggacac agaaggagtt cgaggcattg gccaaagggg ccggatttca gggcttccaa 1740
   gtcatgtgct gcgctttcgg cactcacgtc atggagttcc tgaagaccgc ttgatctgct 1800
   cctctgtggt gatgttcatg gttcttggat ttgaaaggtc gtgaaggagc ccttttctca 1860
   cagttggctt cggcatacca agttcttctc ataaaaggaa acaataagaa gcgactgtat 1920
   gatggcgcaa gtggaagtta caagatttgt tgttttatgt ctataaagtt ttgagtcttc 1980
   tgcatactga tttcacagaa tgtgtaacga aacggcgtat atggatgtgc ctgaatgatg 2040
   gaaattgtga tattctgtct tctttttcag taaatcactt cgaacaaaaa aaaaaa 2096
<210> 61
   <211> 522
   <212> DNA
   <213> Eucalyptus grandis
<400> 61
   ctaaaacgct aatcctgccc tgcccttccc ttctgctgct gctgctcgtc acctctctct 60
   ccctctcgcg gccagctgcg agatctgccg agtttaagcc tcgtacatca aaatgggtaa 120
   ggagaagatt cacatcagca ttgtggtcat tggccatgtc gattctggga agtcaaccac 180
   aactggccac ttgatataca agctcggagg aatcgacaag cgtgtgattg agagattcga 240
   gaaggaagct gctgagatga acaagagatc gttcaagtat gcttgggtgc ttgacaagct 300
   caaggccgag cgcgagcgcg gtattaccat tgatattgcc ttgtggaagt tcgagaccac 360
   caagtactac tgcactgtca ttgatgctcc tggacatcgt gactttatta agaatatgat 420
   tactggaacc tcccaggccg actgtgctgt ccttatcatt gattccacca ctggtggttt 480
   cgaagctggt atttccaagg atggccagac ccgtgaacat gc 522
<210> 62
   <211> 420
   <212> DNA
   <213> Eucalyptus grandis
<400> 62
   tttgatacgc taacaaacaa aacatgtgaa aagcttaatt atggcaatta tcataaatag      60
   aaaaaaatta gaaaaaaaga gaggaaatgg gccattattt aaattgcaat cgaaagattg     120
   agggcaattc tgtttctcta gtgtaaataa gggtgtattt aataattgag ggatggaaat     180
   agcatggtca ctcggtaatt atcaaggaaa gcaagaataa aaatggaaaa aaaaaaaaaa     240
   aaagcttgaa gaggccaatg tcgaaattat gagcgcgaga tgaggacact cctgggaaac     300
   gaaaaatggc attcgcgggg ggtgctatat aaagcctcgt gtaagggtgc gttcctcact     360
   ctcaaaccct aatcctgccc ttcccttctg ctgctgctgc tcgtcacctc tctcctccct     420
<210> 63
   <211> 65
   <212> PRT
   <213> Eucalyptus grandis
<400> 63
   Met Asp Asn Ser Lys Met Gly Phe Asn Ala Gly Gln Ala Lys Gly Gln
    1               5                  10                  15
   Thr Gln Glu Lys Ser Asn Gln Met Met Asp Lys Ala Ser Asn Thr Ala
               20                  25                  30
   Gln Ser Ala Arg Asp Ser Met Gln Glu Thr Gly Gln Gln Met Lys Ala
           35                  40                  45
   Lys Ala Gln Gly Ala Ala Asp Ala Val Lys Asn Ala Thr Gly Met Asn
       50                  55                  60
   Lys
   65
<210> 64
   <211> 152
   <212> PRT
   <213> Eucalyptus grandis
<400> 64
   Met Gly Gly Pro Leu Thr Leu Asp Ala Glu Val Glu Val Lys Ser Pro
    1               5                  10                  15
   Ala Asp Lys Phe Trp Val Ser Val Arg Asp Ser Thr Lys Leu Phe Pro
               20                  25                  30
   Lys Ile Phe Pro Asp Gln Tyr Lys Asn Ile Glu Val Leu Glu Gly Asp
           35                  40                  45
   Gly Lys Ala Pro Gly Ser Val Arg Leu Phe Thr Tyr Gly Glu Gly Ser
       50                  55                  60
   Pro Leu Val Lys Val Ser Lys Glu Lys Ile Asp Gly Val Asp Glu Ala
   65                  70                  75                  80
   Asp Lys Val Val Thr Tyr Ser Val Ile Asp Gly Asp Leu Leu Lys Tyr
                   85                  90                  95
   Tyr Lys Asn Phe Asn Gly Ser Ile Lys Val Ile Pro Lys Gly Asp Gly
               100                 105                 110
   Ser Leu Val Lys Trp Ser Cys Gly Phe Glu Lys Ala Ser Asp Glu Ile
           115                 120                 125
   Pro Asp Pro His Val Ile Lys Asp Phe Ala Ile Gln Asn Phe Lys Glu
       130                 135                 140
   Leu Asp Glu Phe Ile Leu Lys Ala
   145                 150
<210> 65
   <211> 117
   <212> PRT
   <213> Eucalyptus grandis
<400> 65
   Met Ala Ala Asn Phe Val Ile Pro Thr Lys Met Lys Ala Trp Val Tyr
    1               5                   10                  15
   Arg Glu His Gly Asn Val Ala Asp Val Leu Gly Leu Asp Pro Glu Leu
               20                  25                  30
   Lys Val Pro Glu Leu Gln Glu Gly Gln Val Leu Val Lys Val Leu Ala
           35                  40                  45
   Ala Ala Leu Asn Pro Val Asp Ala Ala Arg Met Lys Gly Val Ile Lys
       50                  55                  60
   Leu Pro Gly Phe Ser Leu Pro Ala Val Pro Gly Tyr Asp Leu Ala Gly
   65                  70                  75                  80
   Val Val Val Lys Val Gly Arg Glu Val Lys Glu Leu Lys Ile Gly Asp
                   85                  90                  95
   Glu Val Tyr Gly Phe Met Phe His Ala Lys Lys Asp Gly Thr Leu Ala
               100                 105                 110
   Glu Tyr Ala Ala Val
           115
<210> 66
   <211> 318
   <212> PRT
   <213> Eucalyptus grandis
<400> 66
   Met Ala Ala Asn Phe Val Ile Pro Thr Lys Met Lys Ala Trp Val Tyr
   1               5                   10                  15
   Arg Glu His Gly Asp Val Ala Asn Val Leu Gly Leu Asp Pro Glu Leu
               20                  25                  30
   Lys Val Pro Glu Leu Gln Glu Gly Gln Val Leu Val Lys Val Leu Ala
           35                  40                  45
   Ala Ala Leu Asn Pro Ile Asp Thr Ala Arg Val Lys Gly Val Ile Lys
       50                  55                  60
   Leu Pro Gly Phe Ser Leu Pro Ala Val Pro Gly Tyr Asp Leu Ala Gly
   65                  70                  75                  80
   Val Val Val Lys Val Gly Arg Glu Val Lys Glu Leu Lys Val Gly Asp
                   85                  90                  95
   Glu Val Tyr Gly Phe Met Phe His Ala Lys Lys Asp Gly Thr Leu Ala
               100                 105                 110
   Glu Tyr Ala Ala Val Glu Glu Ser Phe Leu Ala Leu Lys Pro Lys Lys
           115                 120                 125
   Leu Arg Phe Gly Glu Ala Ala Ser Leu Pro Val Val Ile Gln Thr Ala
       130                 135                 140
   Tyr Gly Gly Leu Glu Arg Ala Gly Leu Ser His Gly Lys Ser Leu Leu
   145                 150                 155                 160
   Val Leu Gly Gly Ala Gly Gly Val Gly Thr Leu Ile Ile Gln Leu Ala
                   165                 170                 175
   Lys Glu Val Phe Gly Ala Ser Arg Val Ala Ala Thr Ser Ser Thr Gly
               180                 185                 190
   Lys Leu Glu Leu Leu Lys Ser Leu Gly Ala Asp Leu Ala Ile Asp Tyr
           195                 200                 205
   Thr Lys Val Asn Phe Glu Asp Leu Pro Glu Lys Phe Asp Val Val Tyr
       210                 215                 220
   Asp Thr Val Gly Glu Ile Glu Arg Ala Ala Lys Ala Val Lys Pro Gly
   225                 230                 235                 240
   Gly Ser Ile Val Thr Ile Val Lys Gln Asn Lys Thr Leu Pro Pro Pro
                   245                 250                 255
   Ala Phe Phe Phe Ala Val Thr Ser Asn Arg Ser Thr Leu Glu Lys Leu
               260                 265                 270
   Lys Pro Phe Leu Glu Ser Gly Lys Val Lys Pro Val Ile Asp Pro Lys
           275                 280                 285
   Ser Pro Phe Pro Phe Ser Gln Ala Ile Glu Ala Phe Ser Tyr Leu Gln
       290                 295                 300
   Thr Arg Arg Ala Thr Gly Lys Leu Val Ile His Pro Val Pro
   305                 310                 315
<210> 67
   <211> 156
   <212> PRT
   <213> Eucalyptus grandis
<400> 67
   Met Gln Ile Phe Val Lys Thr Leu Thr Gly Lys Thr Ile Thr Leu Glu
    1               5                  10                  15
   Val Glu Ser Ser Asp Thr Val Asp Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ala Asp Tyr Asn Ile Gln Lys Glu
       50                  55                  60
   Ser Thr Leu His Leu Val Leu Arg Leu Arg Gly Gly Met Gln Ile Phe
   65                  70                  75                  80
   Val Lys Thr Leu Thr Gly Lys Thr Ile Thr Leu Glu Val Glu Ser Ser
                   85                  90                  95
   Asp Thr Val Asp Asn Val Lys Ala Lys Ile Gln Asp Lys Glu Gly Ile
               100                 105                 110
   Pro Pro Asp Gln Gln Arg Leu Ile Phe Ala Gly Lys Gln Leu Glu Asp
           115                 120                 125
   Gly Arg Thr Leu Ala Asp Tyr Asn Ile Gln Lys Glu Ser Thr Leu His
       130                 135                 140
   Leu Val Leu Arg Leu Lys Gly Gly Met Gln Ile Phe
   145                 150                 155
<210> 68
   <211> 238
   <212> PRT
   <213> Eucalyptus grandis
<400> 68
   Met Ala Thr His Ala Ala Leu Ala Pro Ser Thr Leu Pro Ala Asn Ala
    1               5                  10                  15
   Lys Phe Ser Ser Lys Ser Ser Ser His Ser Phe Pro Thr Gln Cys Phe
               20                  25                  30
   Ser Lys Arg Leu Glu Val Ala Glu Phe Ser Gly Leu Arg Ala Gly Ser
           35                  40                  45
   Cys Val Thr Tyr Ala Lys Asn Ala Gly Glu Gly Ser Phe Phe Asp Ala
       50                  55                  60
   Val Ala Ala Gln Leu Thr Pro Lys Thr Ser Ala Pro Ala Pro Ala Lys
   65                  70                  75                  80
   Gly Glu Thr Val Ala Lys Leu Lys Val Ala Ile Asn Gly Phe Gly Arg
                   85                  90                  95
   Ile Gly Arg Asn Phe Leu Arg Cys Trp His Gly Arg Lys Asn Ser Pro
               100                 105                 110
   Leu Asp Val Ile Val Val Asn Asp Ser Gly Gly Val Lys Asn Ala Ser
           115                 120                 125
   His Leu Leu Lys Tyr Asp Ser Met Leu Gly Thr Phe Lys Ala Asp Val
       130                 135                 140
   Lys Ile Val Asp Asn Glu Thr Ile Ser Val Asp Gly Lys Pro Val Lys
   145                 150                 155                 160
   Val Val Ser Asn Arg Asp Pro Leu Lys Leu Pro Trp Ala Glu Leu Gly
                   165                 170                 175
   Ile Asp Ile Val Ile Glu Gly Thr Gly Val Phe Val Asp Gly Pro Gly
               180                 185                 190
   Ala Gly Lys His Ile Gln Ala Gly Ala Lys Lys Val Ile Ile Thr Ala
           195                 200                 205
   Pro Ala Lys Gly Ala Asp Ile Pro Thr Tyr Val Tyr Gly Val Asn Glu
       210                 215                 220
   Thr Asp Tyr Ser His Glu Val Ala Asn Ile Ile Ser Asn Ala
   225                 230                 235
<210> 69
   <211> 168
   <212> PRT
   <213> Eucalyptus grandis
<400> 69
   Met Ser Thr Ser Pro Val Ser Ser Trp Cys Ala Thr Ser Phe Ser Pro
    1               5                  10                  15
   Ala His Ser Ser Leu Lys Arg Ala Ala Gly Leu Arg Pro Ser Leu Ser
               20                  25                  30
   Ala Arg Leu Gly Pro Ser Ser Ser Ser Ser Ser Val Ser Pro Pro Thr
           35                  40                  45
   Leu Ile Arg Asn Glu Pro Val Phe Ala Ala Pro Ala Pro Val Ile Asn
       50                  55                  60
   Pro Thr Trp Thr Glu Glu Met Gly Lys Asp Tyr Asp Glu Ala Ile Glu
   65                  70                  75                  80
   Ala Leu Lys Lys Leu Leu Ser Glu Lys Gly Asp Leu Lys Ala Thr Ala
                   85                  90                  95
   Ala Ala Lys Val Glu Gln Ile Thr Ala Glu Leu Gln Thr Ala Ser Pro
               100                 105                 110
   Asp Ile Lys Pro Ser Ser Ser Val Asp Arg Ile Lys Thr Gly Phe Thr
           115                 120                 125
   Phe Phe Lys Lys Glu Lys Tyr Asp Lys Asn Pro Ala Leu Tyr Gly Glu
       130                 135                 140
   Leu Ala Lys Gln Ser Pro Lys Phe Met Val Phe Ala Cys Ser Asp Ser
   145                 150                 155                 160
   Arg Val Cys Pro Ser His Val Leu
                   165
<210> 70
   <211> 214
   <212> PRT
   <213> Eucalyptus grandis
<400> 70
   Met Pro Cys Pro Arg Ala Pro Pro Met Met Glu Arg Arg Ile Lys Pro
    1               5                  10                  15
   Gln Thr Glu Gln Ala Leu Lys Cys Pro Arg Cys Asp Ser Thr Asn Thr
               20                  25                  30
   Lys Phe Cys Tyr Tyr Asn Asn Tyr Asn Leu Ser Gln Pro Arg His Phe
           35                  40                  45
   Cys Lys Thr Cys Arg Arg Tyr Trp Thr Lys Gly Gly Ala Leu Arg Asn
       50                  55                  60
   Val Pro Val Gly Gly Gly Cys Arg Lys Asn Lys Arg Ala Lys Arg Ala
   65                  70                  75                  80
   Val Asp His Pro Val Ser Ala Gln Asn Glu Ala Ser Thr Ser Ala Ala
                   85                  90                  95
   Pro Gly Asn Glu Val Pro Asp Arg Ser Pro Phe Glu Pro Pro Ser Ser
               100                 105                 110
   Lys Ser Ile Tyr Tyr Gly Gly Glu Asn Met Asn Leu Thr Gly Leu Pro
           115                 120                 125
   Phe Ser Arg Ile Gln Gln Asp Arg Ala Ala Leu Ala His Cys Asn Ser
       130                 135                 140
   Ser Ser Phe Leu Gly Met Ser Cys Gly Thr Gln Ser Ala Ser Leu Glu
   145                 150                 155                 160
   Pro His Leu Ser Ala Leu Asn Thr Phe Asn Ser Phe Lys Ser Asn Asn
                   165                 170                 175
   Pro Gly Leu Asp Phe Pro Ser Leu Ser Thr Asp Gln Asn Ser Leu Phe
               180                 185                 190
   Glu Thr Ser Gln Pro Gln Leu Ser Arg Ala Met Ala Ser Ala Leu Phe
           195                 200                 205
   Ser Met Pro Met Ala Pro
       210
<210> 71
   <211> 166
   <212> PRT
   <213> Pinus radiata
<400> 71
   Met Ala Ala Leu Ala Thr Thr Glu Val Cys Asp Thr Tyr Pro Arg Leu
    1              5                  10                  15
   Val Glu Asn Gly Glu Leu Arg Val Leu Gln Pro Ile Phe Gln Ile Tyr
               20                  25                  30
   Gly Arg Arg Arg Ala Phe Ser Gly Pro Ile Val Thr Leu Lys Val Phe
           35                  40                  45
   Glu Asp Asn Val Leu Leu Arg Glu Phe Leu Glu Glu Arg Gly Asn Gly
       50                  55                  60
   Arg Val Leu Val Val Asp Gly Gly Gly Ser Leu Arg Cys Ala Ile Leu
   65                  70                  75                  80
   Gly Gly Asn Val Val Val Ser Ala Gln Asn Asn Gly Trp Ser Gly Ile
                   85                  90                  95
   Ile Val Thr Gly Cys Ile Arg Asp Val Asp Glu Ile Asn Arg Cys Asp
               100                 105                 110
   Ile Gly Ile Arg Ala Leu Thr Ser Asn Pro Leu Lys Ala Asn Lys Lys
           115                 120                 125
   Gly Val Gly Glu Lys His Ala Pro Ile Tyr Ile Ala Gly Thr Arg Ile
       130                 135                 140
   Leu Pro Gly Glu Trp Cys Tyr Ala Asp Ser Asp Gly Ile Leu Val Ser
   145                 150                 155                 160
   Gln Gln Glu Leu Ser Leu
                   165
<210> 72
   <211> 236
   <212> PRT
   <213> Pinus radiata
<400> 72
   Met Leu Val Leu Ile Ile Phe Gly Cys Cys Phe Ile Gly Val Ile Ala
    1              5                  10                  15
   Thr Ser Phe Asp Phe Tyr Tyr Phe Val Gln Gln Trp Pro Gly Ser Tyr
               20                  25                  30
   Cys Asp Thr Arg Arg Gly Cys Cys Tyr Pro Arg Thr Gly Arg Pro Ala
           35                  40                  45
   Ser Glu Phe Ser Ile His Gly Leu Trp Pro Asn Tyr Lys Thr Gly Lys
       50                  55                  60
   Trp Pro Gln Phe Cys Gly Ser Ser Glu Glu Phe Asp Tyr Ser Lys Ile
   65                  70                  75                  80
   Ser Asp Leu Glu Glu Glu Leu Asn Arg Tyr Trp Gly Ser Leu Ser Cys
                   85                  90                  95
   Pro Ser Ser Asp Gly Gln Glu Phe Trp Gly His Glu Trp Glu Lys His
               100                 105                 110
   Gly Thr Cys Ser Leu Asn Leu Asp Glu His Ser Tyr Phe Glu Lys Ala
           115                 120                 125
   Leu Ser Leu Arg Gln Asn Ile Asp Ile Leu Gly Ala Leu Lys Thr Ala
       130                 135                 140
   Gly Ile Lys Pro Asp Gly Ser Gln Tyr Ser Leu Ser Asp Ile Lys Glu
   145                 150                 155                 160
   Ala Ile Lys Gln Asn Thr Gly Gln Leu Pro Gly Ile Asp Cys Asn Thr
                   165                 170                 175
   Ser Ala Glu Gly Glu His Gln Leu Tyr Gln Val Tyr Val Cys Val Asp
               180                 185                 190
   Lys Ser Asp Ala Ser Thr Val Ile Glu Cys Pro Ile Tyr Pro His Ser
           195                 200                 205
   Asn Cys Pro Ser Met Val Val Phe Pro Pro Phe Gly Glu Asp Gln Glu
       210                 215                 220
   Asp Arg Asp Gly Tyr Thr Glu Gly Met Tyr Glu Leu
   225                 230                 235
<210> 73
   <211> 92
   <212> PRT
   <213> Pinus radiata
<400> 73
   Met Ala Ala Pro Arg Ser Ser Ala Lys Leu Gly Ala Leu Leu Ala Ile
   1               5                   10                  15
   Leu Leu Ile Val Ala Ala Ala Gln Ala Gln Asp Cys Ser Asn Ala Met
               20                  25                  30
   Asp Lys Leu Ala Pro Cys Thr Ser Ala Val Gly Leu Ser Ser Asn Gly
           35                  40                  45
   Val Lys Pro Ser Ser Glu Cys Cys Asp Ala Leu Lys Gly Thr Ser Thr
       50                  55                  60
   Gly Cys Val Cys Lys Ser Val Arg Ala Val Ile Ser Leu Pro Ala Lys
   65                  70                  75                  80
   Cys Asn Leu Pro Ala Ile Thr Cys Ser Gly Ser Arg
                   85                  90
<210> 74
   <211> 92
   <212> PRT
   <213> Pinus radiata
<400> 74
   Met Ala Ala Pro Arg Ser Ser Ala Lys Ser Ala Ala Leu Phe Ala Ile
    1               5                  10                  15
   Leu Leu Ile Val Ala Ala Val Gln Ala Glu Asp Cys Ser Asn Ala Met
               20                  25                  30
   Asp Lys Leu Ala Pro Cys Thr Ser Ala Val Gly Leu Ser Ser Asn Gly
           35                  40                  45
   Val Lys Pro Ser Ser Glu Cys Cys Asp Ala Leu Lys Gly Thr Ser Thr
       50                  55                  60
   Gly Cys Val Cys Lys Ser Val Arg Ala Val Ile Ser Leu Pro Ala Lys
   65                  70                  75                  80
   Cys Asn Leu Pro Ala Leu Thr Cys Ser Gly Ser Arg
                   85                  90
<210> 75
   <211> 92
   <212> PRT
   <213> Pinus radiata
<400> 75
   Met Ala Ala Pro Arg Ser Ser Ala Lys Leu Gly Ala Leu Leu Ala Ile
    1               5                  10                  15
   Leu Leu Ile Val Ala Ala Ala Gln Ala Gln Asp Cys Ser Asn Ala Met
               20                  25                  30
   Asp Lys Leu Ala Pro Cys Thr Ser Ala Val Gly Leu Ser Ser Asn Gly
           35                  40                  45
   Val Lys Pro Ser Ser Glu Cys Cys Asp Ala Leu Lys Gly Thr Ser Thr
       50                  55                  60
   Gly Cys Val Cys Lys Ser Val Arg Ala Val Ile Ser Leu Pro Ala Lys
   65                  70                  75                  80
   Cys Asn Leu Pro Ala Ile Thr Cys Ser Gly Ser Arg
                   85                  90
<210> 76
   <211> 125
   <212> PRT
   <213> Eucalyptus grandis
<400> 76
   Met Ala Asp Arg Met Leu Thr Arg Ser His Ser Leu Arg Glu Arg Leu
    1               5                  10                  15
   Asp Glu Thr Leu Ser Ala His Arg Asn Asp Ile Val Ala Phe Leu Ser
               20                  25                  30
   Arg Val Glu Ala Lys Gly Lys Gly Ile Leu Gln Arg His Gln Ile Phe
           35                  40                  45
   Ala Glu Phe Glu Ala Ile Ser Glu Glu Ser Arg Ala Lys Leu Leu Asp
       50                  55                  60
   Gly Ala Phe Gly Glu Val Leu Lys Ser Thr Gln Glu Ala Ile Val Ser
   65                  70                  75                  80
   Pro Pro Trp Val Ala Leu Ala Val Arg Pro Arg Pro Gly Val Trp Glu
                   85                  90                  95
   His Ile Arg Val Asn Val His Ala Leu Val Leu Glu Gln Leu Glu Val
               100                 105                 110
   Ala Glu Tyr Leu His Phe Lys Glu Glu Leu Ala Asp Gly
           115                 120                 125
<210> 77
   <211> 805
   <212> PRT
   <213> Eucalyptus grandis
<400> 77
   Met Ala Asp Arg Met Leu Thr Arg Ser His Ser Leu Arg Glu Arg Leu
    1               5                  10                  15
   Asp Glu Thr Leu Ser Ala His Arg Asn Asp Ile Val Ala Phe Leu Ser
               20                  25                  30
   Arg Val Glu Ala Lys Gly Lys Gly Ile Leu Gln Arg His Gln Ile Phe
           35                  40                  45
   Ala Glu Phe Glu Ala Ile Ser Glu Glu Ser Arg Ala Lys Leu Leu Asp
       50                  55                  60
   Gly Ala Phe Gly Glu Val Leu Lys Ser Thr Gln Glu Ala Ile Val Ser
   65                  70                  75                  80
   Pro Pro Trp Val Ala Leu Ala Val Arg Pro Arg Pro Gly Val Trp Glu
   85 90 95
   His Ile Arg Val Asn Val His Ala Leu Val Leu Glu Gln Leu Glu Val
   100 105 110
   Ala Glu Tyr Leu His Phe Lys Glu Glu Leu Ala Asp Gly Ser Leu Asn
   115 120 125
   Gly Asn Phe Val Leu Glu Leu Asp Phe Glu Pro Phe Thr Ala Ser Phe
   130 135 140
   Pro Arg Pro Thr Leu Ser Lys Ser Ile Gly Asn Gly Val Glu Phe Leu
   145 150 155 160
   Asn Arg His Leu Ser Ala Lys Leu Phe His Asp Lys Glu Ser Leu His
   165 170 175
   Pro Leu Leu Glu Phe Leu Gln Val His Cys Tyr Lys Gly Lys Asn Met
   180 185 190
   Met Val Asn Ala Arg Ile Gln Asn Val Phe Ser Leu Gln His Val Leu
   195 200 205
   Arg Lys Ala Glu Glu Tyr Leu Thr Ser Leu Lys Pro Glu Thr Pro Tyr
   210 215 220
   Ser Gln Phe Glu His Lys Phe Gln Glu Ile Gly Leu Glu Arg Gly Trp
   225 230 235 240
   Gly Asp Thr Ala Glu Arg Val Leu Glu Met Ile Gln Leu Leu Leu Asp
   245 250 255
   Leu Leu Glu Ala Pro Asp Pro Cys Thr Leu Glu Lys Phe Leu Asp Arg
   260 265 270
   Val Pro Met Val Phe Asn Val Val Ile Met Ser Pro His Gly Tyr Phe
   275 280 285
   Ala Gln Asp Asp Val Leu Gly Tyr Pro Asp Thr Gly Gly Gln Val Val
   290 295 300
   Tyr Ile Leu Asp Gln Val Arg Ala Leu Glu Glu Glu Met Leu His Arg
   305 310 315 320
   Ile Lys Gln Gln Gly Leu Asp Ile Thr Pro Arg Ile Leu Ile Ile Thr
   325 330 335
   Arg Leu Leu Pro Asp Ala Val Gly Thr Thr Cys Gly Gln Arg Leu Glu
   340 345 350
   Lys Val Phe Gly Thr Glu Tyr Ser His Ile Leu Arg Val Pro Phe Arg
   355 360 365
   Asn Glu Lys Gly Val Val Arg Lys Trp Ile Ser Arg Phe Glu Val Trp
   370 375 380
   Pro Tyr Leu Glu Arg Tyr Thr Glu Asp Val Ala Ser Glu Leu Ala Gly
   385 390 395 400
   Glu Leu Gln Gly Lys Pro Asp Leu Ile Ile Gly Asn Tyr Ser Asp Gly
   405 410 415
   Asn Ile Val Ala Ser Leu Leu Ala His Lys Leu Gly Val Thr Gln Cys
   420 425 430
   Thr Ile Ala His Ala Leu Glu Lys Thr Lys Tyr Pro Glu Ser Asp Ile
   435 440 445
   Tyr Trp Lys Lys Phe Glu Glu Lys Tyr His Phe Ser Cys Gln Phe Thr
   450 455 460
   Ala Asp Leu Ile Ala Met Asn His Thr Asp Phe Ile Ile Thr Ser Thr
   465 470 475 480
   Phe Gln Glu Ile Ala Gly Ser Lys Asp Thr Val Gly Gln Tyr Glu Ser
   485 490 495
   His Met Asn Phe Thr Leu Pro Gly Leu Tyr Arg Val Val His Gly Ile
   500 505 510
   Asp Val Phe Asp Pro Lys Phe Asn Ile Val Ser Pro Gly Ala Asp Met
   515 520 525
   Ser Ile Tyr Phe Ala Tyr Thr Glu Gln Glu Arg Arg Leu Lys Ser Phe
   530 535 540
   His Pro Glu Ile Glu Glu Leu Leu Phe Ser Asp Val Glu Asn Lys Glu
   545 550 555 560
   His Leu Cys Val Leu Lys Asp Lys Lys Lys Pro Ile Ile Phe Thr Met
   565 570 575
   Ala Arg Leu Asp Arg Val Lys Asn Leu Thr Gly Leu Val Glu Trp Tyr
               580                 585                 590
   Gly Lys Asn Ser Lys Leu Arg Glu Leu Ala Asn Leu Val Val Val Gly
           595                 600                 605
   Gly Asp Arg Arg Lys Asp Ser Lys Asp Leu Glu Glu Gln Ser Glu Met
       610                 615                 620
   Lys Lys Met Tyr Asp Leu Ile Glu Lys Tyr Lys Leu Asn Gly Gln Phe
   625                 630                 635                 640
   Arg Trp Ile Ser Ser Gln Met Asn Arg Val Arg Asn Gly Glu Leu Tyr
                   645                 650                 655
   Arg Tyr Ile Cys Asp Thr Lys Gly Val Phe Val Gln Pro Ala Ile Tyr
               660                 665                 670
   Glu Ala Phe Gly Leu Thr Val Val Glu Ala Met Thr Cys Gly Leu Pro
           675                 680                 685
   Thr Phe Ala Thr Cys Asn Gly Gly Pro Ala Glu Ile Ile Val His Gly
       690                 695                 700
   Lys Ser Gly Tyr His Ile Asp Pro Tyr His Gly Asp Gln Ala Ala Glu
   705                 710                 715                 720
   Leu Leu Val Asp Phe Phe Asn Lys Cys Lys Ile Asp Gln Ser His Trp
                   725                 730                 735
   Asp Glu Ile Ser Lys Gly Ala Met Gln Arg Ile Glu Glu Lys Tyr Thr
               740                 745                 750
   Trp Lys Ile Tyr Ser Glu Arg Leu Leu Asn Leu Thr Ala Val Tyr Gly
           755                 760                 765
   Phe Trp Lys His Val Thr Asn Leu Asp Arg Arg Glu Ser Arg Arg Tyr
       770                 775                 780
   Leu Glu Met Phe Tyr Ala Leu Lys Tyr Arg Pro Leu Ala Gln Ser Val
   785                 790                 795                 800
   Pro Pro Ala Val Glu
                   805
<210> 78
   <211> 264
   <212> PRT
   <213> Eucalyptus grandis
<400> 78
   Met Gly Ser Thr Gly Ser Glu Thr Gln Met Thr Pro Thr Gln Val Ser
    1               5                  10                  15
   Asp Glu Glu Ala Asn Leu Phe Ala Met Gln Leu Ala Ser Ala Ser Val
               20                  25                  30
   Leu Pro Met Val Leu Lys Ala Ala Ile Glu Leu Asp Leu Leu Glu Ile
           35                  40                  45
   Met Ala Lys Ala Gly Pro Gly Ala Phe Leu Ser Pro Gly Glu Val Ala
       50                  55                  60
   Ala Gln Leu Pro Thr Gln Asn Pro Glu Ala Pro Val Met Leu Asp Arg
   65                  70                  75                  80
   Ile Phe Arg Leu Leu Ala Ser Tyr Ser Val Leu Thr Cys Thr Leu Arg
                   85                  90                  95
   Asp Leu Pro Asp Gly Lys Val Glu Arg Leu Tyr Gly Leu Ala Pro Val
               100                 105                 110
   Cys Lys Phe Leu Val Lys Asn Glu Asp Gly Val Ser Ile Ala Ala Leu
           115                 120                 125
   Asn Leu Met Asn Gln Asp Lys Ile Leu Met Glu Ser Trp Tyr Tyr Leu
       130                 135                 140
   Lys Asp Ala Val Leu Glu Gly Gly Ile Pro Phe Asn Lys Ala Tyr Gly
   145                 150                 155                 160
   Met Thr Ala Phe Glu Tyr His Gly Thr Asp Pro Arg Phe Asn Lys Ile
                   165                 170                 175
   Phe Asn Arg Gly Met Ser Asp His Ser Thr Ile Thr Met Lys Lys Ile
               180                 185                 190
   Leu Glu Thr Tyr Lys Gly Phe Glu Gly Leu Glu Thr Val Val Asp Val
           195                 200                 205
   Gly Gly Gly Thr Gly Ala Val Leu Ser Met Ile Val Ala Lys Tyr Pro
       210                 215                 220
   Ser Met Lys Gly Ile Asn Phe Asp Arg Pro Asn Gly Leu Lys Thr Pro
   225                 230                 235                 240
   His Pro Phe Leu Val Ser Ser Thr Ser Glu Ala Thr Cys Ser Ser Ala
                   245                 250                 255
   Phe Gln Arg Glu Met Pro Phe Ser
               260
<210> 79
   <211> 136
   <212> PRT
   <213> Eucalyptus grandis
<400> 79
   Met Gly Lys Glu Lys Ile His Ile Ser Ile Val Val Ile Gly His Val
    1               5                  10                  15
   Asp Ser Gly Lys Ser Thr Thr Thr Gly His Leu Ile Tyr Lys Leu Gly
               20                  25                  30
   Gly Ile Asp Lys Arg Val Ile Glu Arg Phe Glu Lys Glu Ala Ala Glu
           35                  40                  45
   Met Asn Lys Arg Ser Phe Lys Tyr Ala Trp Val Leu Asp Lys Leu Lys
       50                  55                  60
   Ala Glu Arg Glu Arg Gly Ile Thr Ile Asp Ile Ala Leu Trp Lys Phe
   65                  70                  75                  80
   Glu Thr Thr Lys Tyr Tyr Cys Thr Val Ile Asp Ala Pro Gly His Arg
                   85                  90                  95
   Asp Phe Ile Lys Asn Met Ile Thr Gly Thr Ser Gln Ala Asp Cys Ala
               100                 105                 110
   Val Leu Ile Ile Asp Ser Thr Thr Gly Gly Phe Glu Ala Gly Ile Ser
           115                 120                 125
   Lys Asp Gly Gln Thr Arg Glu His
       130                 135
<210> 80
   <211> 229
   <212> PRT
   <213> Eucalyptus grandis
<400> 80
   Met Gln Ile Phe Val Lys Thr Leu Thr Gly Lys Thr Ile Thr Leu Glu
    1               5                  10                  15
   Val Glu Ser Ser Asp Thr Ile Asp Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ala Asp Tyr Asn Ile Gln Lys Glu
       50                  55                  60
   Ser Thr Leu His Leu Val Leu Arg Leu Arg Gly Gly Met Gln Ile Phe
   65                  70                  75                  80
   Val Lys Thr Leu Thr Gly Lys Thr Ile Thr Leu Glu Val Glu Ser Ser
                   85                  90                  95
   Asp Thr Ile Asp Asn Val Lys Ala Lys Ile Gln Asp Lys Glu Gly Ile
               100                 105                 110
   Pro Pro Asp Gln Gln Arg Leu Ile Phe Ala Gly Lys Gln Leu Glu Asp
           115                 120                 125
   Gly Arg Thr Leu Ala Asp Tyr Asn Ile Gln Lys Glu Ser Thr Leu His
       130                 135                 140
   Leu Val Leu Arg Leu Arg Gly Gly Met Gln Ile Phe Val Lys Thr Leu
   145                 150                 155                 160
   Thr Gly Lys Thr Ile Thr Leu Glu Val Glu Ser Ser Asp Thr Ile Asp
                   165                 170                 175
   Asn Val Lys Ala Lys Ile Gln Asp Lys Glu Gly Ile Pro Pro Asp Gln
               180                 185                 190
   Gln Arg Leu Ile Phe Ala Gly Lys Gln Leu Glu Asp Gly Arg Thr Leu
           195                 200                 205
   Ala Asp Tyr Asn Ile Gln Lys Glu Ser Thr Leu His Leu Val Leu Arg
       210                 215                 220
   Leu Arg Gly Gly Phe
   225
<210> 81
   <211> 345
   <212> DNA
   <213> Eucalyptus grandis
<400> 81
   taataaatga tgaatttatt ataaacgtat ccgtttgaga tttttgtggg tcataggtgt      60
   atcaatttga aatctttgat agtaacaaaa ataattttag gtagtttatg tttttcatga     120
   tataaacctt gaaagttaat gctactaaat tgttatatat atattaggca aattacaacc     180
   ttaatgcaac agttaatgac gtgatactgt tcagattata gatacaatgg ttatccttga     240
   atgaataaga agaagtccta agggcaagtg ctatgagctt gcacgactgc ttttgcgcca     300
   tttttgttta ccagcccggg ccgtcgacca cgcgtgccct atagt                     345
<210> 82
   <211> 72
   <212> DNA
   <213> Eucalyptus grandis
<400> 82
   cagtagggga cttgttcccc caagggcacg tgtcgttggt gaagctctgg cggtggatga      60
   accgcgtggg cc                                                          72
<210> 83
   <211> 544
   <212> DNA
   <213> Eucalyptus grandis
<400> 83
   actagtgatt tcgtcgtctt cgtcttcttc gtcttctgga acttcgttgc tccgagcttt      60
   atcagaaccg gcgatggaaa tgaaaccctc gttctctctc cctcgctcct ctctttcttc     120
   tatccaggag cgtttgtaca ctgggagtac agagcttctt gcgataccga aactaccctt     180
   ggacgactgg cctttttgcc tcgcgccccc tctctgagcc ggggcgcaat ttgtcccttt     240
   cccagagcga agtgtcgatt ttgtccttcc acgaggcttt acctactccc atcgcccgag     300
   ccccaagccc aggcccaaat gcctgttcct tgtggccctg ccaacattcc ctttgaaatt     360
   aaaaaattaa aaaaaaactc tctgccaggc aaaagtaaag attaacacca ccaaaattta     420
   taacaaattt atcattcatt aattttcgtt aaatttattt ttcaaattac tgagtcgaat     480
   tacatgtata aattcacgga tgtatcggtt cgagatttta tcctctaatt atcattagtg     540
   tatg                                                                  544
<210> 84
   <211> 515
   <212> DNA
   <213> Eucalyptus grandis
<400> 84
   gattactata gggcacgcgt ggtcgacggc ccgggctggt ctgccttcct ttaactcccc      60
   ttttttgtaa ctttttaaaa tgtagtttta aatttaattt aattactttt tatattaatt     120
   atttaccaca tcagagacaa aacaatgtct tttttgtatt ttctagtcac gtcaacatgc     180
   aaaacaacgc cattttgcac tcaccttgcc ggaaaattgc cacgtcaaca atttggctag     240
   agtggcgctt aagtgatcta ttttgctcca attttggcac ttaagtgtca ttttcctaaa     300
   ttttagcact taaagtattc ctctatgtca agttttgaca cttggggtgt actttgtcca     360
   atcataaacc gtataagttc actttaaaca aaaatggcgc aaaagcagtc gtgcaagctc     420
   atagcacttg cccttaggac ttcttcttat tcattcaagg ataaccattg tatctataat     480
   ctgaacagta tcacgtcatt aactgttgca ttaag                                515
<210> 85
   <211> 515
   <212> DNA
   <213> Eucalyptus grandis
<400> 85
   actagtgatt tcgtcgtctt cgtcttcttc gtcttctgga acttcgttgc tccgagcttt      60
   atcagaaccg gcgatggaaa tgaaaccctc gttctctctc cctcgctcct ctctttcttc     120
   tatccaggag cgtttgtaca ctgggagtac agagcttctt gcgataccga aactaccctt     180
   ggacgactgg cctttttgcc tcgtgccccc tctctgagcc ggggcgcaat ttgtcccttt     240
   cccagagcga agtgtcgatt ttgtccttcc acgaggcttt acctactccc atcgcccgag     300
   ccccaagccc aggcccaaat gcctgttcct tgtggccctg ccaacattcc ctttgaaatt     360
   aaaaaattaa aaaaaaactc tctgccaggc aaaagtaaag attaacacca ccaaaattta     420
   taacaaattt atcattcatt aattttcgtt aaattttatt ttcaaattac tgagtcgaat     480
   tacatgtata aattcacgga tgtatcggtt cgaga                                515
<210> 86
   <211> 782
   <212> DNA
   <213> Eucalyptus grandis
<400> 86
   gagggtttca tttccatcgc cggttctgat aaagctcgga gcaacgaagt tccagaagac      60
   gaagaagacg aagacgacga cggcgacatg ccttgcttga acatctccac caacgtcagc     120
   ctcgacggcc tcgacacctc cgccattctc tccgagacca cctccggcgt cgccaagctc     180
   atcggcaagc ccgaggccta tgtgatgatt gtgttgaagg ggtcagtccc catggctttt     240
   ggtgggactg agcaacctgc tgcctatggc gagttggtgt caatcggcgg tttgaacccc     300
   gatgtgaaca agaagctgag tgctgcaatt gcttcaatcc tcgaaaccaa gctgtccatc     360
   cccaagtcgc ggttcttcct gaaattttat gataccaagg gttccttctt tggatggaat     420
   ggatccacct tctgagctgt tggtcgcatt ctcctcagtg tttaccatgt atttcggccc     480
   taaactctac ttctaggcct gttaaaagtg tcttttttaa ggtaattctg ctattacccc      540
   tcttaagtgc atcttatcag taaacatgga atatcctgaa ctttgattat atgccggctc     600
   gtggctgtgg aagcacttct ttatgttacc accagcttct caggtgaata taagctttgc     660
   ccagtctgtt ctctggggga tttgcttggt gggtagtggc aatcagatgg ttttgtcact     720
   tttgtgcata tttaagtagt aaatgtccac gacagcccaa agagtagcaa tccgggtgca     780
   ct                                                                    782
<210> 87
   <211> 115
   <212> PRT
   <213> Eucalyptus grandis
<400> 87
   Met Pro Cys Leu Asn Ile Ser Thr Asn Val Ser Leu Asp Gly Leu Asp
    1               5                  10                  15
   Thr Ser Ala Ile Leu Ser Glu Thr Thr Ser Gly Val Ala Lys Leu Ile
               20                  25                  30
   Gly Lys Pro Glu Ala Tyr Val Met Ile Val Leu Lys Gly Ser Val Pro
           35                  40                  45
   Met Ala Phe Gly Gly Thr Glu Gln Pro Ala Ala Tyr Gly Glu Leu Val
       50                  55                  60
   Ser Ile Gly Gly Leu Asn Pro Asp Val Asn Lys Lys Leu Ser Ala Ala
   65                  70                  75                  80
   Ile Ala Ser Ile Leu Glu Thr Lys Leu Ser Ile Pro Lys Ser Arg Phe
                   85                  90                  95
   Phe Leu Lys Phe Tyr Asp Thr Lys Gly Ser Phe Phe Gly Trp Asn Gly
               100                 105                 110
   Ser Thr Phe
           115
<210> 88
   <211> 1521
   <212> DNA
   <213> Pinus radiata
<400> 88
   ccttcaaaga caacagagaa agttatgcaa tatgctggca gctagctctt gggataatct      60
   atttagcgat gggtttgtcg agaagttggg agcatttatt gtgaagcttc acagaaaaaa     120
   tgtcgaatac atcaagcaca tgaagaagca atttgtgcca taggctatct ttagcctcat     180
   ggatgttaaa ataatttctt tctttccttc cttcttcttt cttacccacc aaaacacaaa     240
   ataatagttt caaattttga attttcaccc aattttatga gaggacaaaa ttacttagag     300
   tctttcactc tttaatttat attctacata agtacctaaa gaggctctcc gacaatcata     360
   tgataccata aaagtaacct cgattagaga gcgcctctcc atacaatcat ttgattttcg     420
   agttaaatca aaattatagg ctatttccaa atcaatctat cgtccaactg aaaatttcaa     480
   atgaatggaa ccagcacgga gtttcgtagg aaatagaagt aataggtgaa aagaagcatt     540
   gtcgaatttg aaagaatacc ctacgttttc atttcaaaaa ccatggtttt ttgtaagagg     600
   gattaagttg actcaaggtt gtagaaggtt gacataacaa tagcatgcag gcacaggatg     660
   catgtagtgc ccgtaatttg gaccaaccta gtaagattgt cacccgtttc aaatgactgc     720
   ctacaagtgc atgcaaaggc catggaagtt gatggttagt gaaaagatcc ggagagacga     780
   ttattccatc atgcaatgca catcgcacgc ttgctttatt actcacacga ccaacgttcc     840
   cttcatccac ggaattaatt tctctaatcg atccaataaa ccgccttcga tgtcgatttc     900
   caaatgaatt aaatcgttac atgcccaccc gacttcacac atgctccctg cacgtgcaac     960
   caaatccatt acgcccaccg ggcccggccc tgctcacaca tcttgcatcg cccaactact    1020
   ctgattttac atgaatatca atactattcc ctccacttat aaaatggcca aacgccctgc    1080
   ttagttctca aagcagatca gagcctttca agagcttccg caaagatttt ctttgcgagt    1140
   aatttgatcg agaaggatgt ctgcatcgaa cggaactaat ggtgttgtcg cagtcaagtc    1200
   tcgccgacag cacagacctg ggaaaacgac agccatggcg ttcgggaggg cgtttccaga    1260
   tcagctggtg atgcaggagt tcctcgtcga tggatatttc cgcaacacga attgccagga    1320
   ccccgtcctc cgccagaagc tcgaaaggct ttgcaagacg acgacggtga agacgcgata    1380
   cgtggtgatg tcggatgaaa tattggcgca gcatcctgag ctggcagtgg aaggttcggc    1440
   caccgtccga cagcgactcg agatctcgaa cgtggccgtg accgacatgg cggtggacgc    1500
   gtgccgtgac tgcctcaaag a                                              1521
<210> 89
   <211> 2590
   <212> DNA
   <213> Eucalyptus grandis
<400> 89
   ctgaaactgt cgctcggcga tgcataccaa aggctgaagg tatcagaatc taatgcagct      60
   tatgtaaaag cgcgatcaat ttattgaccc cgacgacctt gactccatac ttcacgcctc     120
   agctttgtgt tggatggtct tgacctctct caccctaaaa ggtagctcaa aagaatgaga     180
   ctttccgtca tacttataaa ccgaccacca gcctctttca caaccgacat gggacaacct     240
   caaatagaat ttttaacaac acccttgcac gctctttcta tccactttat tatgccatca     300
   catgagcgtt ttccacgcgt aaatcggcta ccacccactt tcacacggcg gcgaaacgag     360
   aaaaaggtcc tacctttgac tccccccgcg tcccaaattc tcactcccga ccggtaaccg     420
   agctcacaag tttcagcctt tcatcatcat cactcgaagg cagagagaag gacatacact     480
   aaagacaacg aaacagtctc tccatcccgc catccgacac gatccacatt acggtacgga     540
   acacatcccg cggagcaacc cgacgtccca aactcttcgc tgatcaaaac cagtccggtc     600
   gactccgttt cgcgcggacg caacgtgaga gagggagaga gagagagaga gtaccggcga     660
   ggggatgatg ctgtgcggaa gcgtcgtcgg gcgctctccc ggcgaacgcg tctctacatt     720
   ccggcgacgg cgacggcgac gaaggcgggg aggggaatgc cgcggggttt ctgcaacgac     780
   ggaagctcac ggcatttttc agagagagag agagagatgg cacgtcagag cgccattccc     840
   ccacgcgacg ttccgccttc cggtattcct tccgggagaa aaagtgggca aattgcaata     900
   gacaaaaaaa aaaagaaaaa aaagacggtc acccaaatta tttcttataa cacaaaaaat     960
   cgtacctata taatatatct atcactaact tgtgcagtat gacaaattta cacatttacc    1020
   tgaaactgtt tttataacat aaaaaattta aacatttttc tgtgacaata aatgttcaca    1080
   caaatataaa actgggattt ttatttcaat tacaaattta gaataaatgc gcaacataaa    1140
   tacaaattta tgatttttcg tgttggcaag aaagtttgag ataaatgtat cattgtaggt    1200
   aaagtttaga gttttttttt atggctttta accaaaatgc acattttagt tccgagttct    1260
   aaaagaaaaa ttactatttt cctttacatt tacttatgta ggtgtgtaat tataaatatt    1320
   aattctcttt aggatttgta acaattcttt gagcttttgt tttgccttta ggccattaga    1380
   attactaaaa agttaataat ataaacattt tttcgaccac ggtcaccatt catacctaac    1440
   ttctaattat tgaaagattc tcgcatttga tcgaaatcca tttactctca taaatttgag    1500
   gttttgaacg gtatctacca taagatcatg gtttattaca aaacacttat ggcgggtggc    1560
   gcggacctgg cgagaatgtg gctactttaa tgatgaggat ttgagatatt ataccacgat    1620
   ccataataat aaaggagcgc ggcaatcata tcttttttca tataaaggac gatttatttt    1680
   ctatgctgtg agtatttgct cttggaatta taagatatta gagatcaaac ctatcaccaa    1740
   cggtgatttg aaattaaaga agtccttgta tcacttacaa aaataaatat ataaaaaaag    1800
   ctttcattgt gcacttgaat atttaaacat aaattattag tagtagataa ttttttaatt    1860
   taactaataa tgagcactca tttttagaaa aatagttttc aaatcattca ttttctactt    1920
   aaaaaaacca attgaccaac taaattagta tctctcattc agttggtgaa tgaatgactc    1980
   gcactctaac ccttcacttg gcgagtcatt ctgtgtagac cagtctctgc aaatctagcc    2040
   atgctcatct agcaactacc ttcaagcgca agtactttgt catgtagacc aaacgttgag    2100
   caacacggaa tgaatcctaa cgcacttgga aaacaatcaa tccacgctac gcaagctaat    2160
   gctcacacaa gcatcatgat acccgaagcc gaaaatacat gagtcgaaag acatcgaact    2220
   ccgccgtcct cgcgaatcat ccgaatcgca tgtcacgccg ctcgacttgg tagcttaacg    2280
   agccttccag tacctgctgt ttaaatgctt tgtcaatgtg attcgaatcc tttcaaagat    2340
   cctgaaagtg cagcttcaaa aatggcgtcg accaaatggg cttgcgttgc tgcaatctcg    2400
   ctcctactga gcctaggatc gagcgctgct cagaggtctc tccttatgag cagcgccaac    2460
   tggcaagagg ccggtgagcc gacggatctg gacttacgtg gaggaattgc cggaaccctg    2520
   gggtcatcaa gtgagggcgg caccatggcc agctccgaca tgggcggttt tggccaggac    2580
   atgcctggtg                                                           2590
<210> 90
   <211> 1172
   <212> DNA
   <213> Eucalyptus grandis
<400> 90
   actctcacta attctttagt tttccaattt agccccttct gtaattgctc atcttcttta      60
   ccaaattctc taatttggcc ggcgaagggc tgacaaggga ttggtcatgt caccctcacc     120
   aaaggttgcc gaaggtccgg tgacctcagc tgacggccac ctacaccaaa tctagctcac     180
   tagcagccta agcccttcat caactctagt gaaaggtttt gagtattttt taataaaaaa     240
   tatttaaaaa atatatagcg agagctcatt acaaaaaaat tttaaaaaaa aatctaaaca     300
   ttacttgaac tcaaagtgac tttataaaga gtttttacca aaggatcttg gtttcatcat     360
   ttgcactaca cccaaaaccc aatttctaag ttaaatcaaa cccactgtct aatagagata     420
   aggtaaatgt tataaaccaa attccaaaat tccgaagcac taaatatatt tgctgatctt     480
   ataatcgcca attgagaggg tctcattctc caagggattg tgacatatta gtaattgata     540
   gggtctcatc cgtaggactc cgactcagcc gcgccacgtg actggatcgc tgaacggcgc     600
   ggaaccagag gagcgtgatt acctaatatt ttctcctacc ttggccttga gattgaattt     660
   cagaaaaaga aaaagaaaaa ggaacaactt cgccgactgt tctataaaat gcatgcgcca     720
   ccccgacccc cacccacgca tcacatccat ccagcctcca cgacagacgc ataaacacaa     780
   cacacgtcgg ttagagagag agagagagag agagagagag agagagagat gcttggacag     840
   ttgtcgcacg agacggaaat gaaggtggga gcaggcaaag catgggagct gtatggcacg     900
   ctcaagctgg tcctgctggc caagcaggaa ttctctaata ccatctgcga cgtcttggaa     960
   ggtgatggcg gcgttggcac cgtcatcaag ctcaattttg gaagtttatc ctatacagag    1020
   aagtacacaa aggtggacca cgagcgccgc gtgaaagaaa cggaggcgat cgaaggtggg    1080
   ttcctggaca tggggtctcg ctgtatcgat tgcgattcga agtgataggc aaggacgagg    1140
   aggagtcgtt ccgttattaa agcccccccc cc                                  1172
<210> 91
   <211> 446
   <212> DNA
   <213> Eucalyptus grandis
<400> 91
   gggtgaaaac aattaatgag atcatttgaa ttaaggaaag tggaaaggcg gttttctgat      60
   tggtacactg aaacaacagg aaggtggtgg aggccgcaat gatggaattt atccacttta     120
   atcattttat gaaatcgata cactaacctt tgtttctcct aaacccaaag gcattaatcc     180
   ctgtcctcct cactcgatct cgaaggccag aagggggagg ccgagcctct tgcttttttt     240
   cgtgtataaa agggcctccc ccattcctca tttttcacca tcctccgttc gttcgttccc     300
   ttccctttcc attgttgcgt ttaagccctc caattttctt ttggcgtccc gtttttgggg     360
   ctcccttgaa gatctcctct tcatttcggg atttcctgcc ttcgccgcgc catttgaagt     420
   tctttttctg agagaagaat ttagac                                          446
<210> 92
   <211> 2119
   <212> DNA
   <213> Pinus radiata
<400> 92
   atcttattcc cacctcacat caataaattt tatacgattt taacatcttt aaaattaaaa      60
   gaatcaagaa ggcatccagg tgataaagcc acgtccaata taaaatctcc tcggtggatc     120
   ctttcaatcc agctacccaa tgcggcgaaa ataacgctga ttggactggg ctacactgta     180
   atcacaaatt cccttccgtt tagatttcaa ctcgttgacc tacgagtatt ttatcgattt     240
   aaaattatac aaaaaattgt ggaatgtttt acataagcaa aacttaaata atgtaaatag     300
   cgatgatgct ttacttgtac ctaaaaattt cttccaaatt aaaccaaata tcaaatccta     360
   gattgatgag ttccagtgga gtctgccatt ttatttcttt ctctctttca ttctttgcaa     420
   cgaaaggaga aaatccttaa cacaattcga aaacgataat gattctggca aaagagaaaa     480
   aaaacgtgaa gattagacac ttgttttgtt ttaaatgagc aatcacatgt gaatagagag     540
   ggttttatgg gcctggtttt gtgtgcataa tttcttatga aagcgatgtg cctggagcgt     600
   tgaagctcat agaacattgc aacaagagat cgagagtgtg ggttagaaaa ccgcaacaat     660
   agtttgtgtc gtgtttttct atattcagag gtgttgtgtg gtaaatatct ctggatttat     720
   ctcgaatgcg tcacttttac agacacagaa gctcagcgga aaccctcaac gctttaaggg     780
   ccataaattt gctcagtttt aaaaattgtt tgatttccca ggtttgaata ttttcttttt     840
   gttatcggaa gtggctctgc cttatgagta tcatgttctt ggttttgtgt tgggcgctta     900
   ttgattcagg tatgtattat ttctagtcct ttttatcagc ataggtggaa tgttctgtat     960
   tttatatttt ggggccatac acatggaacc gttgtcatta ccatgcttta tagataatgt    1020
   ctctctgaat ttgtttttat aggcttttgc ctcctacgca gatttttaaa ggaaaataca    1080
   aagatattta gccaattttt gttgttgtga ccttgaattt ctaaaaaatt taatggattc    1140
   gttttctaaa ttcctgattc gtcaaaggct gaagggcgcg atagtaatag aaaatggacg    1200
   agagtttatc ttttcatggc tggacacaca gaatttgtgg aggggattct ccattctggt    1260
   ttatccaccg ttagttctct ctgtactcca cccttagttc tctttgtact cgagaccttt    1320
   aatgattaac cctgcttatg ctgtcagtac tgaactcact tccagagccc caaaaatctc    1380
   tcccaagttt gccttatttc ttaaaataat tcacaagtag aaaatgagat ttttgcaatt    1440
   ttgtaactaa catttcccgg tctcctctgt atgttttcac cccttaatgt aattgaaatt    1500
   tgcacccggg ttagattcaa agcggagaat aacatcgggg ccttgttcta gacagagatt    1560
   tttcacaaat aacaggttcg aaggtatgtg tagacatctg ggtagttgta gaataaagac    1620
   ggagcccatt aggtggatcc aatcgaagaa ctcagatggg aaaacagata aaaattatcg    1680
   ggtggacctt cctccacatg ttaattatat atcaagtgtc gccaatcctt atgtgaaaca    1740
   tttagtaaag cttcgccaga gcacttctta taggcattct gtgggctctg ttgttgtggt    1800
   tggaagtact cctttaaggg aggtatctga atatttgcaa cagaagtcag taaaacaagt    1860
   ggttgactgt ctgtttgtac aagatgttac tggcatacct gtgggcttga tagagacttc    1920
   caggcgcatt gtgcatgtaa atcatttggt gatgcagaag ctagccggag tagagtctat    1980
   agagcccact gaagcaattg gtgtaatcaa gcttcctagc agcttctaca acttggaatc    2040
   tcttgaaatc actctagttc ccagatatgg tgctcgtcgc cacatcgtct gcttgtactt    2100
   gatggcattc aggatcctg                                                 2119
<210> 93
   <211> 2571
   <212> DNA
   <213> Eucalyptus grandis
<400> 93
   aaggtaactg gttcagcaga gcgcagatac caaatacttg ttcttctagt gtagccgtag      60
   ttaggccacc acttcaagaa ctctgtagca ccgcctacat acctcgctct gctaatcctg     120
   ttaccagtgg ctgctgccag tggcgataag tcgtgtctta ccgggttgga ctcaagacga     180
   tagttaccgg ataaggcgca gcggtcgggc tgaacggggg gttcgtgcac acagcccagc     240
   ttggagcgaa cgacctacac cgaactgaga tacctacagc gtgagctatg agaaagcgcc    300
   acgcttcccg aagggagaaa ggcggacagg tatccggtaa gcggcagggt cggaacagga    360
   gagcgcacga gggagcttcc agggggaaac gcctggtatc tttatagtcc tgtcgggttt    420
   cgccacctct gacttgagcg tcgatttttg tgatgctcgt caggggggcg gagcctatgg    480
   aaaaacgcca gcaacgcggc ctttttacgg ttcctggcct tttgctggcc ttttgctcac    540
   atgttctttc ctgcgttatc ccctgattct gtggataacc gtattaccgc ctttgagtga    600
   gctgataccg ctcgccgcag ccgaacgacc gagcgcagcg agtcagtgag cgaggaagcg    660
   gaagagcgcc caatacgcaa accgcctctc cccgcgcgtt ggccgattca ttaatgcagc    720
   tggcacgaca ggtttcccga ctggaaagcg ggcagtgagc gcaacgcaat taatgtgagt    780
   tagctcactc attaggcacc ccaggcttta cactttatgc ttccggctcg tatgttgtgt    840
   ggaattgtga gcggataaca atttcacaca ggaaacagct atgaccatga ttacgccaag    900
   ctatttaggt gacactatag aatactcaag ctatgcatcc aacgcgttgg gagctctccc    960
   atatggtcga cctgcaggcg gccgcgaatt cactagtgat tggcccgggc tggtctggag   1020
   tggccaccat cggcataatg actaggaacc cggaacatca actgatggaa gaaaagccga   1080
   cattcctcat caagagctcc tctcactcct tccccactac tactataggg cacgcgtggt   1140
   cgacggcccg ggctggtctg ctgtcatatt tgtatatgag gtcctatgta tgcttgctat   1200
   gtgacctcct tcatgtatgc tgtgaagaga gtgtagcagt aacatggcca tctgcgaaat   1260
   atggattcac cttaaaatct gatgattttc agaaaacgag gaaggtgctt gccgagaaga   1320
   ttgcacagct caattcagct atagatgatg tatcctctga gctccgaact gaagaatcat   1380
   cagatgagat tgctgttgcc cctgatgaaa ttgaagctgc tgtttgatgg cccaaacctc   1440
   ccaggcctac gatcatggtc atcttctgtt ttggtgcaat tggctctacc tttttggtgg   1500
   cctccatata acagaataat ggttcatatt gtaaaatctt ctgtttattt ctaaagacca   1560
   atgcactcag tttcttttga tatgattgtc tcgattgagg aagtgcatca ttcgtggtat   1620
   gattatgcag aataccattt aactcagcag actttgtacc gtatcatcgc agcttttccc   1680
   ttcttgtgta tgcataaatc tagtccttca ttgaaggtga tcgccgttac agtctggata   1740
   gtgtgtgcca tcagatggca ctacgattag tgtggttgac atggtgtcaa cttgaaagcc   1800
   aattggtgac gatggtactt aatgtaagat tggcagatgg tgagaacgag attttgctcc   1860
   agaatggcaa agcaaggcta agttgtagcg aatcaaatga tctacgaacc atcctagctg   1920
   gctgtgtgac cacacactga agttctattg aactaagcca gttatggatg atatgggagg   1980
   agaaaattga gaaatccatc agatggagtg ttggccgtgt tgggcttttg tcgcaggccg   2040
   atacttcgaa ttcaggcgta tttttattcc tgactgccgc ctctcccgga aagggaaggc   2100
   ggatattatt ctctgaacga tttccaccat caactccaca tcgatctcca agccagaaat   2160
   atacacaccc caattttctt ttaaatatat gggacatata tggtgtaggc tctcgcgcat   2220
   gttaacacat aagctctctc aacaaaaatc tggctcgtgc ttttaaccga gaagttcacg   2280
   agtcattgaa ggagtggcct ttaggggagg gagagagatg gattggtggt taaaatcagt   2340
   ctgtggctca catttatacc gtggagatcc cccaacagca accttatccc attatatatc   2400
   cccacaacac catattcacc actcgttcct tctaattggc ttccaaccat aattcacaga   2460
   cacacatgta gtgaccaatg agaaaggaag aaaaatacag gctttcgaaa gctagtgcgg   2520
   tataaataac ctgggaaaag caagccgctt gagctttagt ttcagtcagc c             2571
<210> 94
   <211> 1406
   <212> DNA
   <213> Pinus radiata
<400> 94
   aaagaggcgg aggaattgtc tagatggtca aaagtgaccg gaatctaagc aaaaaatttc      60
   aaaaaatgtt gtaaaggtag cgtttgaatt gtgtttttga tggtggaaat ggattcaacg     120
   ccatcaaaaa cgtctaagac acctaaaatt ttgaatttta acaactatat cttggattta     180
   caaaaatcct tgccggattt tctctaaact ccttcacctt acgcaaaaga tatatatttt     240
   tttgtgtgat gttgtgcatt ataagtttga tagtgaagta atgatatata tcctttatgt     300
   gatggatgat tgaataatga atatattaaa tgaaataaat aatgatggga taatgaatat     360
   attatatgaa ataaatataa agtaaaatgc tattttttaa tggtgttaat gatgaattag     420
   tatcatcctt aaataatttg ttagtgaatt attaaaatga tgagttagca tggtcgttaa     480
   ataaattgtt agtgaattat tatatttata tatttcctta ttagaaagtt ttttttttgt      540
   aaaagttttc cttgaacttc acccatattt aattatcaat aatttatatt taataaatga     600
   tatatataac ttctagcaga atgacacgcg acttgtatat cttttcattt tttaacccat     660
   gaaaaccgat tagggtattg caaattaggg cattgccatt caaataattc tcagatgaaa     720
   gattctctct aacaattaca aatgattatt tttttccatg agtgttgcat gttcgaacgg     780
   tctgcccagt ctgtgagaga gcatagagaa ccctccctgc ccaatttgtt agagcataga     840
   gaaccctact gcatgagtag taagaaaaat attcggtctc aattcggcaa agaccacctc     900
   gaatggatga cttcaacgac aatctcatga tagtgttctg atcagcacca gttcacctat     960
   atattttatc tagggtttag tttgcatgta tcaatcctct ggtgcactag gtaattcttt    1020
   cctagtatca tatatcctta atactgtttt gtcttttaat ccatggctac catcagaaca    1080
   agctcaaagc agaaatcggg agcatcagcc atcctcttgc ttatcgcgct tgcagggtta    1140
   gtaaatgcgt gcaacgctgt gggtattgag ccaatgtgcg acactgtggt gtcgagtctt    1200
   ctgaggcttc tgccatgcag gacggctgtt gatccctcaa ttgccgccat tccacttcca    1260
   agctgctgca acgcggttga gtcagctggg cttcaatgcc tctgtctcgt cgttaacggc    1320
   cctccttttc caggggtcga ccgcggcctc gcaatgcagc tgcctgccaa atgccatctc    1380
   acccttcctc cctgtaacag ttagtt                                          1406
<210> 95
   <211> 2546
   <212> DNA
   <213> Pinus radiata
<400> 95
   ctggtagaac aagcagctca aggagcacca aggcacgagc ccactttgca tgttgtagac      60
   taacgaattt tacattagaa taaaatatgt cgacaatatc gaggagatct tctccaaaat     120
   ccaactcatt aatctctatt atgcacaaac gagtgatgtg tcgagactca tctgccaaca     180
   agccatcaac atcaagaagg gaacggaata gagccaaagg gaaccctaga gaccctcatc     240
   cacataataa tgaaatattc cacgtgtgtt tttcaaaatt tgaaaatttc atgtattttt     300
   tggttgattg gttgtggtct ggttttttcc aaattcaatc tagttcaagt ttttggagtc     360
   gaccagttgg gtaaccagtc taattctggt aacattgcat tgtacttgat ctcaataaaa     420
   gcatatagga tagaattatc ttctgtcttg atggtttcca tgagaaccaa ctgctatact     480
   atgaaaaata tcaatgttcc acaatatttt tgggacaagg gaacacaaga ttgagtcaac     540
   agttcaggac cccagaaaaa ttattcctga gttcgcagat tattttccta aaagtgaaca     600
   attcaagacc ctagccaaat cattcccaag tccaagttat gtgacactgc gactaacaag     660
   gcaagttgga agaaaccatc aatcaatctc ctagttaatg acagtccttg taagaagttc     720
   aagaagatta acaccagaag aggtcatgct gactgctttt atccaattct ctctgctctt     780
   caccaacaga aatagccaag atggttgtac ccattcccta atctaattta ttatatgaat     840
   ttctctttat ttttctacat ataaaaaaca aaaacttttc ttgatggtca aacagaaaag     900
   gcagttcgat tggatttaaa catccaaata cctcccacag attgagaagg ccaagcccca     960
   atccaacagt ccatgatata atatttattc aatcacactc aagataatgc aatgaaggtg    1020
   caccacgcta ttagattctg cacagaactc agatgactgt aattatcaac tttaaccagg    1080
   agtaatttaa aaactcaatt gtgcttcagc tatgtggaaa aactttggca ctggaaatgg    1140
   tataaatgtt gttgaataag caaacatttt tcaagcactg aattcaaagt caagtcaaag    1200
   gaacatctta cttgggctgt acaggaaatc tgaagtacaa aattagcgaa aaaacaggag    1260
   aaagagagta gtcattacat gttataacat taccatatag gattttgtaa tacttcttga     1320
   tatttcaact tcccgactga tgaaatgtat gccactacag aacaggtcag tcatgtatgt    1380
   gagcaattag ccaaactagg tcctaaggtt caaccagtgc agacaacgct gtaactgaaa    1440
   caaatttgtg ggacaattaa aaattctcta ccaggatagt tgtaccagta ggtgcccttt    1500
   tcaaaccatg atttaaaaca caagggtggc ttaccacttg accaaatcat ttaataacca    1560
   acccctcgaa catatcaaga aagaaaacat ctgcatataa gtaaattgaa agatgatatt    1620
   taagaggcac tgccttaaat tttccatttg gacaaatcca cattgcttga taagcataaa    1680
   accttggtta agagcaagtt tagggaacca tcaaatattt ctacatactt tacaatagtg    1740
   tgtttataaa gctaatcaaa tgcttctatt taaatatata gcaacctaca caagaaattc    1800
   actaggacag caatcacttg gccaatgtga ttaccaatat aaccatactt gaagagcata    1860
   cataaatcac aaataatgat tcaattagaa atatcttaaa gataaactat tattcaatgt    1920
   acatgttaca aagaacctca cctgtccgcc tttgaggagc aagtagacaa ctaaaagcgg    1980
   aggttacatc ctgaactgaa cttgttctcc tctgttccaa gaacttgcat tgtattttga    2040
   gtaacttcac tcgtgccgaa ttcggcacga gaaaacactt tgattgcttc cgcgggtggg    2100
   ttttactttc tctggaatag ttagttccgc cgtttttgga agatttatca gaatggccaa    2160
   aattcaggtg tcaaacggga gcgtcgtggt ggtggcggcg atgatattta tggtggcggt    2220
   ggccatgcaa aaccatcacg tcgccgccca aagtgctgac tgcgcaccac cgcggagttg    2280
   ctgagcccct gcgcctcggc ggtgggaaac aacccgcaga ccccactccc gaatgctgtg    2340
   ctgttctcca gaccgccgat gtcgactgca tctgcgccct cgtcgaatca accataaaat    2400
   tgccttccga atgtggtctt gacacccccc agtgcccaag cgactagatt ctcaagaccg    2460
   tgactgagtg ttggtttcag agccagtaaa cattcattct gctaataaat gagtgtatgg    2520
   agctttaata ttggaaaatg cttcat                                         2546
<210> 96
   <211> 4726
   <212> DNA
   <213> Pinus radiata
<400> 96
   gattactata gggcacgcgt ggtcgacggc cctggctggt cctaggacac cgtaatatat      60
   aacctcgaca tggcttacaa agctttgact tgcattctca ttgggcttac aatggtgctg     120
   ccaaaaatga aaaagtacat atgtacccct gttgaaatga gcagtaatag gcttgaacaa     180
   tagtgaattg ctacaaaatt atgaatgcct ttctttgctt gaatgtgggc taaggagaag     240
   tgggatttac atttgacttg caaatcctaa gacttgtcta gagctaagcc tccagaggag     300
   gaaccatctt acatagtctt gagtctagag cggagaagat agccaaattt gaaaggaaac     360
   ttttatttat ggggagaagg caaacaactt gagggggaag gatgatcaat aagtagggta     420
   agggaatcca caacagaggg cactaaggaa atgggggtgt tagaattggc aactagggcc     480
   aaattccacc ttgggatagc tctctggatg gagatgatga ttgcattaga ttcctctttt     540
   cgagaggacc aagattgata taaagatcat ctcatttgga caagcatagg tatgattttg     600
   aatttatacc cactcatgca caattttttt aggtccgcca catcatcatg taggctcatg     660
   aagcccaacg gacatgactc ttcgccctta tcgtcttgta taaatacaag tgtcctccca     720
   cctcatttgg catcttcatc tcttacagat tctctcttct tccctcattg gttcttgcat     780
   cattgggcat tctctctctc ccacgtgtgg cacaaggagg atgaaattac aagaccgaaa     840
   ataatagaaa ttttgcaatt tgaccagcat tgaccatgac cttccaagca tcattcgact     900
   tcaatttttt tgggttattt ttgtctcaac aagccgcata ttttggcaaa aaaatcgagg     960
   cattctgggc acttcgacta caaaccaaaa ttgtaggttg actgcaaatt tcaaatagtt    1020
   tgactattga cattgtcact gttttcgatt gactttgacc tcctaattag gccgagtttg    1080
   actaggggag gctgatttgt tttaaggaca tttgattgat gctttgacta gcattgactt    1140
   ttatagttaa ggttgaagtt tgactacagt tgactgcata aatttgcaga gatgttttga    1200
   ctttgaattg ggcaagtcaa tttgaatttt gtactatctc tctattttga acatttgata    1260
   taataataag aagattcgat caaagggttt tccccgcatt gggttttttc cctggcatcc    1320
   gccaaatctg gtgttctctt gtctttgctt gtcttatgca ttttgtttca ttttctatct    1380
   acttttactg tcaatgtgat tattgtcagt gttattggaa attggaaatt gtgattgggc    1440
   tgctaaggaa cattgaagta aattgtgcta aacaaagaac ataccattgt taacgaaaat    1500
   taacaaaggg gaaacacaga ggaatggttg caattgcaag attgtcattg attttgactt    1560
   caagtgagga aggtcgcgtg gaggtcgcaa ggggagagga ataggagaga aggccctatc    1620
   aacttgttca aggagagggg caatacaagg aatggaggaa ccctcaccaa tgaataatcc    1680
   atgcacaaaa gtaatagaat gaacaaactt accacacgga agagcttcct tgttgccaaa    1740
   agccttgcct ccgagacctg aatcctccaa tgcatcaaaa ttattgatca ttgaatcaac    1800
   cacgattagg gccacttcct tggctaataa agcaattagt gtagcaaatt ctaaagctaa    1860
   cttcaaagaa accttagctt tccaaaaaac aattgaaggg aggcaatgaa gatggcttat    1920
   cacactaagc ctaaacatgc cccaccctat ggcatctaaa acatctaaaa gggattcact    1980
   agtaatcgat cttttgtact tatgaaaaat tcccatgaac caattcgatc tcttccaaaa    2040
   agccatctat gaggtcaacc tcaacctggc tctaatgttg attgagcttg taatcctagc    2100
   cctactccaa tcttaagaac caaccaattt tatttccaat tgattcaagg acccctacac    2160
   tccaaaagaa gcaagggaag gccaaggaga atggcccaaa cttgagcaga gaataaggat    2220
   tctctgtgag ggtcgaaact aacatcccat tcacgtaaaa tcaaaccaga gagacctcaa    2280
   ctccaactct tcttaatgat gaagcacaaa tattattttg agtgaaattt gaaaccaaga    2340
   aaacctctca ctaatatatg gaagaggggc aatattcaac cattggtacc caaatcgcct    2400
   caagacactt accaagggag ccaaccaaac aatcttacca caaaaccaac caacagtgtt    2460
   tttacccaca agctcttgga tggaatccag gataatgtct tcaccaacaa ccatcttatg    2520
   tctatccttg caagcacaaa tgcattgagc tttagatttg gagtgcataa atacaggggg    2580
   gtatccaggg gggggagggg gtttgctaga accccagact caccaaggca tgaagacaaa    2640
   atgaggagag agggatctag attgggggat gcaagttgat gaagcatgaa aaggcaatcc    2700
   atcaccctgc atggcatatt tacgaaggtt gttcagagga atgagaacta atggatgaac    2760
   aacagctggt agaacaagca gctcaaggag cgccaaggca cgagcccact ttgcatgttg    2820
   tagactaacg aattttacat tagaataaaa tatgtcgaca atatcgagga gatcttctcc    2880
   aaaatccaac tcattaatct ctattatgca caaacgagtg atgtgtcgag actcatctgc    2940
   caacaagcca tcaacatcaa gaagggaacg gaatagagcc aaagggaacc ctagagaccc    3000
   tcatccacat aataatgaaa tattccacgt gtgtttttca aaatttggaa atttcatgta    3060
   ttttttggtt gattgttgtg gtctggtttt ttccaaattc aatctagttc aagtttttgg    3120
   agtcgaccag ttgggtaacc agtctaattc tggtaacatt gcattgtact tgatctcaat    3180
   aaaagcatat aggatagaat tatcttctgt cttgatggtt gccatgagaa ccaactgcta    3240
   tactatgaaa aatatcaatg ttccacaata tttttgggac aagggaacac aagattgagt    3300
   caacagttca ggaccccaga aaaattattc ctgagtttgc agattatttt cctaaaagtg    3360
   aacaattcaa gaccctagcc aaatcattcc caagtccaag ttatgtgaca ctgcgactaa    3420
   caaggcaagt tggaagaaac catcaatcaa tctcctagtt aatgacagtc cttgtaagaa    3480
   gttcaagaag attaacacca gaagaggtca tgctgactgc ttttatccaa ttctctctgc    3540
   tcttcaccaa cagaaatagc caagatggtt gtacccattc cctaatctaa tttattatat    3600
   gaatttctct ttatttttct acatataaaa aacaaaaact tttcttgatg gtgaaacaga    3660
   aaaggcagtt cgattggatt taaacatcca aatacctccc acagattgag aaggccaagc    3720
   cccaatccaa cagtccatga tataatattt attcaatcac actcaagata atgcaatgaa    3780
   ggtgcaccac gctattagat tctgcacaga actcagatga ctgtaattat caactttaac    3840
   caggagtaat ttaaaaactc aattgtgctt cagctatgtg gaaaaacttt ggcactggaa    3900
   atggtataaa tgttgttgaa taagcaaaca ttttagaaca tttttcaagc actgaattca    3960
   aagtcaagtc aaaggaacat cttacttggg ctgtacagga aatctgaagt acaaaattag    4020
   tgaaaaaaca ggagaaagag agtagtcatt acatgttata acattaccat ataggatttt    4080
   gtaatacttc ttgatatttc aacttcccga ctgatgaaat gtataccact acagaacagg    4140
   tcagtcatgt atgtgagcaa ttagccaaac taggtcctaa ggttcaacca gtgcagacaa    4200
   cgctgtaact gaaacaaatt tgtgggacaa ttaaaaattc tctaccagga tagttgtgcc    4260
   agtaggtgcc cttttcaaac catgatttaa aacacaaggg tggcttacca cttgaccaaa    4320
   tcatttaata accaacccct cgaacatatc aagaaagaaa acatctgcat ataagtaaat    4380
   tgaaagatga tatttaagag gcactgcctt aaatttccca tttggcaaat ccacattgct    4440
   tgataagcat aaaaccttgg ttaagagcaa gtttagggaa ccatcaaata tttctacata    4500
   ctttacaata gtgtgtttat aaagctaatc aaatgcttct atttaaatat atagcaacct    4560
   acacaagaaa ttcactagga cagcaatcac ttggccaatg tgattaccaa tataaccata    4620
   cttgaagagc atacataaat cacaaataat gattcaatta gaaatatctt aaagataaac    4680
   tattattcaa tgtacatgtt acaaagaacc tcacctgtcc gccttt                   4726
<210> 97
   <211> 635
   <212> DNA
   <213> Pinus radiata
<400> 97
   aaattctatg aaaaaaatcc aatcatatta aaagtccaat tgattagcaa ttttatgaga      60
   aaaatccaat tatgttaaaa gtcactgagt gtggccgaaa ttgtgaccga aattgaatgc     120
   aataaccgag ggtttttcaa accaaggtta agcctctcat cattggggtg tgtatgaaaa     180
   tgtaatgggc atcgataacc ttttattaca acttcacgaa aattgcctct attcaatggg     240
   tgtggatgaa aatgtaagtg cgcatcgata atggaaagcg atatgcagca aaatcaataa     300
   acctgacttc ccatgtgagt gatgatttga tcgtacaact gatggtgtga agttactttc     360
   agcttcacct tcgggcataa tcagggaagt agggccaagt ttgcttagta tcactctaat     420
   ccccaacacc gtgattacta tcttcatcaa caatggccac cttcgtcatt actttaactg     480
   gtgggataca gctactttac aactgtaaat ttgttgaggc agcctatcct cagcctatac     540
   atactaatta ttgcagctcg attaggtatc tgctgtgaga atagctgtgt atctctgcgc     600
   tggttgcagg atccaagttc ctctcagagc cctcc                                635
<210> 98
   <211> 468
   <212> DNA
   <213> Pinus radiata
<400> 98
   ctggtaaatt gagattccaa attattgatg cgaagcttcc tcgtggctgg tcggtgctgc      60
   tggcatccaa accctaaatg aaaaagaaaa aggtgtccgg acggattttt ttagtatttt     120
   tttcttattt tttttatgaa ccgtcggatt cgagatcgga cggcgatccg aaactgcaag     180
   cgtcggccgt cggatgcagc atcggacggc aaagaaggaa ccctaaaacg cattgcaacg     240
   tgcttggtgg gtggagggtc tatggccagt atatgttgat aacaagggag aggaagtagt     300
   cctcttcatc tagtgcgagt ctctctgctt ttctacgccg ctgcgaagct gttctgtggt     360
   gtttctgatt ctccagactc aggcagtcgt ttttgtaaga gaatttagtt catcatggga     420
   aaggagaaaa cccatatcaa cattgtggtt attggccatg tcgactcc                   468
<210> 99
   <211> 222
   <212> DNA
   <213> Pinus radiata
<400> 99
   atccaaaccc taaatgaaaa agaaaaaggt gtccggacgg atttttttag tatttttttt      60
   tcttattttt tttttatgaa ccgtcggatt cgagatcgga cggcgatccg aaactgcaag     120
   cgtcggccgt cggatgcagc atcggacggc aaagaaggaa ccctaaaacg cattgcaacg     180
   tgcttggtgg gtggagggtc tatggccaga tatgttgtaa tc                        222
<210> 100
   <211> 597
   <212> DNA
   <213> Pinus radiata
<400> 100
   aaatgaggca gctaactatt tatttggttt tggcttcact gacttgttcc ttagtgtatt      60
   aatgaacaat ctctttagac tcagagatgg tgagaaagat tctatgagaa atattcttgt     120
   tattgcttcg actcatatcc cccaaagagt ggatccagct ctaatagctc caaatcgatt     180
   agatagatcg atcaatattc gaatgcttgt tatcccacaa cgacaaaggg aatttcctat     240
   tcttttatgt agcaaaggat tatactcggg aaaatgtccc gatgaatttg gatctataac     300
   catagattat gatgcacgag ctctattagc tcaggcctct ctgctgctcc ttggattgca     360
   atctcattct ctgatttgcc gtgctgtttg ctctgctcac ttcagcccag atggagacct     420
   tcttgttcac atcggagtct gtaaatgagg gacacccaga caaactctgt gaccagattt     480
   ctgatgcagt gttggatgca tgcctcaccc aggaccccga cagcaaggta gcatgcgaga     540
   cttgcactaa aacgaacatg gtcatggttt ttggtgaaat caccaccaag gccgatg        597
<210> 101
   <211> 669
   <212> DNA
   <213> Pinus radiata
<400> 101
   cctggaaatg ctatattaac tcaacaaagg attttcagcc aatcacaatt tgacaggttt      60
   gaaatgaaag attacaggca tttccaatgg aacagaatat aattacttta ttccctcaaa     120
   gtatcgtata aaataaatct tttgctccac acactttgga aaatacattt tcaacaatgc     180
   accgacaaac tttttctacc acgttatgga accatacaag ttaaatttaa acacgaatta     240
   cgcgtatatt tctaataaat cgatggttga gattgaatgc cgtgggcgat tctcacgcgt     300
   ccgattggga tcactagtcc atcactcatg gtctgcattg cctttaaatt ggcggggcga     360
   ggaaagacca atgcgtcatt ggtgtagacg agctctatta gctcaggcct ctctgctgct     420
   ccttggattg caatctcatt ctctgatttg ccgtgctgtt tgctctgctc acttcagccc     480
   agatggagac cttcttgttc acatcggagt ctgtaaatga gggacaccca gacaaactct     540
   gtgaccagat ttctgatgca gtgttggatg catgcctcac ccaggacccc gacagcaagg     600
   tagcatgcga gacttgcact aaaacgaaca tggtcatggt ttttggtgaa atcaccacca      660
   aggccgatg                                                             669
<210> 102
   <211> 230
   <212> DNA
   <213> Pinus radiata
<400> 102
   atccacctcg gaatgaaatc actatgcaca ctccaccttt tttttggctt cttttctcgt      60
   tgcctttacc atcagaatca agcacgaaga gtaaatatca cccatgcttt acaagtgggt     120
   tggtagcatt agcgattccc ttcaccaaat gaaccctttg ctggtgatga gtggacaacc     180
   taaagttgtt tgctggtgat gagtggacaa ccagagtggg ggttggggaa                230
<210> 103
   <211> 596
   <212> DNA
   <213> Eucalyptus grandis
<400> 103
   actttgaaag ggtctcgagt caaagtgctc aaattgagag ggagaatttt agaacaaaat      60
   cagatttgga gaatacatgc cattttaggg ggattttggg gatttcgcat atggcgtcgc     120
   gtcgtcggcg ccttcttctt tacagattgt atcctcccat taaccgcgtg gacctgcact     180
   gtaaccccga aacggtgggg gccaatttcg tctttccgcc tcctccactc agcttcgtgg     240
   aagattaaaa tcctcaccgt ccgtgcaaac gccacgtggc gcgttagttt gcgcgtggaa     300
   aggtcctcac gaaccgtaaa gggcaaaaaa aagggaaaat aaaaaaggag gaggaggagg     360
   gaggaggaag aattgtccga ttgaaaataa gagtgcggtg gtgtggtgtg ggtagatctt     420
   gaattgaacg agctcaatcc gcgtatttaa acccgccccg cttcctcatt cttccttgtc     480
   catttcaact ctccctctct ccctctcttc tgcccctcga tcgatccagc gatcttccta     540
   tttccggacg cggggagcag ctcctcttgt cgaaggttct aaattagtgt ggagag         596
<210> 104
   <211> 653
   <212> DNA
   <213> Eucalyptus grandis
<400> 104
   aaaattttcc tttattttct tttcattaaa aagataaata aataaaaaaa aaaaagaagg      60
   aaaacacatc gaggtgaggc ttaaaggtgc taggcaagga ccaccaagcc tacacaaggg     120
   tcggcgaccc tcaccaatgc tggggcgagg gtgagcaacc ctcatccaaa tctggagagg     180
   gttgtcactc gagaaagggt cactggccct cccctaaccg ctactaacat cgttggcctt     240
   cgtcaccacc gcactaacaa tgggccacta attttatatt tttcgtgata ttaatcctat     300
   taaaaatgaa aatatctcct taattaatta agcttgtcag gaccgatgta aacaaaatta     360
   atgtaaatgg acgcgccttt gacttgccaa caaactcgaa acgacgtttc ctccgtctga     420
   taactatctc gcgacctccg acgacatccg acggtgcaga tcgggtcccg gtcaaccatc     480
   cagatccacc cgattttctc ccggccctcg acaactccca ccaccacctc tttcctccct     540
   ctttccttcc ttcctttctc accagatttt cccgagaaaa tcacagagag agaaagaaaa     600
   acctcaccgc ctagagagag aaagagagaa agagggaaga gagagagaga gag            653
<210> 105
   <211> 342
   <212> DNA
   <213> Eucalyptus grandis
<400> 105
   agttgggtaa ccagtctaat tctggtaaca ttgcattgta cttgatctca ataaaagcat      60
   ataggataga attatcttct gtcttgatgg tttccatgag aaccaactgc tatactatga     120
   aaaatatcaa tgttccacaa tatttttggg acaagggaac acaagattga gtcaacagtt     180
   caggacccca gaaaaattat tcctgagttc gcagattatt ttcctaaaag tgaacaattc     240
   aagaccctag ccaaatcatt cccaagtcca agttatgtga cactgcgact aacaaggcaa     300
   gttggaagaa accatcaatc aatctcctag ttaatgacag tc                        342
<210> 106
   <211> 342
   <212> DNA
   <213> Eucalyptus grandis
<400> 106
   ggtctggaag ctcatctctc caatttggtg aagattacag ctataagagg tagctatgat      60
   gtgctggcca aatgcaagtg atgaaatacg tggaccacca agtgcgaagg cattcgaaga     120
   acgagggtcg aatttatagt gggcgaagga tgattaggtg gaatatgaca agaaaatagg     180
   tttgaaagag aaataaatat tatgatagtg aagggtcttc acatggttag tttgatctgt     240
   ccgagggtgt ccacccttgt ctgatccgca attgctcttg gtcgtgctga attttagagt     300
   gtagccaaag taagaatttt cctttcactg tccggacatt tc                        342
<210> 107
   <211> 948
   <212> DNA
   <213> Eucalyptus grandis
<400> 107
   ctgacaaatg caaatatcta aaaccattgg ttgtttggtg cttgcaagtc tggattaccc      60
   cactttatgt ttcacctttc aataatgaat aacaaggtac tcgggaaaaa aaggaaaggg 120
   aaattcgcac aaccaaagtt gctatgcaga agtcaactca atcctaatca agttgatgag 180
   agtgttgggc cctattttct gcagcaaaca tgaatctcga ttcatctccc tcgcaaaaga 240
   taaggaagct gcaaaagctt tcctcctaag tttgttggca agcaaattga ttttgtacca 300
   gaaataaata caaagtgaaa cccaagcaat cacgcatggc ctgatttgtg ccatgtccat 360
   ttgatctccc tctactattt ttcctgcttt ctcaagcaaa ctagttgctg taacagtgaa 420
   tgatcccccg gctctctctc tctctctctc tctctctctc catttattcc atccatgttt 480
   ttgcttttcg cacaacactt atcattgagg tgctaactac tgaattcccc taactaaaaa 540
   ttggaacctc tcacctaatt tcattttctc ccactttgat gagcaccact ctctttccca 600
   gatttcaaat aaattgccac tctctccctc ctctttcctc acacaaccaa aagccttctt 660
   caagtaccac ttcttcactg tcctctcttc acaatccccc tcttaccaag agcaaagcaa 720
   aaaacatgat gaagagactg tcatttctgc tcctactggt cctgctcttc caatgctcta 780
   ccaccttggc tcagcctgcg gccgccccag ctccgcctgt gatagccccg gctgcacctg 840
   ctacgcctgc cttaggcccg gctcctcctg tcttaggccc agctcctgca ggcccaaccg 900
   acatcacgaa ggtcctcaag aaggtgagcc aatttacggt gctgctca 948
<210> 108
   <211> 362
   <212> DNA
   <213> Eucalyptus grandis
<400> 108
   ccatcactca taatcaacaa ggatatctca tcatgtcttc caaccaaatt aaaccccaga 60
   catctctaaa gcagtatgga aaagaaaaca gtccggaagt ctctagctca aaaactgtaa 120
   ccccgaccta attccggttg tctctgatta catcaattct tatgtcttaa cactccattc 180
   gcacctccac aataaataga tcggcccttc atctcccctt accatcgaat ccaatcccaa 240
   aaacacttgc tcagacacca tcaaatcctt cgcaaagtct ttttcttaca aaaaacaaac 300
   gaaagcaacc atgaagcacc agttcattgt tctggctctc ttattcctca tcaacacagc 360
   cc 362
<210> 109
   <211> 326
   <212> DNA
   <213> Eucalyptus grandis
<400> 109
   aaaaattaca atcaatggtt atcaatggat gttacaaagg gaggttacat atagaggtta 60
   taaaagaggg ttacaaatag atgtctcaaa caattaccaa gcggttagat tgactccact 120
   attttgacgg ttctcttgac tttactatct caacgattac tttatttcat catgttgacg 180
   gttgcatcca tgattgttga cttcactttt tgtcgattcc ttcaagctgc tgattcttca 240
   agttgccaat aattttattc ataaatgacg aaactctagc ctcatccatt aagtttgtta 300
   cttgtccaca ataattaaat tcggta 326
<210> 110
   <211> 296
   <212> DNA
   <213> Pinus radiata
<400> 110
   tgctcccggt catgacaccg ccattctcgc tcttcatttc caattcaaat cacttggttg      60
   ttgttcacac acacgggtct ttatatgacg agtgctgctg cgattataaa tagacggggc     120
   aattacaaca aaaactcaca gcatttgaag gaagttggag tggtagagtg agaaatacac     180
   agcctaatct gaaggaagtt cgagtaatag agtgagaaat ggatcttctt ctcctcatga     240
   tgatgcttgt gatgatgggt gtagcaatgc ctactcattc tcaacaaatc actagt         296
<210> 111
   <211> 723
   <212> DNA
   <213> Pinus radiata
<400> 111
   cgttttacgc gggaacaatg aaaacagtac aatcgaaaga gtcaagtcgt gaggttcatt      60
   tcgatgaagt tcccagagat tgtctcgttc aacgtttcct cttttttcgg gtcaagtcgg     120
   gtacagaaga ccactttctt tacgcggtca agacaccgcc attctcgggt caagtcggga     180
   ggtccctcct gctcttcctt tttccaaatc cgtaaaattt acagattttt ttaatgtatg     240
   aagcccactt tctttatgcg gttgctccca gtcaagacac cgccattgtt gttcacacgc     300
   acgggtcttt atatgacgag tgctgctgcg attataaata gacggggcaa ttacaacaaa     360
   aactcacagc atttgaagga agttggagtg gtagagtgag aaatcatttg aagggagttg     420
   gagtggtaga gtgagaaatc atttgaaggg agttgagaaa tatattggga atctctcttt     480
   tttgcagcaa ttagatcttt cctttaatgc tttgagtggg agaattccga cagagtttgg     540
   gaacctctct cttttgcggc aataagttgg agtggtagtt ggagtggtag agtgagaaat     600
   acacagccta atctgaagga agttggagtg atagagtgag aaatggatcg tcttcttctc     660
   ttcatgttga tgcttgtgat gatgggtgta gcaatgccta ctcattctca acaaatcact     720
   agt                                                                   723
<210> 112
   <211> 1301
   <212> DNA
   <213> Pinus radiata
<400> 112
   actatagggc acgcgtggtc gacggccctg gctggtagcg acagagctgg ttcagtgacc      60
   gttcgtgatt agccgcagta aaacaaaacc ctaaccgtaa ccctttcgcg cagattccat     120
   ccttccccgt cctaccaaaa cccaaacttc ttgcccgaac tcaccttcta tgtattaatt     180
   cttattatta tttaataata ataaatagtt aaacataaat ttataaatta attaattttt     240
   atgattttta ttttagttta aaaatgtgac attgttatag attaatgctt atgaacgttt     300
   attggccata attaccctaa ttaattataa ttaaaatata tagttataat taaaaaattg     360
   tatattttat aaattgaatt aagaatttct gatgatattt catcattcaa ttccatctta     420
   tcaaagttag agggaatagt taaccatgta ctagatctat tcatagctaa catttgccaa     480
   gttcgtacta ggagacttgg atttttttta aaacataatt ttggcagtaa aaagtgaatt      540
   ctattgtttt gaaaacaaaa caaaatacag gaagcgtgat tgtggggttg ttgttgaact     600
   tgcccgggca aaagaagaat gattagcggt agaggagtta gtagttacgt tcaactaaat     660
   gcgtgactaa attatttatc ctccgccatg gaagcaggtg attcacacac aacttgctgc     720
   acacattgct ctcaaacctt tcctataaat atccgtagca ggggctgcga tgatacacaa     780
   cgcatttaat caaactactt tgattacttt ctgtgggttc tactttcttt gaatagtcag     840
   ttctgctgtt tttagaagat ttataagaat ggccaaaatt caggtatcaa acgggaacgt     900
   cgtggtggtg gctgcgatgt tatttatggt ggtggtggcc atgcaaaacc atcacgtcgc     960
   cgcccaaagt gctgactgcg ccgccaccgc ggagtccctg agcccctgcg cctcggcggt    1020
   gggaaacaac ccacaggatc ccactcccga atgctgtgct gttcttcaga ccgctaatgt    1080
   cgactgcatc tgcgccctcg tccaatcaac catgcaattg ccttccgaat gcggtcttga    1140
   gactcctcag tgcccaagcg actagggtct caagaccgtg actgagtgct ggtttcagag    1200
   acagtagaca ttctgcctaa taaatgattg tatgagagct tttatatatg gaattgctca    1260
   tatgctttcc tagatatgaa attattaaat tccatatgct t                        1301